# EUROPEAN PATENT APPLICATION

(11) **EP 2 423 226 A2**
(43) Date of publication of application: **29.02.2012**
(21) Application number: 11179368.3
(22) Date of filing: 09.11.2007
(51) Int. Cl.: C07K 16/22, A61K 39/395

(54) **Antibody-based diagnostics and therapeutics**

(30) Priority: 10.11.2006 US 857882 P
(62) Divisional of application: 07868714.2
(71) Applicant: Amgen Inc., Thousand Oaks, CA 91320-1799 (US); UCB Pharma, S.A., 1070 Brussels (BE)
(72) Inventor: Robinson, Martyn Kim, Woodburn Green Buckinghamshire HP10 0JH (GB); Paszty, Christopher J., Ventura, CA 93003 (US); Lawson, Alistair, Woodburn Green Buckinghamshire HP10 0JH (GB); Popplewell, Andy, Slough, Berkshire SL1 2WE (GB); Henry, Alistair James, Uxbridge, Middlesex UB8 2LX (GB)
(74) Representative: Campbell, Patrick John Henry

(57) **Abstract**

Compositions and methods relating to sclerostin binding agents, such as antibodies and polypeptides capable of binding to sclerostin, are provided.

SEQUENCE LISTING

<110> AMGEN INC. and UCB PHARMA S.A.

<120> ANTIBODY-BASED DIAGNOSTICS AND THERAPEUTICS

<130> 31173/40011

<150> US 60/857,882
<151> 2006-11-10

<160> 327

<170> FastSEQ for Windows Version 4.0

<210> 1
<211> 190
<212> PRT
<213> Homo sapiens

<400> 1
Gln Gly Trp Gln Ala Phe Lys Asn Asp Ala Thr Glu Ile Ile Pro Glu
1               5                   10                  15
Leu Gly Glu Tyr Pro Glu Pro Pro Pro Glu Leu Glu Asn Asn Lys Thr
            20                  25                  30
Met Asn Arg Ala Glu Asn Gly Gly Arg Pro Pro His His Pro Phe Glu
        35                  40                  45
Thr Lys Asp Val Ser Glu Tyr Ser Cys Arg Glu Leu His Phe Thr Arg
    50                  55                  60
Tyr Val Thr Asp Gly Pro Cys Arg Ser Ala Lys Pro Val Thr Glu Leu
65                  70                  75                  80
Val Cys Ser Gly Gln Cys Gly Pro Ala Arg Leu Leu Pro Asn Ala Ile
                85                  90                  95
Gly Arg Gly Lys Trp Trp Arg Pro Ser Gly Pro Asp Phe Arg Cys Ile
            100                 105                 110
Pro Asp Arg Tyr Arg Ala Gln Arg Val Gln Leu Leu Cys Pro Gly Gly
        115                 120                 125
Glu Ala Pro Arg Ala Arg Lys Val Arg Leu Val Ala Ser Cys Lys Cys
    130                 135                 140
Lys Arg Leu Thr Arg Phe His Asn Gln Ser Glu Leu Lys Asp Phe Gly
145                 150                 155                 160
Thr Glu Ala Ala Arg Pro Gln Lys Gly Arg Lys Pro Arg Pro Arg Ala
                165                 170                 175
Arg Ser Ala Lys Ala Asn Gln Ala Glu Leu Glu Asn Ala Tyr
            180                 185                 190

<210> 2
<211> 106
<212> PRT
<213> Mus musculus

<400> 2
Thr Asp Ala Ala Pro Thr Val Ser Ile Phe Pro Pro Ser Ser Glu Gln
1               5                   10                  15
Leu Thr Ser Gly Gly Ala Ser Val Val Cys Phe Leu Asn Asn Phe Tyr
            20                  25                  30
Pro Lys Asp Ile Asn Val Lys Trp Lys Ile Asp Gly Ser Glu Arg Gln
        35                  40                  45
Asn Gly Val Leu Asn Ser Trp Thr Asp Gln Asp Ser Lys Asp Ser Thr
    50                  55                  60
Tyr Ser Met Ser Ser Thr Leu Thr Leu Thr Lys Asp Glu Tyr Glu Arg
65                  70                  75                  80

His Asn Ser Tyr Thr Cys Glu Ala Thr His Lys Thr Ser Thr Ser Pro
                85                  90                  95
Ile Val Lys Ser Phe Asn Arg Asn Glu Cys
            100                 105

<210> 3
<211> 324
<212> PRT
<213> Mus musculus

<400> 3
Ala Lys Thr Thr Pro Pro Ser Val Tyr Pro Leu Ala Pro Gly Ser Ala
1               5                   10                  15
Ala Gln Thr Asn Ser Met Val Thr Leu Gly Cys Leu Val Lys Gly Tyr
            20                  25                  30
Phe Pro Glu Pro Val Thr Val Thr Trp Asn Ser Gly Ser Leu Ser Ser
        35                  40                  45
Gly Val His Thr Phe Pro Ala Val Leu Gln Ser Asp Leu Tyr Thr Leu
    50                  55                  60
Ser Ser Ser Val Thr Val Pro Ser Ser Thr Trp Pro Ser Glu Thr Val
65                  70                  75                  80
Thr Cys Asn Val Ala His Pro Ala Ser Ser Thr Lys Val Asp Lys Lys
                85                  90                  95
Ile Val Pro Arg Asp Cys Gly Cys Lys Pro Cys Ile Cys Thr Val Pro
            100                 105                 110
Glu Val Ser Ser Val Phe Ile Phe Pro Pro Lys Pro Lys Asp Val Leu
        115                 120                 125
Thr Ile Thr Leu Thr Pro Lys Val Thr Cys Val Val Val Asp Ile Ser
    130                 135                 140
Lys Asp Asp Pro Glu Val Gln Phe Ser Trp Phe Val Asp Asp Val Glu
145                 150                 155                 160
Val His Thr Ala Gln Thr Gln Pro Arg Glu Glu Gln Phe Asn Ser Thr
                165                 170                 175
Phe Arg Ser Val Ser Glu Leu Pro Ile Met His Gln Asp Trp Leu Asn
            180                 185                 190
Gly Lys Glu Phe Lys Cys Arg Val Asn Ser Ala Ala Phe Pro Ala Pro
        195                 200                 205
Ile Glu Lys Thr Ile Ser Lys Thr Lys Gly Arg Pro Lys Ala Pro Gln
    210                 215                 220
Val Tyr Thr Ile Pro Pro Pro Lys Glu Gln Met Ala Lys Asp Lys Val
225                 230                 235                 240
Ser Leu Thr Cys Met Ile Thr Asp Phe Phe Pro Glu Asp Ile Thr Val
                245                 250                 255
Glu Trp Gln Trp Asn Gly Gln Pro Ala Glu Asn Tyr Lys Asn Thr Gln
            260                 265                 270
Pro Ile Met Asp Thr Asp Gly Ser Tyr Phe Val Tyr Ser Lys Leu Asn
        275                 280                 285
Val Gln Lys Ser Asn Trp Glu Ala Gly Asn Thr Phe Thr Cys Ser Val
    290                 295                 300
Leu His Glu Gly Leu His Asn His His Thr Glu Lys Ser Leu Ser His
305                 310                 315                 320
Ser Pro Gly Lys

<210> 4
<211> 132
<212> PRT
<213> Mus musculus

<400> 4
Met Glu Thr Asp Thr Ile Leu Leu Trp Val Leu Leu Leu Trp Val Pro

 1               5                   10                  15
Gly Ser Thr Gly Asp Ile Val Leu Thr Gln Ser Pro Ala Ser Leu Ala
            20                  25                  30
Val Ser Leu Gly Gln Arg Ala Thr Ile Ser Cys Lys Ala Ser Gln Ser
        35                  40                  45
Val Asp Tyr Ala Gly Asp Asn Tyr Met Asn Trp Tyr Gln Gln Lys Pro
    50                  55                  60
Gly Gln Pro Pro Lys Leu Leu Ile Tyr Thr Ala Ser Asn Leu Glu Ser
65                  70                  75                  80
Gly Ile Pro Ala Arg Phe Ser Gly Ser Gly Ser Gly Thr Asp Phe Thr
                85                  90                  95
Leu Asn Ile His Pro Val Glu Glu Glu Asp Ala Ala Thr Tyr Tyr Cys
            100                 105                 110
Gln Gln Ser Asn Glu Asp Pro Pro Thr Phe Gly Gly Gly Thr Lys Leu
        115                 120                 125
Glu Leu Lys Arg
    130

<210> 5
<211> 393
<212> DNA
<213> Mus musculus

<400> 5
atggagacag acacaatcct gctatgggtg ctgctgctct gggttccagg ctccactggt 60
gacattgtgc tgacccaatc tccagcttct ttggctgtgt ctctagggca gagggccacc 120
atctcctgca aggccagcca aagtgttgat tatgctggtg ataattatat gaactggtac 180
caacagaaac caggacagcc acccaaactc ctcatctata ctgcatccaa tctagagtct 240
gggatcccag ccaggtttag tggcagtggg tctgggacag acttcaccct caacattcat 300
cctgtggagg aggaggatgc tgcaacctat tactgtcagc aaagtaatga ggatcctccg 360
acgttcggtg gaggcaccaa gttggagctc aaa                              393

<210> 6
<211> 138
<212> PRT
<213> Mus musculus

<400> 6
Met Gly Trp Asn Trp Ile Phe Ile Leu Ile Leu Ser Val Thr Thr Gly
 1               5                   10                  15
Val His Ser Glu Val Gln Leu Gln Gln Ser Gly Pro Glu Leu Val Lys
            20                  25                  30
Pro Gly Ala Ser Val Lys Ile Ser Cys Lys Ala Ser Gly Tyr Pro Phe
        35                  40                  45
Thr Gly Tyr Phe Met His Trp Val Lys Gln Ser Pro Glu Asn Ser Leu
    50                  55                  60
Glu Trp Ile Gly Glu Ile Asn Pro Ser Thr Gly Gly Thr Thr Tyr Asn
65                  70                  75                  80
Gln Arg Phe Lys Gly Lys Ala Thr Leu Thr Val Asp Lys Ser Ser Ser
                85                  90                  95
Thr Ala Tyr Met Gln Leu Lys Ser Leu Thr Ser Glu Asp Ser Ala Val
            100                 105                 110
Tyr Tyr Cys Thr Arg Trp Gly Tyr Asn Pro Tyr Ala Leu Asp Tyr Trp
        115                 120                 125
Gly Gln Gly Thr Ser Val Thr Val Ser Ser
    130                 135

<210> 7
<211> 411
<212> DNA
<213> Mus musculus

<400> 7
atgggatgga actggatctt tattttaatc ctgtcagtaa ctacaggtgt ccactctgag 60
gtccaactgc agcagtctgg acctgagctg gtgaagcc[?]g gggcttcagt gaagatatcc 120
tgcaaggctt ctggttaccc attcactggc tacttcatgc actgggtgaa acaaagtcct 180
gaaaatagtc ttgagtggat tggagagatt aatcctagca ctgggggtac tacctacaac 240
cagaggttca agggcaaggc cacattaact gtagataaat cctccagcac agcctacatg 300
cagctcaaga gcctgacatc tgaagactct gcagtcta[?]t actgtacaag atggggatat 360
aacccctatg ctttggacta ctggggtcaa ggaacctcag tcaccgtctc g 411

<210> 8
<211> 127
<212> PRT
<213> Mus musculus

<400> 8
   Met Arg Pro Ser Ile Gln Phe Leu Gly Leu Leu Leu Phe Trp Leu His
   1 5 10 15
   Gly Ala Gln Cys Asp Ile Gln Met Thr Gln Ser Pro Ser Ser Leu Ser
   20 25 30
   Ala Ser Leu Gly Gly Lys Val Thr Ile Thr Cys Lys Ala Ser Gln Asp
   35 40 45
   Ile Asn Lys Tyr Ile Ala Trp Tyr Gln His Lys Pro Gly Lys Gly Pro
   50 55 60
   Arg Leu Leu Ile His Tyr Thr Ser Thr Leu Gln Pro Gly Ile Pro Ser
   65 70 75 80
   Arg Phe Ser Gly Ser Gly Ser Gly Arg Asp Tyr Ser Phe Ser Ile Ser
   85 90 95
   Asn Leu Glu Pro Glu Asp Ile Ala Thr Tyr Tyr Cys Leu Gln Tyr Asp
   100 105 110
   Asn Leu Tyr Thr Phe Gly Gly Gly Thr Lys Leu Glu Leu Lys Arg
   115 120 125

<210> 9
<211> 378
<212> DNA
<213> Mus musculus

<400> 9
   atgagaccgt ctattcagtt cctggggctc ttgttgttct ggcttcatgg tgctcagtgt 60
   gacatccaga tgacacagtc tccatcctca ctgtctgcat ctctgggagg caaagtcacc 120
   atcacttgca aggcaagcca agacattaac aagtatatag cttggtacca acacaagcct 180
   ggaaaaggtc ctaggctgct catacattac acatctacat tacagccagg catcccatca 240
   aggttcagtg gaagtgggtc tgggagagat tattccttca gcatcagcaa cctggagcct 300
   gaagatattg caacttatta ttgtctacag tatgataatc tatacacgtt cggagggggg 360
   accaagctgg aactgaaa 378

<210> 10
<211> 134
<212> PRT
<213> Mus musculus

<400> 10
   Met Glu Trp Ser Trp Val Phe Leu Phe Leu Leu Ser Val Thr Ala Gly
   1 5 10 15
   Val His Ser Gln Val Gln Leu Gln Gln Ser Gly Ala Glu Leu Met Lys
   20 25 30
   Pro Gly Ala Ser Val Lys Ile Ser Cys Lys Ala Thr Gly Tyr Thr Phe
   35 40 45
   Ser Ser Tyr Trp Ile Glu Trp Val Lys Gln Arg Pro Gly His Gly Pro
   50 55 60
   Glu Trp Ile Gly Glu Ile Phe Pro Arg Asn Gly Ser Thr Tyr Tyr Asn
   65 70 75 80
   Glu Lys Phe Lys Gly Lys Ala Thr Phe Thr Ala Asp Thr Ser Ser Asn
                   85                  90                  95
   Thr Ala Tyr Met Gln Leu Ser Ser Leu Thr Ser Asp Asp Ser Ala Val
               100                 105                 110
   Tyr Tyr Cys Ala Ile Ile Asn Thr Leu Asp Tyr Trp Gly Gln Gly Thr
           115                 120                 125
   Thr Leu Thr Val Ser Ser
       130

<210> 11
<211> 399
<212> DNA
<213> Mus musculus

<400> 11
atggaatgga gctgggtctt tctcttcctc ctgtcagtaa ctgcaggtgt ccactcccag 60
gttcagctgc agcagtctgg agctgagctg atgaagcctg gggcctcagt gaagatatcc 120
tgcaaggcta ctggctacac attcagtagt tattggatag agtgggtaaa gcagaggcct 180
ggacatggcc ctgagtggat tggagagatt tttcctagaa atggtagtac ttactacaat 240
gagaaattca agggcaaggc cacattcact gcagatacat cctccaacac agcctacatg 300
caactcagca gcctgacatc tgacgactct gccgtctatt actgtgcaat tattaatacg 360
cttgactact ggggccaagg caccactctc acagtctcg                        399

<210> 12
<211> 132
<212> PRT
<213> Mus musculus

<400> 12
Met Lys Leu Pro Val Arg Leu Leu Val Leu Met Phe Trp Ile Pro Val
1               5                   10                  15
Ser Ser Ser Asp Val Val Met Thr Gln Thr Pro Leu Ser Leu Pro Val
            20                  25                  30
Ser Leu Gly Asp Gln Ala Ser Ile Ser Cys Arg Ser Ser Gln Ser Leu
        35                  40                  45
Val His Ser Asn Gly Asp Thr Tyr Leu His Trp Tyr Leu Gln Lys Pro
    50                  55                  60
Gly Gln Ser Pro Lys Leu Leu Ile Tyr Glu Ile Ser Asn Arg Phe Ser
65                  70                  75                  80
Gly Val Pro Asp Arg Phe Ser Gly Ser Gly Ser Gly Thr Asp Phe Thr
                85                  90                  95
Leu Lys Ile Ser Arg Val Glu Ala Glu Asp Leu Gly Val Tyr Phe Cys
            100                 105                 110
Ser Gln Ser Thr His Val Pro Phe Thr Phe Gly Gly Gly Thr Lys Leu
        115                 120                 125
Glu Ile Lys Arg
    130

<210> 13
<211> 393
<212> DNA
<213> Mus musculus

<400> 13
atgaagttgc ctgttaggct gttggtgctg atgttctgga ttcctgtttc cagtagtgat 60
gttgtgatga cccaaactcc actctccctg cctgtcagtc ttggagatca agcctccatc 120
tcttgcagat ctagtcagag ccttgtacac agtaatggag acacctattt acattggtac 180
ctacagaagc caggccagtc tccaaaactc ctgatctacg aaatttccaa ccgattttct 240
ggggtcccag acaggttcag tggcagtgga tcagggacag atttcacact caagatcagc 300
agagtggagg ctgaggatct gggagtttat ttctgctctc aaagtacaca tgttccgttc 360
acgttcggag gggggaccaa gctggaaata aaa                              393

<210> 14
<211> 135
<212> PRT
<213> Mus musculus

<400> 14
Met Gly Trp Ser Trp Val Ile Phe Phe Leu Met Ala Val Val Ile Gly
1               5                   10                  15
Ile Asn Ser Glu Val Gln Leu Gln Gln Ser Gly Ala Glu Leu Val Arg
            20                  25                  30
Ser Gly Ala Ser Val Arg Leu Ser Cys Thr Ala Ser Gly Phe Asn Ile
        35                  40                  45
Lys Asp Tyr Tyr Met His Trp Val Arg Gln Arg Pro Glu Gln Gly Leu
    50                  55                  60
Glu Trp Ile Gly Trp Asn Asp Pro Glu Thr Gly Asp Thr Glu Tyr Ala
65                  70                  75                  80
Pro Lys Phe Gln Gly Lys Ala Thr Met Thr Ser Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Glu Gly Ser Gly Leu Ile Pro Tyr Trp Gly Pro Gly
        115                 120                 125
Thr Leu Val Thr Val Ser Ser
    130                 135

<210> 15
<211> 402
<212> DNA
<213> Mus musculus

<400> 15
atgggatgga gctgggtcat cttcttcctg atggcagtgg ttataggaat caattcagag 60
gttcagctgc agcagtctgg ggcagaactt gtgaggtcag gggcctcagt caggttgtcc 120
tgcacagctt ctggcttcaa cattaaagac tactatatgc actgggtgag gcagcggcct 180
gaacagggcc tggagtggat tggatggaat gatcctgaga ctggtgatac tgaatatgcc 240
ccgaagttcc agggcaaggc cactatgact tcagacacat cctccaacac agcctacctg 300
cagctcagca gcctgacatc tgaggacact gccgtctatt actgtaatga gggctcgggc 360
ttgattcctt actggggccc agggactctg gtcacagtct cg                    402

<210> 16
<211> 132
<212> PRT
<213> Mus musculus

<400> 16
Met Lys Leu Pro Val Arg Leu Leu Val Leu Met Phe Trp Ile Pro Val
1               5                   10                  15
Ser Ser Ser Asp Ile Val Met Thr Gln Thr Pro Leu Ser Leu Ser Val
            20                  25                  30
Thr Pro Gly Gln Pro Ala Ser Ile Ser Cys Arg Ser Ser Gln Ser Leu
        35                  40                  45
Val His Ser Asn Gly Asp Thr Tyr Leu His Trp Tyr Leu Gln Lys Pro
    50                  55                  60
Gly Gln Pro Pro Gln Leu Leu Ile Tyr Glu Ile Ser Asn Arg Phe Ser
65                  70                  75                  80
Gly Val Pro Asp Arg Phe Ser Gly Ser Gly Ser Gly Thr Asp Phe Thr
                85                  90                  95
Leu Lys Ile Ser Arg Val Glu Ala Glu Asp Val Gly Val Tyr Tyr Cys
            100                 105                 110
Ser Gln Ser Thr His Val Pro Phe Thr Phe Gly Gln Gly Thr Lys Leu
        115                 120                 125
Glu Ile Lys Arg

    130

<210> 17
<211> 396
<212> DNA
<213> Mus musculus

<400> 17
atgaagttgc ctgttaggct gttggtgctg atgttctgga ttcctgtttc cagtagtgat 60
atcgtgatga cccagactcc actctccctg tctgtcactc cgggtcaacc ggcctccatc 120
tcttgccgtt ctagtcagag ccttgtacac agtaatggtg acacctattt acattggtac 180
ttacagaagc caggccagcc tccacaactc ctgatctacg aaatttccaa ccgcttttct 240
ggggtcccag accgtttcag tggcagtggt tcagggacag atttcacact caagatcagc 300
cgcgtggagg ctgaggatgt gggtgtttat tactgctctc aaagtacaca tgttccgttc 360
acgttcggtc aggggaccaa gctggaaatt aaacgt 396

<210> 18
<211> 135
<212> PRT
<213> Mus musculus

<400> 18
Met Lys Cys Ser Trp Val Ile Phe Phe Leu Met Ala Val Val Ile Gly
1               5                   10                  15
Ile Thr Ser Gln Val Gln Leu Val Gln Ser Gly Ala Glu Val Lys Lys
            20                  25                  30
Pro Gly Ser Ser Val Lys Val Ser Cys Lys Ala Ser Gly Phe Asn Ile
        35                  40                  45
Lys Asp Tyr Tyr Met His Trp Val Arg Gln Ala Pro Gly Gln Gly Leu
    50                  55                  60
Glu Trp Met Gly Trp Asn Asp Pro Glu Thr Gly Asp Thr Glu Tyr Ala
65                  70                  75                  80
Pro Lys Phe Gln Gly Arg Val Thr Ile Thr Ala Asp Glu Ser Thr Asn
                85                  90                  95
Thr Ala Tyr Met Glu Leu Ser Ser Leu Arg Ser Glu Asp Thr Ala Val
            100                 105                 110
Tyr Tyr Cys Asn Glu Gly Ser Gly Leu Ile Pro Tyr Trp Gly Gln Gly
        115                 120                 125
Thr Leu Val Thr Val Ser Ser
    130                 135

<210> 19
<211> 405
<212> DNA
<213> Mus musculus

<400> 19
atgaaatgca gctgggtcat cttcttcctg atggcagtgg ttataggaat cacttcacag 60
gttcagctgg tccagtctgg ggcagaggtt aaaaagccag ggtcctcagt caaagtgtcc 120
tgcaaggctt ctggcttcaa cattaaagac tactatatgc actgggtgag acaggcgcct 180
ggtcagggcc tggagtggat gggatggaat gatcctgaga ctggtgatac tgaatatgcc 240
ccgaagttcc agggcagggt cactattact gcagacgaat ccaccaacac agcctacatg 300
gagctctcat ccctgcgttc tgaggacact gccgtctatt actgtaatga gggctcgggc 360
ttgattcctt actggggcca agggactctg gtcaccgtct cgagc 405

<210> 20
<211> 132
<212> PRT
<213> Mus musculus

<400> 20

Met Lys Leu Pro Val Arg Leu Leu Val Leu Met Phe Trp Ile Pro Val
1               5                   10                  15
Ser Ser Ser Asp Val Val Leu Thr Gln Thr Pro Leu Ser Leu Pro Val
            20                  25                  30
Ser Leu Gly Asp Gln Ala Ser Ile Ser Cys Arg Ser Ser Gln Ser Leu
        35                  40                  45
Val His Ser Asn Gly Asp Val Tyr Leu His Trp Tyr Leu Gln Lys Pro
    50                  55                  60
Gly Gln Ser Pro Lys Leu Leu Ile Tyr Glu Val Ser Asn Arg Phe Ser
65                  70                  75                  80
Gly Val Pro Asp Arg Phe Ser Gly Ser Gly Ser Gly Thr Asp Phe Thr
                85                  90                  95
Leu Lys Ile Ser Arg Val Glu Ala Glu Asp Leu Gly Val Tyr Phe Cys
            100                 105                 110
Ser Gln Ser Thr His Val Pro Phe Thr Phe Gly Gly Gly Thr Lys Leu
        115                 120                 125
Glu Ile Lys Arg
    130

<210> 21
<211> 393
<212> DNA
<213> Mus musculus

<400> 21
atgaagttgc ctgttaggct gttggtgctg atgttctgga ttcctgtttc cagcagtgat 60
gttgtattga cccaaactcc actctccctg cctgtcagtc ttggagatca agcctccatc 120
tcttgtagat ctagtcagag ccttgtacac agtaatggag acgtctattt acattggtac 180
ctacagaagc caggccagtc tccaaagctc ctgatctacg aagtttccaa ccgattttct 240
ggggtcccag acaggttcag tggcagtgga tcagggacag atttcacact caagatcagc 300
agagtggagg ctgaggatct gggagtttat ttctgctctc aaagtacaca tgttccgttc 360
acgttcggag gggggaccaa gctggaaata aaa                              393

<210> 22
<211> 135
<212> PRT
<213> Mus musculus

<400> 22
Met Gly Trp Ser Trp Val Ile Phe Phe Leu Met Ala Val Val Ile Gly
1               5                   10                  15
Ile Asn Ser Glu Val Gln Leu Gln Gln Ser Gly Ala Glu Leu Val Arg
            20                  25                  30
Ser Gly Ala Ser Val Arg Leu Ser Cys Thr Ala Ser Gly Phe Asn Ile
        35                  40                  45
Lys Asp Tyr Tyr Met His Trp Val Arg Gln Arg Pro Glu Gln Gly Leu
    50                  55                  60
Glu Trp Ile Gly Trp Asn Asp Pro Glu Thr Gly Asp Thr Glu Tyr Ala
65                  70                  75                  80
Pro Lys Phe Gln Gly Lys Ala Thr Met Thr Ser Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Glu Gly Ser Gly Leu Ile Pro Tyr Trp Gly Pro Gly
        115                 120                 125
Thr Leu Val Thr Val Ser Ser
    130                 135

<210> 23
<211> 402
<212> DNA

<213> Mus musculus

<400> 23
atgggatgga gctgggtcat cttcttcctg atggcagtgg ttataggaat caattcagag 60
gttcagctgc agcagtctgg gacagagctt gtgaggtcag gggcctcagt caagttggcc 120
tgcacagctt ctggcttcaa cattaaaaac tactatatgc actgggtgag gcagaggcct 180
gaacagggcc tggagtggat tggatggatt gatcctgaga atggtgatac tgagtatgcc 240
ccgaagttcc aggacaaggc cactatgact gcagacacat cctccaacac agcctacctg 300
cagctcagca gcctgacatc tgaggacact gccgtctatt actgtaatga gggctcgggc 360
ttgattcctt actggggccc agggactctg gtcactgtct cg 402

<210> 24
<211> 126
<212> PRT
<213> Mus musculus

<400> 24
Met Lys Leu Pro Val Arg Leu Leu Val Leu Met Phe Trp Ile Pro Ala
1               5                   10                  15
Ser Ser Ser Asp Ile Val Met Thr Gln Ser His Lys Phe Met Ser Thr
            20                  25                  30
Ser Val Gly Gly Arg Val Ser Ile Thr Cys Lys Ala Ser Gln Asp Val
        35                  40                  45
Asp Thr Ser Val Ala Trp Tyr Gln Gln Lys Pro Gly Gln Ala Pro Lys
    50                  55                  60
Leu Leu Val Tyr Trp Ala Ser Thr Arg His Thr Gly Val Pro Asp Arg
65                  70                  75                  80
Phe Thr Gly Ser Gly Ser Gly Thr Asp Phe Thr Leu Thr Ile Ser Asn
                85                  90                  95
Val Gln Ser Glu Asp Leu Ala Asp Tyr Phe Cys Gln Gln Tyr Ser Asn
            100                 105                 110
Tyr Pro Thr Phe Gly Ala Gly Thr Lys Leu Glu Leu Lys Arg
        115                 120                 125

<210> 25
<211> 393
<212> DNA
<213> Mus musculus

<400> 25
atgaagttgc ctgttaggct gttggtgctg atgttctgga ttcctgcttc cagcagtggt 60
gttgtgatga cccaaactcc actctccctg cctgtcagtc ttggagatca agcctccatc 120
tcgtgcagat ctagtcagag ccttgttcac agtaatggaa acacctattt atattggtac 180
ctacagaagc caggccagtc tccaaagctc ctgctcttca aagtttccac ccgattttct 240
ggggtcccag acaggttcag tggcagtgga tcagggacag atttcacact caagatcacc 300
agagtagagg ctgaggatct gggaatttat ttctgctctc aaagttcaca tattcctccg 360
acgttcggtg gaggcaccaa gttggagctc aaa 393

<210> 26
<211> 133
<212> PRT
<213> Mus musculus

<400> 26
Met Asp Trp Leu Trp Thr Leu Leu Phe Leu Met Ala Ala Ala Gln Ser
1               5                   10                  15
Ala Gln Ala Gln Ile Gln Leu Val Gln Ser Gly Pro Glu Leu Lys Lys
            20                  25                  30
Pro Gly Glu Thr Val Lys Ile Ser Cys Lys Ala Ser Gly Tyr Thr Val
        35                  40                  45
Thr Asp Tyr Gly Met Asn Trp Val Lys Gln Ser Pro Gly Lys Asp Leu
    50                  55                  60

Lys Trp Met Gly Trp Ile Asp Thr Tyr Thr Glu Lys Pro Thr Tyr Ala
65                  70                  75                  80
Asp Asp Phe Lys Gly Arg Phe Ala Phe Ser Leu Glu Thr Ser Ala Ser
                85                  90                  95
Thr Ala Tyr Leu Gln Ile Ser Asn Leu Lys Asn Glu Asp Ser Ala Ala
            100                 105                 110
Tyr Phe Cys Leu Arg Ser Asn Phe Asp Phe Trp Gly Gln Gly Thr Thr
        115                 120                 125
Leu Thr Val Ser Ser
    130

<210> 27
<211> 396
<212> DNA
<213> Mus musculus

<400> 27
atggattggc tgtggacctt gctattcctg atggcagctg cccaaagtgc ccaagcacag 60
atccagttgg tgcagtctgg acctgagctg aagaagcctg gagagacagt caagatctcc 120
tgcaaggctt ctgggtatac cgtcacagac tatggaatga actgggtgaa gcagagtcca 180
ggaaaggatt taaagtggat gggctggata gacacctaca ctgaaaagcc aacatatgct 240
gatgacttca agggacgatt tgccttctct ttggaaacct ctgccagcac tgcctacttg 300
cagatcagca acctcaaaaa tgaggactcg gctgcatatt tctgtctaag atccaatttt 360
gacttttggg gccaaggcac cactctcaca gtctcg                           396

<210> 28
<211> 127
<212> PRT
<213> Mus musculus

<400> 28
Met Glu Ser Gln Ile Gln Val Phe Val Tyr Met Leu Leu Trp Leu Ser
 1               5                  10                  15
Gly Val Glu Gly Asp Ile Val Met Thr Gln Ser His Lys Phe Met Ser
            20                  25                  30
Thr Ser Val Gly Gly Arg Val Ser Ile Thr Cys Lys Ala Ser Gln Asp
        35                  40                  45
Val Asp Thr Ser Val Ala Trp Tyr Gln Gln Lys Pro Gly Gln Ala Pro
    50                  55                  60
Lys Leu Leu Val Tyr Trp Ala Ser Thr Arg His Thr Gly Val Pro Asp
65                  70                  75                  80
Arg Phe Thr Gly Ser Gly Ser Gly Thr Asp Phe Thr Leu Thr Ile Ser
                85                  90                  95
Asn Val Gln Ser Glu Asp Leu Ala Asp Tyr Phe Cys Gln Gln Tyr Ser
            100                 105                 110
Asn Tyr Pro Thr Phe Gly Ala Gly Thr Lys Leu Glu Leu Lys Arg
        115                 120                 125

<210> 29
<211> 378
<212> DNA
<213> Mus musculus

<400> 29
atggagtcac agattcaggt ctttgtatac atgttgctgt ggttgtctgg tgttgaagga 60
gacattgtga tgacccagtc tcacaaattc atgtccacat cagtaggagg cagggtcagc 120
atcacctgca aggccagtca ggatgtggat acttctgtag cctggtatca acagaaacca 180
gggcaagctc ctaaacttct ggtttactgg gcatccaccc gccacactgg agtccctgat 240
cgcttcacag gcagtggatc tgggacagat ttcactctca ccattagcaa tgtgcagtct 300
gaagacttgg cagattattt ctgtcagcaa tatagcaact atcccacgtt cggtgctggg 360
accaagctgg aactgaaa                                               378

<210> 30
<211> 139
<212> PRT
<213> Mus musculus

<400> 30
Met Lys Cys Ser Trp Val Ile Phe Phe Leu Met Ala Val Val Thr Gly
1               5                   10                  15
Val Asn Ser Glu Val Gln Leu Gln Gln Ser Gly Ala Glu Leu Val Arg
            20                  25                  30
Pro Gly Ala Ser Val Lys Leu Ser Cys Lys Ala Ser Gly Phe Asn Ile
        35                  40                  45
Lys Asp Tyr Tyr Met His Gly Val Lys His Arg Pro Glu Gln Gly Leu
    50                  55                  60
Glu Trp Ile Gly Arg Ile Asp Pro Glu Asn Gly Asn Thr Ile Tyr Asp
65                  70                  75                  80
Pro Lys Phe Gln Gly Lys Ala Thr Ile Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Ala Arg Ser Pro Tyr Asp Tyr His Ala Trp Phe Ala Tyr
        115                 120                 125
Trp Gly Gln Gly Thr Leu Val Thr Val Ser Ser
    130                 135

<210> 31
<211> 414
<212> DNA
<213> Mus musculus

<400> 31
atgaaatgca gctgggtcat cttcttcctg atggcagtgg ttacaggggt caattcagag 60
gttcagctgc agcagtctgg ggctgagctt gtgaggccag gggcctcagt caagttgtcc 120
tgcaaagctt ctggcttcaa cattaaagac tactatatgc acggggtgaa acataggcct 180
gaacagggcc tggagtggat tggaaggatt gatcctgaaa atggtaatac tatatatgac 240
ccgaagttcc agggcaaggc cactataaca gcagacacat cctccaacac agcctacctg 300
cagctcagca gcctgacatc tgaggacact gccgtctatt actgtgctag atccccttat 360
gattaccacg cctggtttgc ttactggggc caagggactc tggtcaccgt ctcg       414

<210> 32
<211> 128
<212> PRT
<213> Mus musculus

<400> 32
Met Met Ser Ser Ala Gln Phe Leu Gly Ile Leu Leu Leu Trp Phe Pro
1               5                   10                  15
Gly Ile Lys Cys Asp Ile Lys Met Thr Gln Ser Pro Ser Ser Met Tyr
            20                  25                  30
Thr Ser Leu Gly Glu Arg Val Thr Ile Thr Cys Lys Ala Ser Gln Asp
        35                  40                  45
Ile Ser Asn Tyr Phe Thr Trp Phe Gln Gln Lys Pro Gly Lys Ser Pro
    50                  55                  60
Lys Thr Leu Ile Tyr Arg Ala Asn Arg Leu Val Asp Gly Val Pro Ser
65                  70                  75                  80
Arg Phe Ser Gly Ser Gly Ser Gly Gln Asp Phe Ser Leu Thr Ile Ser
                85                  90                  95
Ser Leu Glu Tyr Glu Asp Met Gly Ile Tyr Tyr Cys Leu Gln Tyr Asp
            100                 105                 110
Glu Phe Pro Tyr Thr Phe Gly Gly Gly Thr Lys Leu Glu Leu Lys Arg
        115                 120                 125

<210> 33
<211> 381
<212> DNA
<213> Mus musculus

<400> 33
atgatgtcct ctgctcagtt tcttggaatc ttgttgctct ggtttccagg tatcaaatgt 60
gacatcaaga tgacccagtc tccatcttcc atgtatacat ctctaggcga gagagtcact 120
atcacttgca aggcgagtca ggacattagt aactatttta cctggttcca gcagaaacca 180
gggaaatctc ctaagaccct gatctatcgt gcaaacagat tggtagatgg ggtcccatca 240
aggttcagtg gcagtggatc tgggcaagat ttttctctca ccatcagcag cctcgaatat 300
gaagatatgg gaatttatta ttgtctacag tatgatgagt ttccgtacac gttcggaggg 360
gggaccaagt tggagctcaa a 381

<210> 34
<211> 136
<212> PRT
<213> Mus musculus

<400> 34
Met Asn Phe Gly Leu Ser Leu Ile Phe Leu Val Leu Val Leu Lys Gly
1               5                   10                  15
Val Leu Cys Glu Val Lys Leu Val Glu Ser Gly Gly Gly Val Met Gln
            20                  25                  30
Pro Gly Gly Ser Leu Lys Leu Ser Cys Ala Ala Ser Gly Phe Thr Phe
        35                  40                  45
Arg Thr Tyr Thr Met Ser Trp Val Arg Gln Thr Pro Glu Lys Arg Leu
    50                  55                  60
Glu Trp Val Ala Tyr Ile Ser Asp Gly Gly Gly Ser Ser Tyr Phe Pro
65                  70                  75                  80
Asp Thr Val Lys Gly Arg Phe Thr Val Ser Arg Asp Asn Ala Lys Asn
                85                  90                  95
Thr Leu Tyr Leu Gln Met Ser Ser Leu Arg Ser Glu Asp Thr Ala Met
            100                 105                 110
Tyr Tyr Cys Thr Arg His Ser Asn Trp Tyr Phe Asp Val Trp Gly Ala
        115                 120                 125
Gly Thr Ser Val Thr Val Ser Ser
    130                 135

<210> 35
<211> 405
<212> DNA
<213> Mus musculus

<400> 35
atgaacttcg ggctcagcct gattttcctt gtccttgttt taaaaggtgt cctgtgtgaa 60
gtgaagttgg tggagtctgg gggaggtgta atgcagcctg gagggtccct gaaactctcc 120
tgtgcagcct ctggtttcac tttcagaacc tataccatgt cttgggttcg ccagactcca 180
gagaagaggc tggagtgggt cgcatatatt agtgatggtg gtggtagttc ctactttcca 240
gacactgtca agggccgatt caccgtctcc agggacaatg ccaagaacac cctatacctg 300
caaatgagca gtctgaggtc tgaggacacg gccatgtatt actgtacaag acattctaac 360
tggtacttcg atgtctgggg cgcagggacc tcagtcaccg tctcg 405

<210> 36
<211> 128
<212> PRT
<213> Mus musculus

<400> 36
Met Met Ser Ser Ala Gln Phe Leu Gly Leu Leu Leu Leu Cys Phe Gln
1               5                   10                  15
Gly Thr Arg Cys Asp Ile Gln Met Thr Gln Thr Thr Ser Ser Leu Ser

20                          25                          30
Ala Ser Leu Gly Asp Arg Val Thr Ile Arg Cys Arg Ala Ser Gln Asp
        35                          40                          45
Ile Ser Asn Tyr Leu Asn Trp Tyr Gln Gln Lys Pro Asp Gly Pro Val
    50                          55                          60
Lys Leu Leu Ile Tyr Tyr Thr Ser Thr Leu Thr Ser Gly Val Pro Ser
65                          70                          75                          80
Arg Phe Ser Gly Ser Gly Ser Gly Thr Asp Tyr Ser Leu Thr Ile Ser
                    85                          90                          95
Asn Leu Asp Gln Glu Asp Ile Ala Thr Tyr Phe Cys Gln Gln Gly Lys
                100                         105                         110
Thr Phe Pro Phe Thr Phe Gly Ser Gly Thr Lys Leu Glu Ile Lys Arg
        115                         120                         125

<210> 37
<211> 381
<212> DNA
<213> Mus musculus

<400> 37
atgatgtcct ctgctcagtt ccttggtctc ctgttgctct gttttcaagg taccagatgt 60
gatatccaga tgacacagac tacatcctcc ctgtctgcct ctctgggaga cagagtcacc 120
atccgttgca gggcaagtca ggacattagc aattatttaa actggtatca gcagaaacca 180
gatggacctg ttaaactcct gatctactac acatctacat tgacctcagg agtcccatca 240
aggttcagtg gcagtgggtc tggaacagat tattctctca ccattagcaa cctggaccaa 300
gaagatattg ccacttactt ttgccaacag ggtaagacgt ttccattcac gttcggctcg 360
gggacaaagt tggaaataaa a                                              381

<210> 38
<211> 139
<212> PRT
<213> Mus musculus

<400> 38
Met Glu Trp Ser Gly Val Phe Ile Phe Leu Leu Ser Val Thr Ala Gly
 1               5                  10                  15
Val His Ser Gln Val Gln Leu Gln Gln Ser Gly Ala Glu Leu Val Arg
                20                          25                          30
Pro Gly Thr Ser Val Lys Val Ser Cys Lys Ala Ser Gly Tyr Ala Phe
        35                          40                          45
Thr Asn Tyr Phe Ile Glu Trp Val Lys Gln Arg Pro Gly Gln Gly Leu
    50                          55                          60
Glu Trp Ile Gly Ala Ile Asn Pro Gly Ser Gly Gly Thr Asn Tyr Asn
65                          70                          75                          80
Glu Arg Phe Lys Gly Lys Ala Thr Leu Ser Ala Asp Lys Ser Ser Ser
                    85                          90                          95
Thr Ala Tyr Met Gln Leu Ser Ser Leu Thr Ser Asp Asp Ser Ala Val
                100                         105                         110
Tyr Phe Cys Ala Arg Glu Asp Tyr Gly Asp Val Tyr Ala Met Asp Tyr
        115                         120                         125
Trp Gly Gln Gly Thr Ser Val Thr Val Ser Ser
    130                         135

<210> 39
<211> 414
<212> DNA
<213> Mus musculus

<400> 39
atggaatgga gcggggtctt tatctttctc ctgtcagtaa ctgcaggtgt tcactcccag 60
gtccagctgc agcagtctgg agctgagctg gtcaggcctg ggacttcagt gaaggtgtcc 120

tgcaaggctt ctggatacgc cttcactaat tacttcatag agtgggtaaa acagaggcct 180
ggacagggcc ttgagtggat tggagcgatt aatcctggaa gtggtggtac taactacaat 240
gagaggttca agggcaaggc aactctgtct gcagacaaat cctccagcac tgcctacatg 300
cagctcagca gcctgacatc tgatgactct gcggtctatt tctgtgcaag agaggactat 360
ggtgatgtct atgctatgga ctactggggt caaggaacct cggtcacagt ctcg 414

<210> 40
<211> 131
<212> PRT
<213> Mus musculus

<400> 40
Met Gly Ile Lys Met Glu Ser His Ser Gln Val Phe Val Tyr Met Leu
1               5                   10                  15
Leu Trp Leu Ser Gly Val Glu Gly Asp Ile Val Met Thr Gln Ser His
            20                  25                  30
Lys Phe Met Ser Thr Ser Val Gly Asp Arg Val Ser Ile Thr Cys Lys
        35                  40                  45
Ala Ser Gln Asp Val Asp Thr Ser Val Ala Trp Tyr Gln Gln Lys Pro
    50                  55                  60
Gly Gln Ala Pro Lys Leu Leu Ile Tyr Trp Ala Ser Thr Arg His Thr
65                  70                  75                  80
Gly Val Pro Asp Arg Phe Thr Gly Ser Gly Ser Gly Thr Asp Phe Thr
                85                  90                  95
Leu Thr Ile Ser Asn Val Gln Ser Glu Asp Leu Ala Asp Tyr Phe Cys
            100                 105                 110
Gln Gln Tyr Ser Ser Tyr Pro Thr Phe Gly Ala Gly Thr Lys Leu Glu
        115                 120                 125
Leu Lys Arg
    130

<210> 41
<211> 390
<212> DNA
<213> Mus musculus

<400> 41
atgggcatca agatggagtc acattctcag gtctttgtat acatgttgct gtggttgtct 60
ggtgttgaag gagacattgt gatgacccag tctcacaaat tcatgtccac atcagtagga 120
gacagggtca gcatcacctg caaggccagt caggatgtgg atacttctgt agcctggtat 180
caacagaaac cagggcaagc tcctaaacta ctgatttact gggcatccac ccggcacact 240
ggagtccctg atcgcttcac aggcagtgga tctgggacag atttcactct caccattagc 300
aatgtgcagt ctgaagactt ggcagattat ttctgtcagc aatatagcag ctatcccacg 360
ttcggtgctg ggaccaagct ggaactgaaa 390

<210> 42
<211> 139
<212> PRT
<213> Mus musculus

<400> 42
Met Glu Trp Ser Gly Val Ile Phe Phe Leu Met Ala Val Val Thr Gly
1               5                   10                  15
Val Asn Ser Glu Val Gln Leu Gln Gln Ser Gly Ala Glu Leu Val Arg
            20                  25                  30
Pro Gly Ala Ser Val Lys Leu Ser Cys Lys Ala Ser Gly Phe Asn Ile
        35                  40                  45
Lys Asp Tyr Tyr Met His Gly Val Lys His Arg Pro Glu Gln Gly Leu
    50                  55                  60
Glu Trp Ile Gly Arg Ile Asp Pro Glu Asn Gly Asn Thr Ile Tyr Asp
65                  70                  75                  80
Pro Lys Phe Gln Gly Lys Ala Thr Ile Thr Ala Asp Thr Ser Ser Asn

                85                  90                  95
Thr Ala Tyr Leu Gln Leu Ser Ser Leu Thr Ser Glu Asp Thr Ala Val
            100                 105                 110
Tyr Tyr Cys Ala Arg Ser Pro Tyr Asp Tyr His Ala Trp Phe Ala Tyr
        115                 120                 125
Trp Gly Gln Gly Thr Leu Val Thr Val Ser Ser
    130                 135

<210> 43
<211> 414
<212> DNA
<213> Mus musculus

<400> 43
atggaatgga gcggagtcat cttcttcctg atggcagtgg ttacaggggt caattcagag 60
gttcagctgc agcagtctgg ggctgagctt gtgaggccag gggcctcagt caagttgtcc 120
tgcaaagctt ctggcttcaa cattaaagac tactatatgc acggggtgaa acataggcct 180
gaacagggcc tggagtggat tggaaggatt gatcctgaga atggtaatac tatatatgac 240
ccgaagttcc agggcaaggc cactataaca gcagacacat cctccaacac agcctacctg 300
cagctcagca gcctgacatc tgaggacact gccgtctatt actgtgctag atccccttat 360
gattaccacg cctggtttgc ttactggggc caagggactc tggtcactgt ctcg       414

<210> 44
<211> 131
<212> PRT
<213> Mus musculus

<400> 44
Met Gly Ile Lys Met Glu Ser His Ser Gln Val Phe Val Tyr Met Leu
 1               5                  10                  15
Leu Trp Leu Ser Gly Val Glu Gly Asp Ile Gln Met Thr Gln Ser Pro
            20                  25                  30
Ser Ser Leu Ser Ala Ser Val Gly Asp Arg Val Thr Ile Thr Cys Lys
        35                  40                  45
Ala Ser Gln Asp Val Asp Thr Ser Val Ala Trp Tyr Gln Gln Lys Pro
    50                  55                  60
Gly Lys Ala Pro Lys Leu Leu Ile Tyr Trp Ala Ser Thr Arg His Thr
65                  70                  75                  80
Gly Val Pro Asp Arg Phe Ser Gly Ser Gly Ser Gly Thr Asp Phe Thr
                85                  90                  95
Leu Thr Ile Ser Ser Leu Gln Pro Glu Asp Phe Ala Thr Tyr Tyr Cys
            100                 105                 110
Gln Gln Tyr Ser Ser Tyr Pro Thr Phe Gly Gln Gly Thr Lys Leu Glu
        115                 120                 125
Ile Lys Arg
    130

<210> 45
<211> 393
<212> DNA
<213> Mus musculus

<400> 45
atgggcatca agatggagtc acattctcag gtctttgtat acatgttgct gtggttgtct 60
ggtgttgaag gagatatcca gatgacccag tctccatcct ccctgtctgc atctgtaggc 120
gaccgtgtca ccatcacttg caaggcaagt caggacgttg atacctctgt agcttggtat 180
cagcagaaac cagggaaagc ccctaagctc ctgatctatt gggcatccac tcgtcatact 240
ggggtcccag accgcttcag tggcagtggt tctgggacag atttcactct caccatcagc 300
agtctgcaac ctgaagattt tgcaacttat tactgtcagc aatatagcag ttaccctacg 360
ttcggtcagg ggaccaagct ggaaattaaa cgt                              393

<210> 46
<211> 139
<212> PRT
<213> Mus musculus

<400> 46
Met Glu Trp Ser Gly Val Ile Phe Phe Leu Met Ala Val Val Thr Gly
1               5                   10                  15
Val Asn Ser Glu Val Gln Leu Val Gln Ser Gly Ala Glu Val Lys Lys
            20                  25                  30
Pro Gly Ala Thr Val Lys Ile Ser Cys Lys Ala Ser Gly Phe Asn Ile
        35                  40                  45
Lys Asp Tyr Tyr Met His Gly Val Gln His Ala Pro Gly Lys Gly Leu
    50                  55                  60
Glu Trp Ile Gly Arg Ile Asp Pro Glu Asn Gly Asn Thr Ile Tyr Asp
65                  70                  75                  80
Pro Lys Phe Gln Gly Arg Val Thr Ile Thr Ala Asp Thr Ser Thr Asn
                85                  90                  95
Thr Ala Tyr Met Glu Leu Ser Ser Leu Arg Ser Glu Asp Thr Ala Val
            100                 105                 110
Tyr Tyr Cys Ala Arg Ser Pro Tyr Asp Tyr His Ala Trp Phe Ala Tyr
        115                 120                 125
Trp Gly Gln Gly Thr Leu Val Thr Val Ser Ser
    130                 135

<210> 47
<211> 417
<212> DNA
<213> Mus musculus

<400> 47
atggaatgga gcggagtcat cttcttcctg atggcagtgg ttacaggggt caattcagag 60
gtccagctgg tacagtctgg ggctgaggtg aagaagcctg gggctacagt gaaaatctcc 120
tgcaaggctt ctggattcaa catcaaagac tactacatgc acggggtgca acatgcccct 180
ggaaaagggc ttgagtggat tggacgtatt gatcctgaaa atggtaacac aatctacgac 240
ccgaagttcc agggccgcgt caccattacc gcggacacgt ctacaaacac agcctacatg 300
gagctgagca gcctgcgttc tgaggacacg gccgtgtatt actgtgcacg ttctccgtat 360
gattatcacg cctggtttgc ttactggggc caagggactc tggtcaccgt ctcgagc    417

<210> 48
<211> 131
<212> PRT
<213> Mus musculus

<400> 48
Met Lys Leu Pro Val Leu Leu Trp Val Leu Leu Leu Trp Val Pro Gly
1               5                   10                  15
Ser Thr Gly Asp Ile Val Leu Thr Gln Ser Pro Ala Ser Leu Ala Val
            20                  25                  30
Ser Leu Gly Gln Arg Ala Thr Ile Tyr Cys Lys Ala Ser Gln Ser Val
        35                  40                  45
Asp Tyr Asp Gly Asp Ser Tyr Met Asn Trp Tyr Gln Gln Lys Pro Gly
    50                  55                  60
Gln Pro Pro Lys Val Leu Ile Phe Ala Ala Ser Asn Leu Glu Ser Gly
65                  70                  75                  80
Ile Pro Ala Arg Phe Ser Gly Ser Arg Ser Gly Thr Asp Phe Thr Leu
                85                  90                  95
Asn Ile His Pro Val Glu Glu Glu Asp Ala Ala Thr Tyr Tyr Cys Gln
            100                 105                 110
Gln Thr Asn Glu Asp Pro Pro Thr Phe Gly Gly Gly Thr Lys Leu Glu
        115                 120                 125
Leu Lys Arg

    130

<210> 49
<211> 390
<212> DNA
<213> Mus musculus

<400> 49
atgaagctgc ctgttctgct atgggtgctg ctgctctggg ttccaggctc cactggtgac 60
attgtgctga cccaatctcc agcttctttg gctgtgtctc tagggcagag ggccaccatt 120
tactgcaagg ccagccaaag tgttgattat gatggtgata gttatatgaa ctggtaccaa 180
cagaaaccag gacagccacc caaagtcctc atctttgctg catccaatct agaatctggg 240
atcccggcca ggtttagtgg cagtagatct gggacagact tcaccctcaa catccatcct 300
gtggaggagg aggatgctgc aacctattac tgtcaacaaa ctaatgagga tcctccgacg 360
ttcggtggcg gcaccaagct ggaactgaaa 390

<210> 50
<211> 138
<212> PRT
<213> Mus musculus

<400> 50
Met Glu Trp Ser Trp Ile Ile Leu Phe Leu Val Ala Thr Ala Thr Asp
1               5                   10                  15
Val His Ser Gln Val Gln Leu Gln Gln Ser Gly Ala Glu Leu Val Lys
            20                  25                  30
Pro Gly Ala Ser Val Lys Leu Ser Cys Lys Ala Ser Gly Tyr Ala Phe
        35                  40                  45
Ile Ser Phe Trp Ile His Trp Val Lys Gln Arg Pro Gly Gln Gly Leu
    50                  55                  60
Glu Trp Ile Gly Glu Ile Asn Pro Ser Asn Gly Arg Thr Asp Tyr Asn
65                  70                  75                  80
Ala Lys Phe Lys Thr Lys Ala Thr Leu Thr Val Asp Arg Ser Ser Ser
                85                  90                  95
Thr Ala Tyr Met Gln Leu Ser Ser Leu Thr Ser Glu Asp Ser Ala Asp
            100                 105                 110
Tyr Tyr Cys Ala Arg Gly Gly Thr Gly Thr Trp Tyr Phe Asp Val Trp
        115                 120                 125
Gly Ala Gly Thr Thr Val Thr Val Ser Ser
    130                 135

<210> 51
<211> 411
<212> DNA
<213> Mus musculus

<400> 51
atggaatgga gctggatcat cctctttttg gtagcaacag ctacagatgt ccactcccag 60
gtccaactgc agcagtctgg ggctgaactg gtgaagcctg gggcttcagt gaagctgtcc 120
tgcaaggctt ctggctacgc ctttattagc ttctggatac actgggtgaa gcagaggcct 180
ggtcaaggcc ttgagtggat tggagagatt aatcctagca acggtcgtac tgactacaat 240
gcgaagttca agaccaaggc cacactgact gttgacagat cctcctccac agcctacatg 300
caactcagca gcctgacatc tgaggactct gcggactatt actgtgcaag aggaggaact 360
gggacctggt acttcgatgt ctggggcgca gggaccacag tcaccgtctc g 411

<210> 52
<211> 133
<212> PRT
<213> Mus musculus

<400> 52

Met Met Ser Pro Ala Gln Phe Leu Phe Leu Leu Val Leu Trp Ile Arg
1               5                   10                  15
Glu Thr Asn Gly Asp Val Val Met Thr Gln Thr Pro Leu Thr Leu Ser
            20                  25                  30
Val Thr Ile Gly Gln Pro Ala Ser Ile Ser Cys Lys Ser Ser Gln Ser
        35                  40                  45
Leu Leu Asp Ser Asp Gly Glu Thr Tyr Leu Asn Trp Leu Leu Gln Arg
    50                  55                  60
Pro Gly Gln Ser Pro Lys Arg Leu Ile Tyr Leu Val Ser Lys Leu Asp
65                  70                  75                  80
Ser Gly Val Pro Asp Arg Phe Thr Gly Ser Gly Ser Gly Thr Asp Phe
                85                  90                  95
Thr Leu Lys Ile Ser Arg Val Glu Thr Glu Asp Leu Gly Val Tyr Tyr
            100                 105                 110
Cys Trp Gln Gly Thr His Phe Pro Tyr Thr Phe Gly Gly Gly Thr Lys
        115                 120                 125
Leu Glu Leu Lys Arg
    130

<210> 53
<211> 396
<212> DNA
<213> Mus musculus

<400> 53
atgatgagtc ctgcccagtt cctgtttctg ttagtgctct ggattcggga aaccaacggt 60
gatgttgtga tgacccagac tccactcact ttgtcggtta ccattggaca accagcctcc 120
atctcttgca agtcaagtca gagcctctta gatagtgatg gagagacata tttgaattgg 180
ttgttacaga ggccaggcca gtctccaaag cgcctaatct atctggtgtc taaactggac 240
tctggagtcc ctgacaggtt cactggcagt ggatcaggga cagatttcac actgaaaatc 300
agcagagtgg agactgagga tttgggagtt tattattgct ggcaaggtac acattttccg 360
tacacgttcg gaggggggac caagctggaa ctgaaa                           396

<210> 54
<211> 135
<212> PRT
<213> Mus musculus

<400> 54
Met Asn Leu Gly Leu Asn Cys Val Phe Ile Val Phe Leu Leu Lys Gly
1               5                   10                  15
Val Gln Ser Glu Val Lys Leu Glu Glu Ser Gly Gly Gly Leu Val Gln
            20                  25                  30
Pro Gly Gly Ser Met Lys Leu Ser Cys Val Ala Ser Gly Phe Thr Phe
        35                  40                  45
Ser Asn Tyr Trp Met Asn Trp Val Arg Gln Ser Pro Glu Lys Gly Leu
    50                  55                  60
Glu Trp Val Ala Glu Ile Arg Leu Lys Ser Asp Asn Tyr Ala Thr His
65                  70                  75                  80
Phe Ala Glu Ser Val Lys Gly Arg Phe Thr Ile Ser Arg Asp Asp Ser
                85                  90                  95
Lys Ser Ser Val Tyr Leu Gln Met Asn Asn Leu Arg Ala Glu Asp Thr
            100                 105                 110
Gly Ile Tyr Tyr Cys Thr Gly Ile Leu Phe Gly Tyr Trp Gly Gln Gly
        115                 120                 125
Thr Leu Val Thr Val Ser Ser
    130                 135

<210> 55
<211> 402
<212> DNA

<213> Mus musculus

<400> 55
atgaacttgg gactgaactg tgtattcata gtttttctct taaaaggtgt ccagagtgaa 60
gtgaaacttg aggagtctgg aggaggcttg gtgcaacctg gaggatccat gaaactctcc 120
tgtgttgcct ctggattcac tttcagtaac tactggatga actgggtccg ccagtctcca 180
gagaaggggc ttgagtgggt tgctgaaatt agattgaaat ctgataatta tgcaacacat 240
tttgcggagt ctgtgaaagg gaggttcacc atctcaagag atgattccaa aagtagtgtc 300
tacctgcaaa tgaacaactt aagagctgaa gacactggca tttattactg tacgggtatc 360
ctctttggtt actggggcca agggactctg gtcactgtct cg 402

<210> 56
<211> 132
<212> PRT
<213> Mus musculus

<400> 56
Met Glu Ser Asp Thr Ile Leu Leu Trp Val Leu Leu Leu Trp Val Pro
1               5                   10                  15
Gly Ser Thr Gly Asp Ile Val Leu Thr Gln Ser Pro Ala Ser Leu Ala
            20                  25                  30
Val Ser Leu Gly Gln Arg Ala Thr Ile Ser Cys Lys Ala Ser Gln Ser
        35                  40                  45
Val Asp Tyr Asp Gly Asp Ser Tyr Met Asn Trp Tyr Gln Gln Lys Pro
    50                  55                  60
Gly Gln Pro Pro Lys Leu Leu Ile Tyr Ala Ala Ser Asn Leu Glu Ser
65                  70                  75                  80
Gly Ile Pro Ala Arg Phe Ser Gly Ser Gly Ser Gly Thr Asp Phe Thr
                85                  90                  95
Leu Asn Ile His Pro Val Glu Glu Glu Asp Ala Ala Thr Tyr Tyr Cys
            100                 105                 110
Gln Gln Ser Asn Glu Asp Pro Trp Thr Phe Gly Gly Gly Thr Lys Leu
        115                 120                 125
Glu Leu Lys Arg
    130

<210> 57
<211> 393
<212> DNA
<213> Mus musculus

<400> 57
atggagtcag acacaatcct gctatgggtg ctgctgctct gggttccagg ctccactggt 60
gacattgtgc tgacccaatc tccagcttct ttggctgtgt ctctagggca gagggccacc 120
atctcctgca aggccagcca aagtgttgat tatgatggtg atagttatat gaactggtac 180
caacagaaac caggacagcc acccaaactc ctcatctatg ctgcatccaa tctagaatct 240
gggatcccag ccaggtttag tggcagtggg tctgggacag acttcaccct caacatccat 300
cctgtggagg aggaggatgc tgctacctat tactgtcagc aaagtaatga ggatccgtgg 360
acgttcggtg gaggcaccaa gctggagctg aaa 393

<210> 58
<211> 136
<212> PRT
<213> Mus musculus

<400> 58
Met Glu Trp Ser Trp Ile Ile Leu Phe Leu Val Ala Thr Ala Thr Asp
1               5                   10                  15
Val His Ser Gln Val Gln Leu Leu Gln Pro Gly Ala Glu Leu Val Lys
            20                  25                  30
Pro Gly Ala Ser Val Lys Leu Ser Cys Lys Ala Ser Gly Tyr Thr Phe
        35                  40                  45

Thr Ser Tyr Trp Met His Trp Val Lys Gln Arg Pro Gly Gln Gly Leu
    50                  55                  60
Glu Trp Ile Gly Glu Ile Asn Pro Ser Asn Gly Arg Thr Asp Tyr Asn
65                  70                  75                  80
Glu Asn Phe Lys Ser Lys Ala Thr Leu Thr Val Asp Lys Ser Ser Ser
                85                  90                  95
Thr Ala Tyr Met Gln Leu Ser Ser Leu Thr Ser Glu Asp Ser Ala Val
            100                 105                 110
Tyr Tyr Cys Ala Arg Gly Gly Val Tyr Ala Met Asp Tyr Trp Gly Gln
        115                 120                 125
Gly Thr Ser Val Thr Val Ser Ser
    130                 135

<210> 59
<211> 405
<212> DNA
<213> Mus musculus

<400> 59
atggaatgga gctggatcat cctctttttg gtagcaacag ctacagatgt ccactcccag 60
gtccaactgc tgcagcctgg ggctgaactg gtgaagcctg gggcttcagt gaagctgtcc 120
tgcaaggctt ctggctacac cttcaccagc tactggatgc actgggtgaa gcagaggcct 180
gggcaaggcc ttgagtggat tggagagatt aatcctagca acggtcgtac tgactacaat 240
gagaacttca agagcaaggc cacactgact gtagacaaat cctccagcac agcctacatg 300
caactcagca gcctgacatc tgaggactct gcggtctatt actgtgcaag agggggggtc 360
tatgctatgg actactgggg tcaaggaacc tcagtcaccg tctcg 405

<210> 60
<211> 132
<212> PRT
<213> Mus musculus

<400> 60
Met Lys Leu Pro Val Arg Leu Leu Val Leu Met Phe Trp Ile Pro Val
 1               5                  10                  15
Ser Ser Ser Asp Val Val Met Thr Gln Thr Pro Leu Ser Leu Pro Val
            20                  25                  30
Ser Leu Gly Asp Gln Ala Ser Ile Ser Cys Arg Ser Ser Gln Ser Leu
        35                  40                  45
Val His Ser Asn Gly Asp Thr Tyr Leu His Trp Tyr Leu Gln Lys Pro
    50                  55                  60
Gly Gln Ser Pro Lys Leu Leu Ile Tyr Glu Ile Ser Asn Arg Phe Ser
65                  70                  75                  80
Gly Val Pro Asp Arg Phe Ser Gly Ser Gly Ser Gly Thr Asp Phe Thr
                85                  90                  95
Leu Lys Ile Ser Arg Val Glu Ala Glu Asp Leu Gly Val Tyr Phe Cys
            100                 105                 110
Ser Gln Ser Thr His Val Pro Phe Thr Phe Gly Gly Gly Thr Lys Leu
        115                 120                 125
Glu Ile Lys Arg
    130

<210> 61
<211> 393
<212> DNA
<213> Mus musculus

<400> 61
atgaagttgc ctgttaggct gttggtgctg atgttctgga ttcctgtttc cagtagtgat 60
gttgtgatga cccaaactcc actctccctg cctgtcagtc ttggagatca agcctccatc 120
tcttgcagat ctagtcagag ccttgtacac agtaatggag acacctattt acattggtac 180

ctacagaagc caggccagtc tccaaaactc ctgatctacg aaatttccaa ccgattttct 240
ggggtcccag acaggttcag tggcagtgga tcagggacag atttcacact caagatcagc 300
agagtggagg ctgaggatct gggagtttat ttctgctctc aaagtacaca tgttccgttc 360
acgttcggag gggggaccaa gctggaaata aaa 393

<210> 62
<211> 135
<212> PRT
<213> Mus musculus

<400> 62
Met Lys Cys Ser Trp Val Ile Phe Phe Leu Met Ala Val Val Ile Gly
1               5                   10                  15
Ile Thr Ser Glu Ile Gln Leu Gln Gln Ser Gly Thr Glu Leu Val Arg
            20                  25                  30
Ser Gly Ala Ser Val Lys Leu Ser Cys Thr Ala Ser Gly Phe Asn Ile
        35                  40                  45
Lys Asp Tyr Tyr Ile His Trp Val Arg Gln Arg Pro Asp Gln Gly Leu
    50                  55                  60
Glu Trp Ile Gly Trp Ile Asp Pro Glu Asn Gly Asp Thr Glu Tyr Ala
65                  70                  75                  80
Pro Lys Phe Gln Asp Arg Ala Thr Leu Thr Ala Asp Thr Ser Ser Asn
                85                  90                  95
Thr Ala Tyr Leu Gln Leu Thr Leu Leu Thr Ser Glu Asp Thr Ala Val
            100                 105                 110
Tyr Tyr Cys Asn Glu Gly Ser Gly Leu Ile Pro Tyr Trp Gly Gln Gly
        115                 120                 125
Thr Pro Val Thr Val Ser Ser
    130                 135

<210> 63
<211> 402
<212> DNA
<213> Mus musculus

<400> 63
atgaaatgca gctgggtcat cttcttcctg atggcagtgg ttataggaat cacttcagag 60
attcagctgc agcagtctgg gacagagctt gtgaggtcag gggcctcagt caaattgtcc 120
tgcacagctt ctggcttcaa cattaaagac tactatattc actgggtgag acagaggcct 180
gaccagggcc tggagtggat tggatggatt gatcctgaga atggtgatac tgaatatgcc 240
ccgaagttcc aggacagggc cactttgact gcagacacat cctccaacac agcctacctg 300
cagctcacac tcctgacatc tgaggacact gccgtctatt actgtaatga gggctcgggc 360
ttgattcctt actggggcca agggactccg gtcaccgtct cg 402

<210> 64
<211> 128
<212> PRT
<213> Mus musculus

<400> 64
Met Met Ser Ser Ala Gln Phe Leu Gly Leu Leu Leu Leu Cys Phe Gln
1               5                   10                  15
Gly Thr Arg Cys Asp Ile Gln Met Thr Gln Thr Thr Ser Ser Leu Ser
            20                  25                  30
Ala Ser Leu Gly Asp Arg Val Thr Ile Arg Cys Arg Ala Ser Gln Asp
        35                  40                  45
Ile Ser Asn Tyr Leu Asn Trp Tyr Gln Gln Lys Pro Asp Gly Pro Val
    50                  55                  60
Lys Leu Leu Ile Tyr Tyr Thr Ser Thr Leu Thr Ser Gly Val Pro Ser
65                  70                  75                  80
Arg Phe Ser Gly Ser Gly Ser Gly Thr Asp Tyr Ser Leu Thr Ile Ser
                85                  90                  95

Asn Leu Asp Gln Glu Asp Ile Ala Thr Tyr Phe Cys Gln Gln Gly Lys
            100                 105                 110
Thr Phe Pro Phe Thr Phe Gly Ser Gly Thr Lys Leu Glu Ile Lys Arg
        115                 120                 125

<210> 65
<211> 381
<212> DNA
<213> Mus musculus

<400> 65
atgatgtcct ctgctcagtt ccttggtctc ctgttgctct gttttcaagg taccagatgt 60
gatatccaga tgacacagac tacatcctcc ctgtctgcct ctctgggaga cagagtcacc 120
atccgttgca gggcaagtca ggacattagc aattatttaa actggtatca gcagaaacca 180
gatggacctg ttaaactcct gatctactac acatctacat tgacctcagg agtcccatca 240
aggttcagtg gcagtgggtc tggaacagat tattctctca ccattagcaa cctggaccaa 300
gaagatattg ccacttactt ttgccaacag ggtaagacgt ttccattcac gttcggctcg 360
gggacaaagt tggaaataaa a                                           381

<210> 66
<211> 139
<212> PRT
<213> Mus musculus

<400> 66
Met Glu Trp Ser Trp Val Phe Ile Phe Leu Leu Ser Val Thr Ala Gly
 1               5                  10                  15
Val His Ser Gln Val Gln Leu Gln Gln Ser Gly Ala Glu Leu Val Arg
            20                  25                  30
Pro Gly Thr Ser Val Lys Val Ser Cys Lys Ala Ser Gly Tyr Val Phe
        35                  40                  45
Ile Asn Tyr Phe Ile Glu Trp Val Lys Gln Arg Pro Gly Gln Gly Leu
    50                  55                  60
Glu Trp Ile Gly Val Ile Asn Pro Glu Asn Gly Gly Thr Asn Tyr Asn
65                  70                  75                  80
Glu Arg Phe Lys Asp Lys Ala Ala Leu Thr Ala Asp Thr Ser Ser Ser
                85                  90                  95
Thr Ala Tyr Met Gln Leu Ser Ser Leu Thr Ser Asp Asp Ser Ala Val
            100                 105                 110
Tyr Phe Cys Ala Arg Glu Asp Tyr Gly Asp Val Tyr Ala Met Asp Tyr
        115                 120                 125
Trp Gly Gln Gly Thr Ser Val Thr Val Ser Ser
    130                 135

<210> 67
<211> 414
<212> DNA
<213> Mus musculus

<400> 67
atggaatgga gctgggtctt tatctttctc ctgtcagtaa ctgcaggtgt tcactcccag 60
gtccagctgc agcagtctgg agctgaactg gtcaggcctg ggacttcagt gaaggtgtcc 120
tgcaaggctt ctggatacgt cttcattaat tacttcatag agtgggttaa acagaggcct 180
ggacagggcc ttgagtggat tggagtgatt aatcctgaaa atggtggtac taactacaat 240
gagagattca aggacaaggc agcactgact gcagacacat cctccagcac tgcctacatg 300
caactcagca gcctgacatc tgatgactct gcggtctatt tctgtgcaag agaggactat 360
ggtgatgtct atgctatgga ctactggggt caaggaacct cagtcacagt ctcg       414

<210> 68
<211> 132
<212> PRT

<213> Mus musculus

<400> 68
Met Lys Leu Pro Val Arg Leu Leu Val Leu Met Phe Trp Ile Pro Val
1               5                   10                  15
Ser Ser Ser Asp Val Val Met Thr Gln Thr Pro Leu Ser Leu Pro Val
            20                  25                  30
Ser Leu Gly Asp Gln Ala Ser Ile Ser Cys Arg Ser Ser Gln Ser Leu
        35                  40                  45
Val His Ser Asn Gly Asp Thr Tyr Leu His Trp Tyr Leu Gln Lys Pro
    50                  55                  60
Gly Gln Ser Pro Lys Leu Leu Ile Tyr Glu Ile Ser Asn Arg Phe Ser
65                  70                  75                  80
Gly Val Pro Asp Arg Phe Ser Gly Ser Gly Ser Gly Thr Asp Phe Thr
                85                  90                  95
Leu Lys Ile Ser Arg Val Glu Ala Glu Asp Leu Gly Val Tyr Phe Cys
            100                 105                 110
Ser Gln Ser Thr His Val Pro Phe Thr Phe Gly Gly Gly Thr Lys Leu
        115                 120                 125
Glu Ile Lys Arg
    130

<210> 69
<211> 393
<212> DNA
<213> Mus musculus

<400> 69
atgaagttgc ctgttaggct gttggtgctg atgttctgga ttcctgtttc cagtagtgat 60
gttgtgatga cccaaactcc actctccctg cctgtcagtc ttggagatca agcctccatc 120
tcttgcagat ctagtcagag ccttgtacac agtaatggag acacctattt acattggtac 180
ctacagaagc caggccagtc tccaaaactc ctgatctacg aaatttccaa ccgattttct 240
ggggtcccag acaggttcag tggcagtgga tcagggacag atttcacact caagatcagc 300
agagtggagg ctgaggatct gggagtttat ttctgctctc aaagtacaca tgttccgttc 360
acgttcggag gggggaccaa gctggaaata aaa                              393

<210> 70
<211> 135
<212> PRT
<213> Mus musculus

<400> 70
Met Lys Cys Ser Trp Val Ile Phe Phe Leu Met Ala Val Val Ile Gly
1               5                   10                  15
Ile Asn Ser Glu Val Gln Leu Gln Gln Ser Gly Thr Glu Leu Val Arg
            20                  25                  30
Ser Gly Ala Ser Val Arg Leu Ser Cys Thr Ala Ser Gly Phe Asn Ile
        35                  40                  45
Lys Asp Tyr Tyr Ile His Trp Val Lys Gln Arg Pro Glu Gln Gly Leu
    50                  55                  60
Glu Trp Ile Gly Trp Ile Asp Pro Glu Asn Gly Asp Ser Glu Tyr Ala
65                  70                  75                  80
Pro Lys Phe Gln Asp Lys Ala Thr Met Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Glu Gly Ser Gly Leu Ile Pro Tyr Trp Gly His Gly
        115                 120                 125
Thr Leu Val Thr Val Ser Ser
    130                 135

<210> 71
<211> 402
<212> DNA
<213> Mus musculus

<400> 71
atgaaatgca gctgggtcat cttcttcctg atggcagtgg ttataggaat caattcagag 60
gttcagctgc agcagtctgg gacagagctt gtgaggtcag gggcctcagt caggttgtcc 120
tgcacagctt ctggcttcaa cattaaagac tactatatac actgggtgaa acagaggcct 180
gaacagggcc tggagtggat tggatggatt gatcctgaga atggtgattc tgaatatgcc 240
ccgaagttcc aggacaaggc caccatgact gcagacacat cctccaacac agcctacctg 300
cagctcagca gcctgacatc tgaggacact gccgtctatt attgtaatga gggctcgggc 360
ttgattcctt actggggcca cgggactctg gtcacagtct cg 402

<210> 72
<211> 133
<212> PRT
<213> Mus musculus

<400> 72
Met Ser Val Pro Thr Gln Val Leu Gly Leu Leu Leu Leu Trp Leu Thr
1               5                   10                  15
Asp Ala Arg Cys Asp Ile Val Leu Thr Gln Thr Pro Leu Ser Leu Pro
            20                  25                  30
Val Ser Leu Gly Asp His Ala Ser Ile Ser Cys Arg Ser Ser Gln Ser
        35                  40                  45
Leu Val His Ser Asn Gly Asp Val Tyr Phe His Trp Tyr Leu Gln Lys
    50                  55                  60
Pro Gly Gln Ser Pro Lys Leu Leu Ile Tyr Glu Val Ser Asn Arg Phe
65                  70                  75                  80
Ser Gly Val Pro Asp Arg Phe Ser Gly Ser Gly Ser Gly Thr Tyr Phe
                85                  90                  95
Thr Leu Lys Ile Ser Arg Val Glu Ala Glu Asp Leu Gly Val Tyr Phe
            100                 105                 110
Cys Ser Gln Ser Thr His Val Pro Tyr Thr Phe Gly Gly Gly Thr Lys
        115                 120                 125
Leu Glu Leu Lys Arg
    130

<210> 73
<211> 405
<212> DNA
<213> Mus musculus

<400> 73
atgtctgtcc ccacccaagt cctcggactc ctgctgctgt ggcttacaga tgccagatgc 60
gaggttcagc tgcagcagtc tgggacagag cttgtgaggt caggggcctc agtcaggttg 120
tcctgcacag cttctggctt caacattaaa gactactata tacactgggt gaaacagagg 180
cctgaacagg gcctggagtg gattggatgg attgatcctg agaatggtga ttctgaatat 240
gccccgaagt tccaggacaa ggccaccatg actgcagaca catcctccaa cacagcctac 300
ctgcagctca gcagcctgac atctgaggac actgccgtct attattgtaa tgagggctcg 360
ggcttgattc cttactgggg ccacgggact ctggtcacag tctcg 405

<210> 74
<211> 135
<212> PRT
<213> Mus musculus

<400> 74
Met Lys Cys Ser Trp Val Ile Phe Phe Leu Met Ala Val Val Ile Gly
1               5                   10                  15
Ile Asn Ser Glu Val Gln Leu Gln Gln Ser Gly Thr Glu Leu Val Arg

                    20                          25                          30
Ser Gly Ala Ser Val Arg Leu Ser Cys Thr Ala Ser Gly Phe Asn Ile
            35                          40                          45
Lys Asp Tyr Tyr Ile His Trp Val Lys Gln Arg Pro Glu Gln Gly Leu
    50                          55                          60
Glu Trp Ile Gly Trp Ile Asp Pro Glu Asn Gly Asp Ser Glu Tyr Ala
65                          70                          75                          80
Pro Lys Phe Gln Asp Lys Ala Thr Met Thr Ala Asp Thr Ser Ser Asn
                        85                          90                          95
Thr Ala Tyr Leu Gln Leu Ser Ser Leu Thr Ser Glu Asp Thr Ala Val
                    100                         105                         110
Tyr Tyr Cys Asn Glu Gly Ser Gly Leu Ile Pro Tyr Trp Gly His Gly
            115                         120                         125
Thr Leu Val Thr Val Ser Ser
    130                         135

<210> 75
<211> 393
<212> DNA
<213> Mus musculus

<400> 75
atgaaatgca gctgggtcat cttcttcctg atggcagtgg ttataggaat caattcagat 60
attgtgttga cccaaactcc tctctctctg cctgtcagtc ttggagatca tgcctccatc 120
tcttgcagat ctagtcagag ccttgtacac agtaatggcg acgtctattt ccattggtac 180
ctgcagaagc caggccagtc tccaaagctc ctgatctacg aagtttccaa ccgattttct 240
ggggtcccag acaggttcag tggcagtgga tcagggacat atttcacact caagatcagc 300
agagtggagg ctgaggatct gggagtttat ttctgctctc aaagtacaca tgttccgtac 360
actttcggag gggggaccaa gttggagctc aaa                                393

<210> 76
<211> 133
<212> PRT
<213> Mus musculus

<400> 76
Met Ser Val Pro Thr Gln Val Leu Gly Leu Leu Leu Leu Trp Leu Thr
 1                   5                          10                          15
Asp Ala Arg Cys Asp Ile Val Leu Thr Gln Thr Pro Leu Ser Leu Pro
                    20                          25                          30
Val Ser Leu Gly Asp His Ala Ser Ile Ser Cys Arg Ser Ser Gln Ser
            35                          40                          45
Leu Val His Ser Asn Gly Asp Val Tyr Phe His Trp Tyr Leu Gln Lys
    50                          55                          60
Pro Gly Gln Ser Pro Lys Leu Leu Ile Tyr Glu Val Ser Asn Arg Phe
65                          70                          75                          80
Ser Gly Val Pro Asp Arg Phe Ser Gly Ser Gly Ser Gly Thr Tyr Phe
                        85                          90                          95
Thr Leu Lys Ile Ser Arg Val Glu Ala Glu Asp Leu Gly Val Tyr Phe
                    100                         105                         110
Cys Ser Gln Ser Thr His Val Pro Tyr Thr Phe Gly Gly Gly Thr Lys
            115                         120                         125
Leu Glu Leu Lys Arg
    130

<210> 77
<211> 396
<212> DNA
<213> Mus musculus

<400> 77

atgtctgtcc ccacccaagt cctcggactc ctgctgctgt ggcttacaga tgccagatgc 60
gatgttgtgt tgacccaaac tccactctcc ctgcctgtca gtcttggaga tcaagcctcc 120
atctcttgca gatctagtca gagccttgta cacagtaatg gagacgtcta tttacattgg 180
tacctgcaga agccaggcca gtctccaaag ctcctgatct acgaagtttc caaccgattt 240
tctggggtcc cagacaggtt cagtggcagt ggatcaggga cagatttcac actcaagatc 300
aacagagtgg aggctgagga tctgggagtt tatttctgct ctcaaagtac acatgttccg 360
tacacgttcg gaggggggac caagctggaa ctgaaa 396

<210> 78
<211> 135
<212> PRT
<213> Mus musculus

<400> 78
Met Glu Trp Ser Trp Val Ile Phe Phe Leu Met Ala Val Val Ile Gly
1               5                   10                  15
Ile Asn Ser Glu Val Gln Leu Gln Gln Ser Gly Thr Glu Leu Val Arg
            20                  25                  30
Ser Gly Ala Ser Val Lys Leu Ser Cys Thr Ala Ser Gly Phe Asn Ile
        35                  40                  45
Lys Asp Tyr Tyr Val His Trp Val Lys Gln Arg Pro Glu Gln Gly Leu
    50                  55                  60
Glu Trp Ile Gly Trp Ile Asp Pro Asp Asn Gly Asp Ser Glu Tyr Ala
65                  70                  75                  80
Pro Lys Phe Gln Asp Lys Ala Thr Met Thr Ala Asp Thr Ser Ser Asn
                85                  90                  95
Thr Ala Tyr Leu Gln Leu Asn Ser Leu Thr Ser Glu Asp Thr Ala Val
            100                 105                 110
Tyr Tyr Cys Asn Glu Gly Ser Gly Leu Ile Pro Tyr Trp Gly Pro Gly
        115                 120                 125
Thr Leu Val Thr Val Ser Ser
    130                 135

<210> 79
<211> 402
<212> DNA
<213> Mus musculus

<400> 79
atggaatgga gctgggtcat cttcttcctg atggcagtgg ttataggaat caattctgag 60
gttcagctgc agcagtctgg gacagagctt gtgaggtcag gggcctcagt caagttgtcc 120
tgcacagctt ctggcttcaa cattaaagac tactatgtac actgggtgaa gcagaggcct 180
gaacagggcc tggaatggat tggatggatt gatcctgaca atggtgatag tgaatatgcc 240
ccgaagttcc aggacaaggc cactatgact gcagacacat cctccaacac agcctacctt 300
caactcaaca gcctgacttc tgaggacact gccgtctatt attgtaatga gggctcgggc 360
ttgattcctt actggggccc agggactctg gtcacagtct cg 402

<210> 80
<211> 133
<212> PRT
<213> Mus musculus

<400> 80
Met Ser Val Pro Thr Gln Val Leu Gly Leu Leu Leu Leu Trp Leu Thr
1               5                   10                  15
Asp Ala Arg Cys Asp Val Val Met Thr Gln Thr Pro Leu Ser Leu Pro
            20                  25                  30
Val Ser Leu Gly Asp Gln Ala Ser Ile Ser Cys Arg Ser Ser Gln Ser
        35                  40                  45
Leu Val His Ser Asn Gly Asp Val Tyr Leu His Trp Tyr Leu Gln Lys
    50                  55                  60
Pro Gly Gln Ser Pro Lys Leu Leu Ile Tyr Glu Val Ser Asn Arg Phe

65                  70                  75                  80
Ser Gly Val Pro Asp Arg Phe Ser Gly Ser Gly Ser Gly Thr Asp Phe
                85                  90                  95
Thr Leu Lys Ile Ser Arg Val Glu Ala Glu Asp Leu Gly Val Tyr Phe
            100                 105                 110
Cys Ser Gln Thr Thr His Val Pro Tyr Thr Phe Gly Gly Gly Thr Lys
        115                 120                 125
Leu Glu Leu Lys Arg
    130

<210> 81
<211> 405
<212> DNA
<213> Mus musculus

<400> 81
atgtctgtcc ccacccaagt cctcggactc ctgctgctgt ggcttacaga tgccagatgc 60
gagattcagc tgcagcagtc tgggacagag cttgtgaggt caggggcctc agtcaaattg 120
tcctgcacag cttctggctt caacattaaa gactactata ttcactgggt gagacagagg 180
cctgaccagg gcctggagtg gattggatgg attgatcctg agaatggtga tactgaatat 240
gccccgaagt tccaggacag ggccactttg actgcagaca catcctccaa cacagcctac 300
ctgcagctca cactcctgac atctgaggac actgccgtct attactgtaa tgagggctcg 360
ggcttgattc cttactgggg ccaagggact ccggtcaccg tctcg                405

<210> 82
<211> 135
<212> PRT
<213> Mus musculus

<400> 82
Met Lys Cys Ser Trp Val Ile Phe Phe Leu Met Ala Val Val Ile Gly
 1               5                  10                  15
Ile Thr Ser Glu Ile Gln Leu Gln Gln Ser Gly Thr Glu Leu Val Arg
            20                  25                  30
Ser Gly Ala Ser Val Lys Leu Ser Cys Thr Ala Ser Gly Phe Asn Ile
        35                  40                  45
Lys Asp Tyr Tyr Ile His Trp Val Arg Gln Arg Pro Asp Gln Gly Leu
    50                  55                  60
Glu Trp Ile Gly Trp Ile Asp Pro Glu Asn Gly Asp Thr Glu Tyr Ala
65                  70                  75                  80
Pro Lys Phe Gln Asp Arg Ala Thr Leu Thr Ala Asp Thr Ser Ser Asn
                85                  90                  95
Thr Ala Tyr Leu Gln Leu Thr Leu Leu Thr Ser Glu Asp Thr Ala Val
            100                 105                 110
Tyr Tyr Cys Asn Glu Gly Ser Gly Leu Ile Pro Tyr Trp Gly Gln Gly
        115                 120                 125
Thr Pro Val Thr Val Ser Ser
    130                 135

<210> 83
<211> 393
<212> DNA
<213> Mus musculus

<400> 83
atgaaatgca gctgggtcat cttcttcctg atggcagtgg ttataggaat cacttcagat 60
gttgtgatga cccaaactcc actctccctg cctgtcagtc ttggagatca agcctccatc 120
tcctgcagat ctagtcagag ccttgtacac agtaatggag acgtctattt acattggtac 180
ctgcagaagc caggccagtc tccaaaactc ctgatctacg aagtttccaa ccgattttct 240
ggggtcccag acaggttcag tggcagtggt tcagggacag atttcacact caagatcagc 300
agagtggagg ctgaggatct gggagtttat ttctgttctc aaactacaca tgttccgtac 360

acgttcggag gggggaccaa gctggagctg aaa                                393

<210> 84
<211> 133
<212> PRT
<213> Mus musculus

<400> 84
Met Ser Val Pro Thr Gln Val Leu Gly Leu Leu Leu Leu Trp Leu Thr
1               5                   10                  15
Asp Ala Arg Cys Asp Val Val Met Thr Gln Thr Pro Leu Ser Leu Pro
            20                  25                  30
Val Ser Leu Gly Asp Gln Ala Ser Ile Ser Cys Arg Ser Ser Gln Ser
        35                  40                  45
Leu Val His Ser Asn Gly Asp Val Tyr Leu His Trp Tyr Leu Gln Lys
    50                  55                  60
Pro Gly Gln Ser Pro Lys Leu Leu Ile Tyr Glu Val Ser Asn Arg Phe
65                  70                  75                  80
Ser Gly Val Pro Asp Arg Phe Thr Gly Ser Gly Ser Gly Thr Asp Phe
                85                  90                  95
Thr Leu Lys Ile Ser Arg Val Glu Ala Glu Asp Leu Gly Val Phe Phe
            100                 105                 110
Cys Ser Gln Ser Thr His Val Pro Tyr Thr Phe Gly Gly Gly Thr Lys
        115                 120                 125
Leu Glu Leu Lys Arg
    130

<210> 85
<211> 396
<212> DNA
<213> Mus musculus

<400> 85
atgtctgtcc ccacccaagt cctcggactc ctgctgctgt ggcttacaga tgccagatgc 60
gatgttgtga tgacccaaac tccactctcc ctgcctgtca gtcttggaga tcaagcctcc 120
atctcctgca gatctagtca gagccttgta cacagtaatg gagacgtcta tttacattgg 180
tacctgcaga agccaggcca gtctccaaaa ctcctgatct acgaagtttc caaccgattt 240
tctggggtcc cagacaggtt cactggcagt ggttcaggga cagatttcac actcaagatc 300
agcagagtgg aggctgagga tctgggagtt tttttctgct ctcaaagtac acatgttccg 360
tacacgttcg gaggggggac caagctggaa ctgaaa                             396

<210> 86
<211> 135
<212> PRT
<213> Mus musculus

<400> 86
Met Lys Cys Ser Trp Val Ile Phe Phe Leu Met Ala Val Val Ile Gly
1               5                   10                  15
Ile Thr Ser Glu Ile Gln Leu Gln Gln Ser Gly Thr Glu Leu Val Arg
            20                  25                  30
Ser Gly Ala Ser Val Lys Leu Ser Cys Thr Ala Ser Gly Phe Asn Ile
        35                  40                  45
Lys Asp Tyr Tyr Ile His Trp Val Lys Gln Arg Pro Asp Gln Gly Leu
    50                  55                  60
Glu Trp Ile Gly Trp Ile Asp Pro Glu Asn Gly Asp Thr Glu Tyr Ala
65                  70                  75                  80
Pro Lys Phe Gln Asp Arg Ala Thr Met Thr Ala Asp Thr Ser Ser Asn
                85                  90                  95
Thr Ala Tyr Leu Gln Leu Thr Ile Leu Thr Ser Glu Asp Thr Ala Val
            100                 105                 110
Tyr Tyr Cys Asn Glu Gly Ser Gly Leu Ile Pro Tyr Trp Gly Gln Gly

115                 120                 125
Thr Leu Val Thr Val Ser Ser
    130                 135

<210> 87
<211> 402
<212> DNA
<213> Mus musculus

<400> 87
atgaaatgca gctgggtcat cttcttcctg atggcagtgg ttataggaat cacttcagag 60
attcagctgc agcagtctgg gacagagctt gtgaggtcag gggcctcagt caaattgtcc 120
tgcacagctt ctggcttcaa cattaaagac tactatattc actgggtgaa acagaggcct 180
gaccagggcc tggagtggat tggatggatt gatcctgaga atggtgatac tgaatatgcc 240
ccgaagttcc aggacagggc cactatgact gcagacacat cctccaacac agcctacctg 300
cagctcacca tcctgacatc tgaggacact gccgtctatt actgtaatga gggctcgggc 360
ttgattcctt actggggcca agggactctg gtcaccgtct cg                    402

<210> 88
<211> 128
<212> PRT
<213> Mus musculus

<400> 88
Met Ser Val Pro Thr Gln Val Leu Gly Leu Leu Leu Leu Trp Leu Thr
 1               5                  10                  15
Asp Ala Arg Cys Asp Ile Gln Met Asn Gln Ser Pro Ser Ser Leu Ser
            20                  25                  30
Ala Ser Leu Gly Asp Thr Ile Thr Ile Thr Cys His Ala Ser Gln Asn
        35                  40                  45
Ile Asn Val Trp Leu Ser Trp Tyr Gln Gln Lys Pro Gly Asn Ile Pro
    50                  55                  60
Lys Leu Leu Ile Tyr Lys Val Ser Asn Leu His Thr Gly Val Pro Ser
65                  70                  75                  80
Arg Phe Ser Gly Ser Gly Ser Gly Thr Gly Phe Thr Leu Thr Ile Ser
                85                  90                  95
Ser Leu Gln Pro Glu Asp Ile Ala Thr Tyr Tyr Cys Gln Gln Gly Gln
            100                 105                 110
Ser Tyr Pro Leu Thr Phe Gly Gly Gly Thr Lys Leu Glu Leu Lys Arg
        115                 120                 125

<210> 89
<211> 381
<212> DNA
<213> Mus musculus

<400> 89
atgtctgtcc ccacccaagt cctcggactc ctgctgctgt ggcttacaga tgccagatgc 60
gacatccaga tgaaccagtc tccatccagt ctgtctgcat cccttggaga cacaattacc 120
atcacttgcc atgccagtca gaacattaat gtttggttaa gctggtacca gcagaaacca 180
ggaaatattc ctaaactatt gatctataag gtttccaact tgcacacagg cgtcccatca 240
aggtttagtg gcagtggatc tggaacaggt ttcacattaa ccatcagcag cctgcagcct 300
gaagacattg ccacttacta ctgtcaacag ggtcaaagtt atcctctgac gttcggtgga 360
ggcaccaagt tggagctcaa a                                           381

<210> 90
<211> 139
<212> PRT
<213> Mus musculus

<400> 90

Met Gly Trp Asn Trp Ile Ile Phe Phe Leu Met Ala Val Val Thr Gly
1               5                   10                  15
Val Asn Ser Glu Val Gln Leu Gln Gln Ser Gly Ala Glu Leu Val Arg
            20                  25                  30
Pro Gly Ala Leu Val Lys Leu Ser Cys Lys Ala Ser Gly Phe Asn Ile
        35                  40                  45
Lys Asp Tyr Tyr Ile His Trp Val Lys Gln Arg Pro Glu Gln Gly Leu
    50                  55                  60
Glu Trp Ile Gly Arg Ile Asp Pro Glu Asn Gly Asn Thr Ile Tyr Asp
65                  70                  75                  80
Pro Lys Phe Gln Gly Lys Ala Ile Ile Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Ala Arg Cys Asp Asn Asp Pro Gly Ser Glu Met Asp Tyr
        115                 120                 125
Trp Gly Gln Gly Thr Thr Val Thr Val Ser Ser
    130                 135

<210> 91
<211> 414
<212> DNA
<213> Mus musculus

<400> 91
atgggatgga actggatcat cttcttcctg atggcagtgg ttacaggggt caattcagag 60
gttcagctgc agcagtctgg ggctgagctt gtgaggccag gggccttagt caaattgtcc 120
tgcaaagctt ctggattcaa cattaaagac tactatatac actgggtgaa gcagaggcct 180
gaacagggcc tggagtggat tggaaggatt gatcctgaaa atggtaatac tatatatgac 240
ccgaagttcc agggcaaggc cattataaca gcagacacat cctccaacac agcctacctg 300
cagctcagca gcctgacatc tgaggacact gccgtctatt actgtgctag atgtgataac 360
gaccccggct ctgaaatgga ctactggggt caaggaacca cggtcaccgt ctcg 414

<210> 92
<211> 133
<212> PRT
<213> Mus musculus

<400> 92
Met Ser Val Pro Thr Gln Val Leu Gly Leu Leu Leu Leu Trp Leu Thr
1               5                   10                  15
Asp Ala Arg Cys Asp Ile Val Leu Thr Gln Thr Pro Leu Ser Leu Pro
            20                  25                  30
Val Ser Leu Gly Asp His Ala Ser Ile Ser Cys Arg Ser Ser Gln Ser
        35                  40                  45
Leu Val His Ser Asn Gly Asp Val Tyr Phe His Trp Tyr Leu Gln Lys
    50                  55                  60
Pro Gly Gln Ser Pro Lys Leu Leu Ile Tyr Glu Val Ser Asn Arg Phe
65                  70                  75                  80
Ser Gly Val Pro Asp Arg Phe Ser Gly Ser Gly Ser Gly Thr Tyr Phe
                85                  90                  95
Thr Leu Lys Ile Ser Arg Val Glu Ala Glu Asp Leu Gly Val Tyr Phe
            100                 105                 110
Cys Ser Gln Ser Thr His Val Pro Tyr Thr Phe Gly Gly Gly Thr Lys
        115                 120                 125
Leu Glu Leu Lys Arg
    130

<210> 93
<211> 396
<212> DNA

<213> Mus musculus

<400> 93
atgtctgtcc ccacccaagt cctcggactc ctgctgctgt ggcttacaga tgccagatgc 60
gatattgtgt tgacccaaac tcctctctct ctgcctgtca gtcttggaga tcatgcctcc 120
atctcttgca gatctagtca gagccttgta cacagtaatg gcgacgtcta tttccattgg 180
tacctgcaga agccaggcca gtctccaaag ctcctgatct acgaagtttc caaccgattt 240
tctggggtcc cagacaggtt cagtggcagt ggatcaggga catatttcac actcaagatc 300
agcagagtgg aggctgagga tctgggagtt tatttctgct ctcaaagtac acatgttccg 360
tacactttcg gaggggggac caagttggag ctcaaa 396

<210> 94
<211> 135
<212> PRT
<213> Mus musculus

<400> 94
Met Glu Trp Ser Trp Val Ile Phe Phe Leu Met Ala Val Val Ile Gly
1               5                   10                  15
Ile Asn Ser Glu Val Gln Leu Gln Gln Ser Gly Thr Glu Leu Val Arg
            20                  25                  30
Ser Gly Ala Ser Val Lys Leu Ser Cys Thr Ala Ser Gly Phe Asn Ile
        35                  40                  45
Lys Asp Tyr Tyr Val His Trp Val Lys Gln Arg Pro Glu Gln Gly Leu
    50                  55                  60
Glu Trp Ile Gly Trp Ile Asp Pro Asp Asn Gly Asp Ser Glu Tyr Ala
65                  70                  75                  80
Pro Lys Phe Gln Asp Lys Ala Thr Met Thr Ala Asp Thr Ser Ser Asn
                85                  90                  95
Thr Ala Tyr Leu Gln Leu Asn Ser Leu Thr Ser Glu Asp Thr Ala Val
            100                 105                 110
Tyr Tyr Cys Asn Glu Gly Ser Gly Leu Ile Pro Tyr Trp Gly Pro Gly
        115                 120                 125
Thr Leu Val Thr Val Ser Ser
    130                 135

<210> 95
<211> 402
<212> DNA
<213> Mus musculus

<400> 95
atggaatgga gctgggtcat cttcttcctg atggcagtgg ttataggaat caattctgag 60
gttcagctgc agcagtctgg gacagagctt gtgaggtcag gggcctcagt caagttgtcc 120
tgcacagctt ctggcttcaa cattaaagac tactatgtac actgggtgaa gcagaggcct 180
gaacagggcc tggaatggat tggatggatt gatcctgaca atggtgatag tgaatatgcc 240
ccgaagttcc aggacaaggc cactatgact gcagacacat cctccaacac agcctacctt 300
caactcaaca gcctgacttc tgaggacact gccgtctatt attgtaatga gggctcgggc 360
ttgattcctt actggggccc agggactctg gtcacagtct cg 402

<210> 96
<211> 106
<212> PRT
<213> Mus musculus

<400> 96
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Cys
            100                 105

<210> 97
<211> 327
<212> PRT
<213> Mus musculus

<400> 97
Ala Ser Thr Lys Gly Pro Ser Val Phe Pro Leu Ala Pro Cys Ser Arg
 1               5                  10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210> 98
<211> 15
<212> PRT

<213> Mus musculus

<400> 98
Lys Ala Ser Gln Ser Val Asp Tyr Ala Gly Asp Asn Tyr Met Asn
1               5                   10                  15


<210> 99
<211> 7
<212> PRT
<213> Mus musculus

<400> 99
Thr Ala Ser Asn Leu Glu Ser
1               5


<210> 100
<211> 9
<212> PRT
<213> Mus musculus

<400> 100
Gln Gln Ser Asn Glu Asp Pro Pro Thr
1               5


<210> 101
<211> 5
<212> PRT
<213> Mus musculus

<400> 101
Gly Tyr Phe Met His
1               5


<210> 102
<211> 17
<212> PRT
<213> Mus musculus

<400> 102
Glu Ile Asn Pro Ser Thr Gly Gly Thr Thr Tyr Asn Gln Arg Phe Lys
1               5                   10                  15
Gly


<210> 103
<211> 10
<212> PRT
<213> Mus musculus

<400> 103
Trp Gly Tyr Asn Pro Tyr Ala Leu Asp Tyr
1               5                   10


<210> 104
<211> 11
<212> PRT
<213> Mus musculus

<400> 104
Lys Ala Ser Gln Asp Ile Asn Lys Tyr Ile Ala
1               5                   10

<210> 105
<211> 7
<212> PRT
<213> Mus musculus

<400> 105
Tyr Thr Ser Thr Leu Gln Pro
1               5

<210> 106
<211> 8
<212> PRT
<213> Mus musculus

<400> 106
Leu Gln Tyr Asp Asn Leu Tyr Thr
1               5

<210> 107
<211> 5
<212> PRT
<213> Mus musculus

<400> 107
Ser Tyr Trp Ile Glu
1               5

<210> 108
<211> 17
<212> PRT
<213> Mus musculus

<400> 108
Glu Ile Phe Pro Arg Asn Gly Ser Thr Tyr Tyr Asn Glu Lys Phe Lys
1               5                   10                  15
Gly

<210> 109
<211> 6
<212> PRT
<213> Mus musculus

<400> 109
Ile Asn Thr Leu Asp Tyr
1               5

<210> 110
<211> 16
<212> PRT
<213> Mus musculus

<400> 110

Arg Ser Ser Gln Ser Leu Val His Ser Asn Gly Asp Thr Tyr Leu His
1               5                   10                  15

<210> 111
<211> 7
<212> PRT
<213> Mus musculus

<400> 111
Glu Ile Ser Asn Arg Phe Ser
1               5

<210> 112
<211> 9
<212> PRT
<213> Mus musculus

<400> 112
Ser Gln Ser Thr His Val Pro Phe Thr
1               5

<210> 113
<211> 5
<212> PRT
<213> Mus musculus

<400> 113
Asp Tyr Tyr Met His
1               5

<210> 114
<211> 17
<212> PRT
<213> Mus musculus

<400> 114
Trp Asn Asp Pro Glu Thr Gly Asp Thr Glu Tyr Ala Pro Lys Phe Gln
1               5                   10                  15
Gly

<210> 115
<211> 7
<212> PRT
<213> Mus musculus

<400> 115
Gly Ser Gly Leu Ile Pro Tyr
1               5

<210> 116
<211> 16
<212> PRT
<213> Mus musculus

<400> 116
Arg Ser Ser Gln Ser Leu Val His Ser Asn Gly Asp Thr Tyr Leu His

1               5                   10                  15


<210> 117
<211> 7
<212> PRT
<213> Mus musculus

<400> 117
Glu Ile Ser Asn Arg Phe Ser
1               5


<210> 118
<211> 9
<212> PRT
<213> Mus musculus

<400> 118
Ser Gln Ser Thr His Val Pro Phe Thr
1               5


<210> 119
<211> 5
<212> PRT
<213> Mus musculus

<400> 119
Asp Tyr Tyr Met His
1               5


<210> 120
<211> 17
<212> PRT
<213> Mus musculus

<400> 120
Trp Asn Asp Pro Glu Thr Gly Asp Thr Glu Tyr Ala Pro Lys Phe Gln
1               5                   10                  15
Gly


<210> 121
<211> 7
<212> PRT
<213> Mus musculus

<400> 121
Gly Ser Gly Leu Ile Pro Tyr
1               5


<210> 122
<211> 16
<212> PRT
<213> Mus musculus

<400> 122
Arg Ser Ser Gln Ser Leu Val His Ser Asn Gly Asp Val Tyr Leu His
1               5                   10                  15

<210> 123
<211> 7
<212> PRT
<213> Mus musculus

<400> 123
Glu Val Ser Asn Arg Phe Ser
1               5


<210> 124
<211> 9
<212> PRT
<213> Mus musculus

<400> 124
Ser Gln Ser Thr His Val Pro Phe Thr
1               5


<210> 125
<211> 5
<212> PRT
<213> Mus musculus

<400> 125
Asn Tyr Tyr Met His
1               5


<210> 126
<211> 17
<212> PRT
<213> Mus musculus

<400> 126
Trp Ile Asp Pro Glu Asn Gly Asp Thr Glu Tyr Ala Pro Lys Phe Gln
1               5                   10                  15
Asp


<210> 127
<211> 7
<212> PRT
<213> Mus musculus

<400> 127
Gly Ser Gly Leu Ile Pro Tyr
1               5


<210> 128
<211> 16
<212> PRT
<213> Mus musculus

<400> 128
Arg Ser Ser Gln Ser Leu Val His Ser Asn Gly Asn Thr Tyr Leu Tyr
1               5                   10                  15

<210> 129
<211> 7
<212> PRT
<213> Mus musculus

<400> 129
Lys Val Ser Thr Arg Phe Ser
1               5


<210> 130
<211> 9
<212> PRT
<213> Mus musculus

<400> 130
Ser Gln Ser Ser His Ile Pro Pro Thr
1               5


<210> 131
<211> 5
<212> PRT
<213> Mus musculus

<400> 131
Asp Tyr Gly Met Asn
1               5


<210> 132
<211> 17
<212> PRT
<213> Mus musculus

<400> 132
Trp Ile Asp Thr Tyr Thr Glu Lys Pro Thr Tyr Ala Asp Asp Phe Lys
1               5                   10                  15
Gly


<210> 133
<211> 5
<212> PRT
<213> Mus musculus

<400> 133
Ser Asn Phe Asp Phe
1               5


<210> 134
<211> 11
<212> PRT
<213> Mus musculus

<400> 134
Lys Ala Ser Gln Asp Val Asp Thr Ser Val Ala
1               5                   10


<210> 135

<211> 7
<212> PRT
<213> Mus musculus

<400> 135
Trp Ala Ser Thr Arg His Thr
1               5


<210> 136
<211> 8
<212> PRT
<213> Mus musculus

<400> 136
Gln Gln Tyr Ser Asn Tyr Pro Thr
1               5


<210> 137
<211> 5
<212> PRT
<213> Mus musculus

<400> 137
Asp Tyr Tyr Met His
1               5


<210> 138
<211> 17
<212> PRT
<213> Mus musculus

<400> 138
Arg Ile Asp Pro Glu Asn Gly Asn Thr Ile Tyr Asp Pro Lys Phe Gln
1               5                   10                  15
Gly


<210> 139
<211> 11
<212> PRT
<213> Mus musculus

<400> 139
Ser Pro Tyr Asp Tyr His Ala Trp Phe Ala Tyr
1               5                   10


<210> 140
<211> 11
<212> PRT
<213> Mus musculus

<400> 140
Lys Ala Ser Gln Asp Ile Ser Asn Tyr Phe Thr
1               5                   10


<210> 141
<211> 7

<212> PRT
<213> Mus musculus

<400> 141
Arg Ala Asn Arg Leu Val Asp
1               5


<210> 142
<211> 9
<212> PRT
<213> Mus musculus

<400> 142
Leu Gln Tyr Asp Glu Phe Pro Tyr Thr
1               5


<210> 143
<211> 5
<212> PRT
<213> Mus musculus

<400> 143
Thr Tyr Thr Met Ser
1               5


<210> 144
<211> 17
<212> PRT
<213> Mus musculus

<400> 144
Tyr Ile Ser Asp Gly Gly Gly Ser Ser Tyr Phe Pro Asp Thr Val Lys
1               5                   10                  15
Gly


<210> 145
<211> 8
<212> PRT
<213> Mus musculus

<400> 145
His Ser Asn Trp Tyr Phe Asp Val
1               5


<210> 146
<211> 11
<212> PRT
<213> Mus musculus

<400> 146
Arg Ala Ser Gln Asp Ile Ser Asn Tyr Leu Asn
1               5                   10


<210> 147
<211> 7
<212> PRT

<213> Mus musculus

<400> 147
Tyr Thr Ser Thr Leu Thr Ser
1               5


<210> 148
<211> 9
<212> PRT
<213> Mus musculus

<400> 148
Gln Gln Gly Lys Thr Phe Pro Phe Thr
1               5


<210> 149
<211> 5
<212> PRT
<213> Mus musculus

<400> 149
Asn Tyr Phe Ile Glu
1               5


<210> 150
<211> 17
<212> PRT
<213> Mus musculus

<400> 150
Ala Ile Asn Pro Gly Ser Gly Gly Thr Asn Tyr Asn Glu Arg Phe Lys
1               5                   10                  15
Gly


<210> 151
<211> 11
<212> PRT
<213> Mus musculus

<400> 151
Glu Asp Tyr Gly Asp Val Tyr Ala Met Asp Tyr
1               5                   10


<210> 152
<211> 11
<212> PRT
<213> Mus musculus

<400> 152
Lys Ala Ser Gln Asp Val Asp Thr Ser Val Ala
1               5                   10


<210> 153
<211> 7
<212> PRT
<213> Mus musculus

<400> 153
Trp Ala Ser Thr Arg His Thr
 1               5

<210> 154
<211> 8
<212> PRT
<213> Mus musculus

<400> 154
Gln Gln Tyr Ser Ser Tyr Pro Thr
 1               5

<210> 155
<211> 5
<212> PRT
<213> Mus musculus

<400> 155
Asp Tyr Tyr Met His
 1               5

<210> 156
<211> 17
<212> PRT
<213> Mus musculus

<400> 156
Arg Ile Asp Pro Glu Asn Gly Asn Thr Ile Tyr Asp Pro Lys Phe Gln
 1               5                   10                  15
Gly

<210> 157
<211> 11
<212> PRT
<213> Mus musculus

<400> 157
Ser Pro Tyr Asp Tyr His Ala Trp Phe Ala Tyr
 1               5                   10

<210> 158
<211> 11
<212> PRT
<213> Mus musculus

<400> 158
Lys Ala Ser Gln Asp Val Asp Thr Ser Val Ala
 1               5                   10

<210> 159
<211> 7
<212> PRT
<213> Mus musculus

<400> 159

Trp Ala Ser Thr Arg His Thr
 1               5

<210> 160
<211> 8
<212> PRT
<213> Mus musculus

<400> 160
Gln Gln Tyr Ser Ser Tyr Pro Thr
 1               5

<210> 161
<211> 5
<212> PRT
<213> Mus musculus

<400> 161
Asp Tyr Tyr Met His
 1               5

<210> 162
<211> 17
<212> PRT
<213> Mus musculus

<400> 162
Arg Ile Asp Pro Glu Asn Gly Asn Thr Ile Tyr Asp Pro Lys Phe Gln
 1               5                   10                  15
Gly

<210> 163
<211> 11
<212> PRT
<213> Mus musculus

<400> 163
Ser Pro Tyr Asp Tyr His Ala Trp Phe Ala Tyr
 1               5                   10

<210> 164
<211> 15
<212> PRT
<213> Mus musculus

<400> 164
Lys Ala Ser Gln Ser Val Asp Tyr Asp Gly Asp Ser Tyr Met Asn
 1               5                   10                  15

<210> 165
<211> 7
<212> PRT
<213> Mus musculus

<400> 165
Ala Ala Ser Asn Leu Glu Ser

1               5


<210> 166
<211> 9
<212> PRT
<213> Mus musculus

<400> 166
Gln Gln Thr Asn Glu Asp Pro Pro Thr
1               5


<210> 167
<211> 5
<212> PRT
<213> Mus musculus

<400> 167
Ser Phe Trp Ile His
1               5


<210> 168
<211> 17
<212> PRT
<213> Mus musculus

<400> 168
Glu Ile Asn Pro Ser Asn Gly Arg Thr Asp Tyr Asn Ala Lys Phe Lys
1               5                   10                  15
Thr



<210> 169
<211> 10
<212> PRT
<213> Mus musculus

<400> 169
Gly Gly Thr Gly Thr Trp Tyr Phe Asp Val
1               5                   10


<210> 170
<211> 16
<212> PRT
<213> Mus musculus

<400> 170
Lys Ser Ser Gln Ser Leu Leu Asp Ser Asp Gly Glu Thr Tyr Leu Asn
1               5                   10                  15


<210> 171
<211> 7
<212> PRT
<213> Mus musculus

<400> 171
Leu Val Ser Lys Leu Asp Ser
1               5

<210> 172
<211> 9
<212> PRT
<213> Mus musculus

<400> 172
Trp Gln Gly Thr His Phe Pro Tyr Thr
1               5


<210> 173
<211> 5
<212> PRT
<213> Mus musculus

<400> 173
Asn Tyr Trp Met Asn
1               5


<210> 174
<211> 19
<212> PRT
<213> Mus musculus

<400> 174
Glu Ile Arg Leu Lys Ser Asp Asn Tyr Ala Thr His Phe Ala Glu Ser
1               5                   10                  15
Val Lys Gly


<210> 175
<211> 5
<212> PRT
<213> Mus musculus

<400> 175
Ile Leu Phe Gly Tyr
1               5


<210> 176
<211> 15
<212> PRT
<213> Mus musculus

<400> 176
Lys Ala Ser Gln Ser Val Asp Tyr Asp Gly Asp Ser Tyr Met Asn
1               5                   10                  15


<210> 177
<211> 7
<212> PRT
<213> Mus musculus

<400> 177
Ala Ala Ser Asn Leu Glu Ser
1               5

<210> 178
<211> 9
<212> PRT
<213> Mus musculus

<400> 178
Gln Gln Ser Asn Glu Asp Pro Trp Thr
1               5

<210> 179
<211> 5
<212> PRT
<213> Mus musculus

<400> 179
Ser Tyr Trp Met His
1               5

<210> 180
<211> 17
<212> PRT
<213> Mus musculus

<400> 180
Glu Ile Asn Pro Ser Asn Gly Arg Thr Asp Tyr Asn Glu Asn Phe Lys
1               5                   10                  15
Ser

<210> 181
<211> 8
<212> PRT
<213> Mus musculus

<400> 181
Gly Gly Val Tyr Ala Met Asp Tyr
1               5

<210> 182
<211> 16
<212> PRT
<213> Mus musculus

<400> 182
Arg Ser Ser Gln Ser Leu Val His Ser Asn Gly Asp Thr Tyr Leu His
1               5                   10                  15

<210> 183
<211> 7
<212> PRT
<213> Mus musculus

<400> 183
Glu Ile Ser Asn Arg Phe Ser
1               5

<210> 184

<211> 9
<212> PRT
<213> Mus musculus

<400> 184
Ser Gln Ser Thr His Val Pro Phe Thr
1               5


<210> 185
<211> 5
<212> PRT
<213> Mus musculus

<400> 185
Asp Tyr Tyr Ile His
1               5


<210> 186
<211> 17
<212> PRT
<213> Mus musculus

<400> 186
Trp Ile Asp Pro Glu Asn Gly Asp Thr Glu Tyr Ala Pro Lys Phe Gln
1               5                   10                  15
Asp



<210> 187
<211> 7
<212> PRT
<213> Mus musculus

<400> 187
Gly Ser Gly Leu Ile Pro Tyr
1               5


<210> 188
<211> 11
<212> PRT
<213> Mus musculus

<400> 188
Arg Ala Ser Gln Asp Ile Ser Asn Tyr Leu Asn
1               5                   10


<210> 189
<211> 7
<212> PRT
<213> Mus musculus

<400> 189
Tyr Thr Ser Thr Leu Thr Ser
1               5


<210> 190
<211> 9

<212> PRT
<213> Mus musculus

<400> 190
Gln Gln Gly Lys Thr Phe Pro Phe Thr
1               5

<210> 191
<211> 5
<212> PRT
<213> Mus musculus

<400> 191
Asn Tyr Phe Ile Glu
1               5

<210> 192
<211> 17
<212> PRT
<213> Mus musculus

<400> 192
Val Ile Asn Pro Glu Asn Gly Gly Thr Asn Tyr Asn Glu Arg Phe Lys
1               5                   10                  15
Asp

<210> 193
<211> 11
<212> PRT
<213> Mus musculus

<400> 193
Glu Asp Tyr Gly Asp Val Tyr Ala Met Asp Tyr
1               5                   10

<210> 194
<211> 16
<212> PRT
<213> Mus musculus

<400> 194
Arg Ser Ser Gln Ser Leu Val His Ser Asn Gly Asp Thr Tyr Leu His
1               5                   10                  15

<210> 195
<211> 7
<212> PRT
<213> Mus musculus

<400> 195
Glu Ile Ser Asn Arg Phe Ser
1               5

<210> 196
<211> 9
<212> PRT

<213> Mus musculus

<400> 196
Ser Gln Ser Thr His Val Pro Phe Thr
1               5

<210> 197
<211> 5
<212> PRT
<213> Mus musculus

<400> 197
Asp Tyr Tyr Ile His
1               5

<210> 198
<211> 17
<212> PRT
<213> Mus musculus

<400> 198
Trp Ile Asp Pro Glu Asn Gly Asp Ser Glu Tyr Ala Pro Lys Phe Gln
1               5                   10                  15
Asp

<210> 199
<211> 7
<212> PRT
<213> Mus musculus

<400> 199
Gly Ser Gly Leu Ile Pro Tyr
1               5

<210> 200
<211> 16
<212> PRT
<213> Mus musculus

<400> 200
Arg Ser Ser Gln Ser Leu Val His Ser Asn Gly Asp Val Tyr Phe His
1               5                   10                  15

<210> 201
<211> 7
<212> PRT
<213> Mus musculus

<400> 201
Glu Val Ser Asn Arg Phe Ser
1               5

<210> 202
<211> 9
<212> PRT
<213> Mus musculus

<400> 202
Ser Gln Ser Thr His Val Pro Tyr Thr
1               5

<210> 203
<211> 5
<212> PRT
<213> Mus musculus

<400> 203
Asp Tyr Tyr Ile His
1               5

<210> 204
<211> 17
<212> PRT
<213> Mus musculus

<400> 204
Trp Ile Asp Pro Glu Asn Gly Asp Ser Glu Tyr Ala Pro Lys Phe Gln
1               5                   10                  15
Asp

<210> 205
<211> 7
<212> PRT
<213> Mus musculus

<400> 205
Gly Ser Gly Leu Ile Pro Tyr
1               5

<210> 206
<211> 16
<212> PRT
<213> Mus musculus

<400> 206
Arg Ser Ser Gln Ser Leu Val His Ser Asn Gly Asp Val Tyr Leu His
1               5                   10                  15

<210> 207
<211> 7
<212> PRT
<213> Mus musculus

<400> 207
Glu Val Ser Asn Arg Phe Ser
1               5

<210> 208
<211> 9
<212> PRT
<213> Mus musculus

<400> 208

Ser Gln Ser Thr His Val Pro Tyr Thr
1               5

<210> 209
<211> 5
<212> PRT
<213> Mus musculus

<400> 209
Asp Tyr Tyr Val His
1               5

<210> 210
<211> 17
<212> PRT
<213> Mus musculus

<400> 210
Trp Ile Asp Pro Asp Asn Gly Asp Ser Glu Tyr Ala Pro Lys Phe Gln
1               5                   10                  15
Asp

<210> 211
<211> 7
<212> PRT
<213> Mus musculus

<400> 211
Gly Ser Gly Leu Ile Pro Tyr
1               5

<210> 212
<211> 16
<212> PRT
<213> Mus musculus

<400> 212
Arg Ser Ser Gln Ser Leu Val His Ser Asn Gly Asp Val Tyr Leu His
1               5                   10                  15

<210> 213
<211> 7
<212> PRT
<213> Mus musculus

<400> 213
Glu Val Ser Asn Arg Phe Ser
1               5

<210> 214
<211> 9
<212> PRT
<213> Mus musculus

<400> 214
Ser Gln Thr Thr His Val Pro Tyr Thr

1               5


<210> 215
<211> 5
<212> PRT
<213> Mus musculus

<400> 215
Asp Tyr Tyr Ile His
1               5


<210> 216
<211> 17
<212> PRT
<213> Mus musculus

<400> 216
Trp Ile Asp Pro Glu Asn Gly Asp Thr Glu Tyr Ala Pro Lys Phe Gln
1               5                   10                  15
Asp


<210> 217
<211> 7
<212> PRT
<213> Mus musculus

<400> 217
Gly Ser Gly Leu Ile Pro Tyr
1               5


<210> 218
<211> 16
<212> PRT
<213> Mus musculus

<400> 218
Arg Ser Ser Gln Ser Leu Val His Ser Asn Gly Asp Val Tyr Leu His
1               5                   10                  15


<210> 219
<211> 7
<212> PRT
<213> Mus musculus

<400> 219
Glu Val Ser Asn Arg Phe Ser
1               5


<210> 220
<211> 9
<212> PRT
<213> Mus musculus

<400> 220
Ser Gln Ser Thr His Val Pro Tyr Thr
1               5

<210> 221
<211> 5
<212> PRT
<213> Mus musculus

<400> 221
Asp Tyr Tyr Ile His
1               5

<210> 222
<211> 17
<212> PRT
<213> Mus musculus

<400> 222
Trp Ile Asp Pro Glu Asn Gly Asp Thr Glu Tyr Ala Pro Lys Phe Gln
1               5                   10                  15
Asp

<210> 223
<211> 7
<212> PRT
<213> Mus musculus

<400> 223
Gly Ser Gly Leu Ile Pro Tyr
1               5

<210> 224
<211> 11
<212> PRT
<213> Mus musculus

<400> 224
His Ala Ser Gln Asn Ile Asn Val Trp Leu Ser
1               5                   10

<210> 225
<211> 7
<212> PRT
<213> Mus musculus

<400> 225
Lys Val Ser Asn Leu His Thr
1               5

<210> 226
<211> 9
<212> PRT
<213> Mus musculus

<400> 226
Gln Gln Gly Gln Ser Tyr Pro Leu Thr
1               5

<210> 227
<211> 5
<212> PRT
<213> Mus musculus

<400> 227
Asp Tyr Tyr Ile His
1               5

<210> 228
<211> 17
<212> PRT
<213> Mus musculus

<400> 228
Arg Ile Asp Pro Glu Asn Gly Asn Thr Ile Tyr Asp Pro Lys Phe Gln
1               5                   10                  15
Gly

<210> 229
<211> 11
<212> PRT
<213> Mus musculus

<400> 229
Cys Asp Asn Asp Pro Gly Ser Glu Met Asp Tyr
1               5                   10

<210> 230
<211> 16
<212> PRT
<213> Mus musculus

<400> 230
Arg Ser Ser Gln Ser Leu Val His Ser Asn Gly Asp Val Tyr Phe His
1               5                   10                  15

<210> 231
<211> 7
<212> PRT
<213> Mus musculus

<400> 231
Glu Val Ser Asn Arg Phe Ser
1               5

<210> 232
<211> 9
<212> PRT
<213> Mus musculus

<400> 232
Ser Gln Ser Thr His Val Pro Tyr Thr
1               5

<210> 233

<211> 5
<212> PRT
<213> Mus musculus

<400> 233
Asp Tyr Tyr Val His
1               5

<210> 234
<211> 17
<212> PRT
<213> Mus musculus

<400> 234
Trp Ile Asp Pro Asp Asn Gly Asp Ser Glu Tyr Ala Pro Lys Phe Gln
1               5                   10                  15
Asp

<210> 235
<211> 7
<212> PRT
<213> Mus musculus

<400> 235
Gly Ser Gly Leu Ile Pro Tyr
1               5

<210> 236
<211> 112
<212> PRT
<213> Mus musculus

<400> 236
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Ala
            20                  25                  30
Gly Asp Asn Tyr Met Asn Trp Tyr Gln Gln Lys Pro Gly Gln Pro Pro
        35                  40                  45
Lys Leu Leu Ile Tyr Thr Ala Ser Asn Leu Glu Ser Gly Ile Pro Ala
    50                  55                  60
Arg Phe Ser Gly Ser Gly Ser Gly Thr Asp Phe Thr Leu Asn Ile His
65                  70                  75                  80
Pro Val Glu Glu Glu Asp Ala Ala Thr Tyr Tyr Cys Gln Gln Ser Asn
                85                  90                  95
Glu Asp Pro Pro Thr Phe Gly Gly Gly Thr Lys Leu Glu Leu Lys Arg
            100                 105                 110

<210> 237
<211> 336
<212> DNA
<213> Mus musculus

<400> 237
gacattgtgc tgacccaatc tccagcttct ttggctgtgt ctctagggca gagggccacc 60
atctcctgca aggccagcca aagtgttgat tatgctggtg ataattatat gaactggtac 120
caacagaaac caggacagcc acccaaactc ctcatctata ctgcatccaa tctagagtct 180
gggatcccag ccaggtttag tggcagtggg tctgggacag acttcaccct caacattcat 240

cctgtggagg aggaggatgc tgcaacctat tactgtcagc aaagtaatga ggatcctccg 300
acgttcggtg gaggcaccaa gttggagctc aaacgt 336

<210> 238
<211> 119
<212> PRT
<213> Mus musculus

<400> 238
Glu Val Gln Leu Gln Gln Ser Gly Pro Glu Leu Val Lys Pro Gly Ala
1               5                   10                  15
Ser Val Lys Ile Ser Cys Lys Ala Ser Gly Tyr Pro Phe Thr Gly Tyr
            20                  25                  30
Phe Met His Trp Val Lys Gln Ser Pro Glu Asn Ser Leu Glu Trp Ile
        35                  40                  45
Gly Glu Ile Asn Pro Ser Thr Gly Gly Thr Thr Tyr Asn Gln Arg Phe
    50                  55                  60
Lys Gly Lys Ala Thr Leu Thr Val Asp Lys Ser Ser Ser Thr Ala Tyr
65                  70                  75                  80
Met Gln Leu Lys Ser Leu Thr Ser Glu Asp Ser Ala Val Tyr Tyr Cys
                85                  90                  95
Thr Arg Trp Gly Tyr Asn Pro Tyr Ala Leu Asp Tyr Trp Gly Gln Gly
            100                 105                 110
Thr Ser Val Thr Val Ser Ser
        115

<210> 239
<211> 357
<212> DNA
<213> Mus musculus

<400> 239
gaggtccaac tgcagcagtc tggacctgag ctggtgaagc ctggggcttc agtgaagata 60
tcctgcaagg cttctggtta cccattcact ggctacttca tgcactgggt gaaacaaagt 120
cctgaaaata gtcttgagtg gattggagag attaatccta gcactggggg tactacctac 180
aaccagaggt tcaagggcaa ggccacatta actgtagata aatcctccag cacagcctac 240
atgcagctca agagcctgac atctgaagac tctgcagtct attactgtac aagatgggga 300
tataacccct atgctttgga ctactggggt caaggaacct cagtcaccgt ctcgagc 357

<210> 240
<211> 107
<212> PRT
<213> Mus musculus

<400> 240
Asp Ile Gln Met Thr Gln Ser Pro Ser Ser Leu Ser Ala Ser Leu Gly
1               5                   10                  15
Gly Lys Val Thr Ile Thr Cys Lys Ala Ser Gln Asp Ile Asn Lys Tyr
            20                  25                  30
Ile Ala Trp Tyr Gln His Lys Pro Gly Lys Gly Pro Arg Leu Leu Ile
        35                  40                  45
His Tyr Thr Ser Thr Leu Gln Pro Gly Ile Pro Ser Arg Phe Ser Gly
    50                  55                  60
Ser Gly Ser Gly Arg Asp Tyr Ser Phe Ser Ile Ser Asn Leu Glu Pro
65                  70                  75                  80
Glu Asp Ile Ala Thr Tyr Tyr Cys Leu Gln Tyr Asp Asn Leu Tyr Thr
                85                  90                  95
Phe Gly Gly Gly Thr Lys Leu Glu Leu Lys Arg
            100                 105

<210> 241

<211> 321
<212> DNA
<213> Mus musculus

<400> 241
gacatccaga tgacacagtc tccatcctca ctgtctgcat ctctgggagg caaagtcacc 60
atcacttgca aggcaagcca agacattaac aagtatatag cttggtacca acacaagcct 120
ggaaaaggtc ctaggctgct catacattac acatctacat tacagccagg catcccatca 180
aggttcagtg gaagtgggtc tgggagagat tattccttca gcatcagcaa cctggagcct 240
gaagatattg caacttatta ttgtctacag tatgataatc tatacacgtt cggagggggg 300
accaagctgg aactgaaacg t 321

<210> 242
<211> 115
<212> PRT
<213> Mus musculus

<400> 242
Gln Val Gln Leu Gln Gln Ser Gly Ala Glu Leu Met Lys Pro Gly Ala
1               5                   10                  15
Ser Val Lys Ile Ser Cys Lys Ala Thr Gly Tyr Thr Phe Ser Ser Tyr
            20                  25                  30
Trp Ile Glu Trp Val Lys Gln Arg Pro Gly His Gly Pro Glu Trp Ile
        35                  40                  45
Gly Glu Ile Phe Pro Arg Asn Gly Ser Thr Tyr Tyr Asn Glu Lys Phe
    50                  55                  60
Lys Gly Lys Ala Thr Phe Thr Ala Asp Thr Ser Ser Asn Thr Ala Tyr
65                  70                  75                  80
Met Gln Leu Ser Ser Leu Thr Ser Asp Asp Ser Ala Val Tyr Tyr Cys
                85                  90                  95
Ala Ile Ile Asn Thr Leu Asp Tyr Trp Gly Gln Gly Thr Thr Leu Thr
            100                 105                 110
Val Ser Ser
        115

<210> 243
<211> 345
<212> DNA
<213> Mus musculus

<400> 243
caggttcagc tgcagcagtc tggagctgag ctgatgaagc ctggggcctc agtgaagata 60
tcctgcaagg ctactggcta cacattcagt agttattgga tagagtgggt aaagcagagg 120
cctggacatg gccctgagtg gattggagag atttttccta gaaatggtag tacttactac 180
aatgagaaat tcaagggcaa ggccacattc actgcagata catcctccaa cacagcctac 240
atgcaactca gcagcctgac atctgacgac tctgccgtct attactgtgc aattattaat 300
acgcttgact actggggcca aggcaccact ctcacagtct cgagc 345

<210> 244
<211> 113
<212> PRT
<213> Mus musculus

<400> 244
Asp Val Val Met Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asp Thr Tyr Leu His Trp Tyr Leu Gln Lys Pro Gly Gln Ser
        35                  40                  45
Pro Lys Leu Leu Ile Tyr Glu Ile Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80
Ser Arg Val Glu Ala Glu Asp Leu Gly Val Tyr Phe Cys Ser Gln Ser
                85                  90                  95
Thr His Val Pro Phe Thr Phe Gly Gly Gly Thr Lys Leu Glu Ile Lys
            100                 105                 110
Arg

<210> 245
<211> 339
<212> DNA
<213> Mus musculus

<400> 245
gatgttgtga tgacccaaac tccactctcc ctgcctgtca gtcttggaga tcaagcctcc 60
atctcttgca gatctagtca gagccttgta cacagtaatg gagacaccta tttacattgg 120
tacctacaga agccaggcca gtctccaaaa ctcctgatct acgaaatttc caaccgattt 180
tctggggtcc cagacaggtt cagtggcagt ggatcaggga cagatttcac actcaagatc 240
agcagagtgg aggctgagga tctgggagtt tatttctgct ctcaaagtac acatgttccg 300
ttcacgttcg gaggggggac caagctggaa ataaaacgt                        339

<210> 246
<211> 116
<212> PRT
<213> Mus musculus

<400> 246
Glu Val Gln Leu Gln Gln Ser Gly Ala Glu Leu Val Arg Ser Gly Ala
 1               5                  10                  15
Ser Val Arg Leu Ser Cys Thr Ala Ser Gly Phe Asn Ile Lys Asp Tyr
            20                  25                  30
Tyr Met His Trp Val Arg Gln Arg Pro Glu Gln Gly Leu Glu Trp Ile
        35                  40                  45
Gly Trp Asn Asp Pro Glu Thr Gly Asp Thr Glu Tyr Ala Pro Lys Phe
    50                  55                  60
Gln Gly Lys Ala Thr Met Thr Ser Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Asn Glu Gly Ser Gly Leu Ile Pro Tyr Trp Gly Pro Gly Thr Leu Val
            100                 105                 110
Thr Val Ser Ser
        115

<210> 247
<211> 348
<212> DNA
<213> Mus musculus

<400> 247
gaggttcagc tgcagcagtc tggggcagaa cttgtgaggt caggggcctc agtcaggttg 60
tcctgcacag cttctggctt caacattaaa gactactata tgcactgggt gaggcagcgg 120
cctgaacagg gcctggagtg gattggatgg aatgatcctg agactggtga tactgaatat 180
gccccgaagt tccagggcaa ggccactatg acttcagaca catcctccaa cacagcctac 240
ctgcagctca gcagcctgac atctgaggac actgccgtct attactgtaa tgagggctcg 300
ggcttgattc cttactgggg cccagggact ctggtcacag tctcgagc              348

<210> 248
<211> 113
<212> PRT

<213> Mus musculus

<400> 248
Asp Ile Val Met Thr Gln Thr Pro Leu Ser Leu Ser Val Thr Pro Gly
1               5                   10                  15
Gln Pro Ala Ser Ile Ser Cys Arg Ser Ser Gln Ser Leu Val His Ser
            20                  25                  30
Asn Gly Asp Thr Tyr Leu His Trp Tyr Leu Gln Lys Pro Gly Gln Pro
        35                  40                  45
Pro Gln Leu Leu Ile Tyr Glu Ile Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Ser Gln Ser
                85                  90                  95
Thr His Val Pro Phe Thr Phe Gly Gln Gly Thr Lys Leu Glu Ile Lys
            100                 105                 110
Arg

<210> 249
<211> 339
<212> DNA
<213> Mus musculus

<400> 249
gatatcgtga tgacccagac tccactctcc ctgtctgtca ctccgggtca accggcctcc 60
atctcttgcc gttctagtca gagccttgta cacagtaatg gtgacaccta tttacattgg 120
tacttacaga agccaggcca gcctccacaa ctcctgatct acgaaatttc caaccgcttt 180
tctggggtcc cagaccgttt cagtggcagt ggttcaggga cagatttcac actcaagatc 240
agccgcgtgg aggctgagga tgtgggtgtt tattactgct ctcaaagtac acatgttccg 300
ttcacgttcg gtcaggggac caagctggaa attaaacgt 339

<210> 250
<211> 116
<212> PRT
<213> Mus musculus

<400> 250
Gln Val Gln Leu Val Gln Ser Gly Ala Glu Val Lys Lys Pro Gly Ser
1               5                   10                  15
Ser Val Lys Val Ser Cys Lys Ala Ser Gly Phe Asn Ile Lys Asp Tyr
            20                  25                  30
Tyr Met His Trp Val Arg Gln Ala Pro Gly Gln Gly Leu Glu Trp Met
        35                  40                  45
Gly Trp Asn Asp Pro Glu Thr Gly Asp Thr Glu Tyr Ala Pro Lys Phe
    50                  55                  60
Gln Gly Arg Val Thr Ile Thr Ala Asp Glu Ser Thr Asn Thr Ala Tyr
65                  70                  75                  80
Met Glu Leu Ser Ser Leu Arg Ser Glu Asp Thr Ala Val Tyr Tyr Cys
                85                  90                  95
Asn Glu Gly Ser Gly Leu Ile Pro Tyr Trp Gly Gln Gly Thr Leu Val
            100                 105                 110
Thr Val Ser Ser
        115

<210> 251
<211> 348
<212> DNA
<213> Mus musculus

<400> 251
caggttcagc tggtccagtc tggggcagag gttaaaaagc cagggtcctc agtcaaagtg 60
tcctgcaagg cttctggctt caacattaaa gactactata tgcactgggt gagacaggcg 120
cctggtcagg gcctggagtg gatgggatgg aatgatcctg agactggtga tactgaatat 180
gccccgaagt tccagggcag ggtcactatt actgcagacg aatccaccaa cacagcctac 240
atggagctct catccctgcg ttctgaggac actgccgtct attactgtaa tgagggctcg 300
ggcttgattc cttactgggg ccaagggact ctggtcaccg tctcgagc 348

<210> 252
<211> 113
<212> PRT
<213> Mus musculus

<400> 252
Asp Val Val Leu Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asp Val Tyr Leu His Trp Tyr Leu Gln Lys Pro Gly Gln Ser
        35                  40                  45
Pro Lys Leu Leu Ile Tyr Glu Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Leu Gly Val Tyr Phe Cys Ser Gln Ser
                85                  90                  95
Thr His Val Pro Phe Thr Phe Gly Gly Gly Thr Lys Leu Glu Ile Lys
            100                 105                 110
Arg

<210> 253
<211> 339
<212> DNA
<213> Mus musculus

<400> 253
gatgttgtat tgacccaaac tccactctcc ctgcctgtca gtcttggaga tcaagcctcc 60
atctcttgta gatctagtca gagccttgta cacagtaatg gagacgtcta tttacattgg 120
tacctacaga agccaggcca gtctccaaag ctcctgatct acgaagtttc caaccgattt 180
tctggggtcc cagacaggtt cagtggcagt ggatcaggga cagatttcac actcaagatc 240
agcagagtgg aggctgagga tctgggagtt tatttctgct ctcaaagtac acatgttccg 300
ttcacgttcg gaggggggac caagctggaa ataaaacgt 339

<210> 254
<211> 116
<212> PRT
<213> Mus musculus

<400> 254
Glu Val Gln Leu Gln Gln Ser Gly Thr Glu Leu Val Arg Ser Gly Ala
1               5                   10                  15
Ser Val Lys Leu Ala Cys Thr Ala Ser Gly Phe Asn Ile Lys Asn Tyr
            20                  25                  30
Tyr Met His Trp Val Arg Gln Arg Pro Glu Gln Gly Leu Glu Trp Ile
        35                  40                  45
Gly Trp Ile Asp Pro Glu Asn Gly Asp Thr Glu Tyr Ala Pro Lys Phe
    50                  55                  60
Gln Asp Lys Ala Thr Met Thr Ala Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95

Asn Glu Gly Ser Gly Leu Ile Pro Tyr Trp Gly Pro Gly Thr Leu Val
            100                 105                 110
Thr Val Ser Ser
        115

<210> 255
<211> 348
<212> DNA
<213> Mus musculus

<400> 255
gaggttcagc tgcagcagtc tgggacagag cttgtgaggt caggggcctc agtcaagttg 60
gcctgcacag cttctggctt caacattaaa aactactata tgcactgggt gaggcagagg 120
cctgaacagg gcctggagtg gattggatgg attgatcctg agaatggtga tactgagtat 180
gccccgaagt tccaggacaa ggccactatg actgcagaca catcctccaa cacagcctac 240
ctgcagctca gcagcctgac atctgaggac actgccgtct attactgtaa tgagggctcg 300
ggcttgattc cttactgggg cccagggact ctggtcactg tctcgagc 348

<210> 256
<211> 113
<212> PRT
<213> Mus musculus

<400> 256
Gly Val Val Met Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asn Thr Tyr Leu Tyr Trp Tyr Leu Gln Lys Pro Gly Gln Ser
        35                  40                  45
Pro Lys Leu Leu Leu Phe Lys Val Ser Thr Arg Phe Ser Gly Val Pro
    50                  55                  60
Asp Arg Phe Ser Gly Ser Gly Ser Gly Thr Asp Phe Thr Leu Lys Ile
65                  70                  75                  80
Thr Arg Val Glu Ala Glu Asp Leu Gly Ile Tyr Phe Cys Ser Gln Ser
                85                  90                  95
Ser His Ile Pro Pro Thr Phe Gly Gly Gly Thr Lys Leu Glu Leu Lys
            100                 105                 110
Arg

<210> 257
<211> 339
<212> DNA
<213> Mus musculus

<400> 257
ggtgttgtga tgacccaaac tccactctcc ctgcctgtca gtcttggaga tcaagcctcc 60
atctcgtgca gatctagtca gagccttgtt cacagtaatg gaaacaccta tttatattgg 120
tacctacaga agccaggcca gtctccaaag ctcctgctct tcaaagtttc cacccgattt 180
tctggggtcc cagacaggtt cagtggcagt ggatcaggga cagatttcac actcaagatc 240
accagagtag aggctgagga tctgggaatt tatttctgct ctcaaagttc acatattcct 300
ccgacgttcg gtggaggcac caagttggag ctcaaacgt 339

<210> 258
<211> 114
<212> PRT
<213> Mus musculus

<400> 258
Gln Ile Gln Leu Val Gln Ser Gly Pro Glu Leu Lys Lys Pro Gly Glu

1               5                   10                  15
Thr Val Lys Ile Ser Cys Lys Ala Ser Gly Tyr Thr Val Thr Asp Tyr
            20                  25                  30
Gly Met Asn Trp Val Lys Gln Ser Pro Gly Lys Asp Leu Lys Trp Met
        35                  40                  45
Gly Trp Ile Asp Thr Tyr Thr Glu Lys Pro Thr Tyr Ala Asp Asp Phe
    50                  55                  60
Lys Gly Arg Phe Ala Phe Ser Leu Glu Thr Ser Ala Ser Thr Ala Tyr
65                  70                  75                  80
Leu Gln Ile Ser Asn Leu Lys Asn Glu Asp Ser Ala Ala Tyr Phe Cys
                85                  90                  95
Leu Arg Ser Asn Phe Asp Phe Trp Gly Gln Gly Thr Thr Leu Thr Val
            100                 105                 110
Ser Ser

<210> 259
<211> 342
<212> DNA
<213> Mus musculus

<400> 259
cagatccagt tggtgcagtc tggacctgag ctgaagaagc ctggagagac agtcaagatc 60
tcctgcaagg cttctgggta taccgtcaca gactatggaa tgaactgggt gaagcagagt 120
ccaggaaagg atttaaagtg gatgggctgg atagacacct acactgaaaa gccaacatat 180
gctgatgact tcaagggacg atttgccttc tctttggaaa cctctgccag cactgcctac 240
ttgcagatca gcaacctcaa aaatgaggac tcggctgcat atttctgtct aagatccaat 300
tttgactttt ggggccaagg caccactctc acagtctcga gc                    342

<210> 260
<211> 107
<212> PRT
<213> Mus musculus

<400> 260
Asp Ile Val Met Thr Gln Ser His Lys Phe Met Ser Thr Ser Val Gly
1               5                   10                  15
Gly Arg Val Ser Ile Thr Cys Lys Ala Ser Gln Asp Val Asp Thr Ser
            20                  25                  30
Val Ala Trp Tyr Gln Gln Lys Pro Gly Gln Ala Pro Lys Leu Leu Val
        35                  40                  45
Tyr Trp Ala Ser Thr Arg His Thr Gly Val Pro Asp Arg Phe Thr Gly
    50                  55                  60
Ser Gly Ser Gly Thr Asp Phe Thr Leu Thr Ile Ser Asn Val Gln Ser
65                  70                  75                  80
Glu Asp Leu Ala Asp Tyr Phe Cys Gln Gln Tyr Ser Asn Tyr Pro Thr
                85                  90                  95
Phe Gly Ala Gly Thr Lys Leu Glu Leu Lys Arg
            100                 105

<210> 261
<211> 321
<212> DNA
<213> Mus musculus

<400> 261
gacattgtga tgacccagtc tcacaaattc atgtccacat cagtaggagg cagggtcagc 60
atcacctgca aggccagtca ggatgtggat acttctgtag cctggtatca acagaaacca 120
gggcaagctc ctaaacttct ggtttactgg gcatccaccc gccacactgg agtccctgat 180
cgcttcacag gcagtggatc tgggacagat ttcactctca ccattagcaa tgtgcagtct 240
gaagacttgg cagattattt ctgtcagcaa tatagcaact atcccacgtt cggtgctggg 300

accaagctgg aactgaaacg t 321

<210> 262
<211> 120
<212> PRT
<213> Mus musculus

<400> 262
Glu Val Gln Leu Gln Gln Ser Gly Ala Glu Leu Val Arg Pro Gly Ala
1               5                   10                  15
Ser Val Lys Leu Ser Cys Lys Ala Ser Gly Phe Asn Ile Lys Asp Tyr
            20                  25                  30
Tyr Met His Gly Val Lys His Arg Pro Glu Gln Gly Leu Glu Trp Ile
        35                  40                  45
Gly Arg Ile Asp Pro Glu Asn Gly Asn Thr Ile Tyr Asp Pro Lys Phe
    50                  55                  60
Gln Gly Lys Ala Thr Ile Thr Ala Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Ala Arg Ser Pro Tyr Asp Tyr His Ala Trp Phe Ala Tyr Trp Gly Gln
            100                 105                 110
Gly Thr Leu Val Thr Val Ser Ser
        115                 120

<210> 263
<211> 360
<212> DNA
<213> Mus musculus

<400> 263
gaggttcagc tgcagcagtc tggggctgag cttgtgaggc caggggcctc agtcaagttg 60
tcctgcaaag cttctggctt caacattaaa gactactata tgcacggggt gaaacatagg 120
cctgaacagg gcctggagtg gattggaagg attgatcctg aaaatggtaa tactatatat 180
gacccgaagt tccagggcaa ggccactata acagcagaca catcctccaa cacagcctac 240
ctgcagctca gcagcctgac atctgaggac actgccgtct attactgtgc tagatcccct 300
tatgattacc acgcctggtt tgcttactgg ggccaaggga ctctggtcac cgtctcgagc 360

<210> 264
<211> 108
<212> PRT
<213> Mus musculus

<400> 264
Asp Ile Lys Met Thr Gln Ser Pro Ser Ser Met Tyr Thr Ser Leu Gly
1               5                   10                  15
Glu Arg Val Thr Ile Thr Cys Lys Ala Ser Gln Asp Ile Ser Asn Tyr
            20                  25                  30
Phe Thr Trp Phe Gln Gln Lys Pro Gly Lys Ser Pro Lys Thr Leu Ile
        35                  40                  45
Tyr Arg Ala Asn Arg Leu Val Asp Gly Val Pro Ser Arg Phe Ser Gly
    50                  55                  60
Ser Gly Ser Gly Gln Asp Phe Ser Leu Thr Ile Ser Ser Leu Glu Tyr
65                  70                  75                  80
Glu Asp Met Gly Ile Tyr Tyr Cys Leu Gln Tyr Asp Glu Phe Pro Tyr
                85                  90                  95
Thr Phe Gly Gly Gly Thr Lys Leu Glu Leu Lys Arg
            100                 105

<210> 265

<211> 324
<212> DNA
<213> Mus musculus

<400> 265
gacatcaaga tgacccagtc tccatcttcc atgtatacat ctctaggcga gagagtcact 60
atcacttgca aggcgagtca ggacattagt aactattt[?]a cctggttcca gcagaaacca 120
gggaaatctc ctaagaccct gatctatcgt gcaaacagat tggtagatgg ggtcccatca 180
aggttcagtg gcagtggatc tgggcaagat ttttctctca ccatcagcag cctcgaatat 240
gaagatatgg gaatttatta ttgtctacag tatgatgagt ttccgtacac gttcggaggg 300
gggaccaagt tggagctcaa acgt 324

<210> 266
<211> 117
<212> PRT
<213> Mus musculus

<400> 266
Glu Val Lys Leu Val Glu Ser Gly Gly Gly Val Met Gln Pro Gly Gly
1               5                   10                  15
Ser Leu Lys Leu Ser Cys Ala Ala Ser Gly Phe Thr Phe Arg Thr Tyr
            20                  25                  30
Thr Met Ser Trp Val Arg Gln Thr Pro Glu Lys Arg Leu Glu Trp Val
        35                  40                  45
Ala Tyr Ile Ser Asp Gly Gly Gly Ser Ser Tyr Phe Pro Asp Thr Val
    50                  55                  60
Lys Gly Arg Phe Thr Val Ser Arg Asp Asn Ala Lys Asn Thr Leu Tyr
65                  70                  75                  80
Leu Gln Met Ser Ser Leu Arg Ser Glu Asp Thr Ala Met Tyr Tyr Cys
                85                  90                  95
Thr Arg His Ser Asn Trp Tyr Phe Asp Val Trp Gly Ala Gly Thr Ser
            100                 105                 110
Val Thr Val Ser Ser
        115

<210> 267
<211> 351
<212> DNA
<213> Mus musculus

<400> 267
gaagtgaagt tggtggagtc tgggggaggt gtaatgcagc ctggagggtc cctgaaactc 60
tcctgtgcag cctctgggtt cactttcaga acctatacca tgtcttgggt tcgccagact 120
ccagagaaga ggctggagtg ggtcgcatat attagtga[?]g gtggtggtag ttcctacttt 180
ccagacactg tcaagggccg attcaccgtc tccagggaca atgccaagaa caccctatac 240
ctgcaaatga gcagtctgag gtctgaggac acggccatgt attactgtac aagacattct 300
aactggtact tcgatgtctg gggcgcaggg acctcagtca ccgtctcgag c 351

<210> 268
<211> 108
<212> PRT
<213> Mus musculus

<400> 268
Asp Ile Gln Met Thr Gln Thr Thr Ser Ser Leu Ser Ala Ser Leu Gly
1               5                   10                  15
Asp Arg Val Thr Ile Arg Cys Arg Ala Ser Gln Asp Ile Ser Asn Tyr
            20                  25                  30
Leu Asn Trp Tyr Gln Gln Lys Pro Asp Gly Pro Val Lys Leu Leu Ile
        35                  40                  45
Tyr Tyr Thr Ser Thr Leu Thr Ser Gly Val Pro Ser Arg Phe Ser Gly
    50                  55                  60

Ser Gly Ser Gly Thr Asp Tyr Ser Leu Thr Ile Ser Asn Leu Asp Gln
65                  70                  75                  80
Glu Asp Ile Ala Thr Tyr Phe Cys Gln Gln Gly Lys Thr Phe Pro Phe
                85                  90                  95
Thr Phe Gly Ser Gly Thr Lys Leu Glu Ile Lys Arg
            100                 105

<210> 269
<211> 324
<212> DNA
<213> Mus musculus

<400> 269
gatatccaga tgacacagac tacatcctcc ctgtctgcct ctctgggaga cagagtcacc 60
atccgttgca gggcaagtca ggacattagc aattatttaa actggtatca gcagaaacca 120
gatggacctg ttaaactcct gatctactac acatctacat tgacctcagg agtcccatca 180
aggttcagtg gcagtgggtc tggaacagat tattctctca ccattagcaa cctggaccaa 240
gaagatattg ccacttactt ttgccaacag ggtaagacgt ttccattcac gttcggctcg 300
gggacaaagt tggaaataaa acgt                                        324

<210> 270
<211> 120
<212> PRT
<213> Mus musculus

<400> 270
Gln Val Gln Leu Gln Gln Ser Gly Ala Glu Leu Val Arg Pro Gly Thr
1               5                   10                  15
Ser Val Lys Val Ser Cys Lys Ala Ser Gly Tyr Ala Phe Thr Asn Tyr
            20                  25                  30
Phe Ile Glu Trp Val Lys Gln Arg Pro Gly Gln Gly Leu Glu Trp Ile
        35                  40                  45
Gly Ala Ile Asn Pro Gly Ser Gly Gly Thr Asn Tyr Asn Glu Arg Phe
    50                  55                  60
Lys Gly Lys Ala Thr Leu Ser Ala Asp Lys Ser Ser Ser Thr Ala Tyr
65                  70                  75                  80
Met Gln Leu Ser Ser Leu Thr Ser Asp Asp Ser Ala Val Tyr Phe Cys
                85                  90                  95
Ala Arg Glu Asp Tyr Gly Asp Val Tyr Ala Met Asp Tyr Trp Gly Gln
            100                 105                 110
Gly Thr Ser Val Thr Val Ser Ser
        115                 120

<210> 271
<211> 360
<212> DNA
<213> Mus musculus

<400> 271
caggtccagc tgcagcagtc tggagctgag ctggtcaggc ctgggacttc agtgaaggtg 60
tcctgcaagg cttctggata cgccttcact aattacttca tagagtgggt aaaacagagg 120
cctggacagg gccttgagtg gattggagcg attaatcctg gaagtggtgg tactaactac 180
aatgagaggt tcaagggcaa ggcaactctg tctgcagaca aatcctccag cactgcctac 240
atgcagctca gcagcctgac atctgatgac tctgcggtct atttctgtgc aagagaggac 300
tatggtgatg tctatgctat ggactactgg ggtcaaggaa cctcggtcac agtctcgagc 360

<210> 272
<211> 107
<212> PRT
<213> Mus musculus

<400> 272
Asp Ile Val Met Thr Gln Ser His Lys Phe Met Ser Thr Ser Val Gly
1               5                   10                  15
Asp Arg Val Ser Ile Thr Cys Lys Ala Ser Gln Asp Val Asp Thr Ser
            20                  25                  30
Val Ala Trp Tyr Gln Gln Lys Pro Gly Gln Ala Pro Lys Leu Leu Ile
        35                  40                  45
Tyr Trp Ala Ser Thr Arg His Thr Gly Val Pro Asp Arg Phe Thr Gly
    50                  55                  60
Ser Gly Ser Gly Thr Asp Phe Thr Leu Thr Ile Ser Asn Val Gln Ser
65                  70                  75                  80
Glu Asp Leu Ala Asp Tyr Phe Cys Gln Gln Tyr Ser Ser Tyr Pro Thr
                85                  90                  95
Phe Gly Ala Gly Thr Lys Leu Glu Leu Lys Arg
            100                 105

<210> 273
<211> 321
<212> DNA
<213> Mus musculus

<400> 273
gacattgtga tgacccagtc tcacaaattc atgtccacat cagtaggaga cagggtcagc 60
atcacctgca aggccagtca ggatgtggat acttctgtag cctggtatca acagaaacca 120
gggcaagctc ctaaactact gatttactgg gcatccaccc ggcacactgg agtccctgat 180
cgcttcacag gcagtggatc tgggacagat ttcactctca ccattagcaa tgtgcagtct 240
gaagacttgg cagattattt ctgtcagcaa tatagcagct atcccacgtt cggtgctggg 300
accaagctgg aactgaaacg t                                           321

<210> 274
<211> 120
<212> PRT
<213> Mus musculus

<400> 274
Glu Val Gln Leu Gln Gln Ser Gly Ala Glu Leu Val Arg Pro Gly Ala
1               5                   10                  15
Ser Val Lys Leu Ser Cys Lys Ala Ser Gly Phe Asn Ile Lys Asp Tyr
            20                  25                  30
Tyr Met His Gly Val Lys His Arg Pro Glu Gln Gly Leu Glu Trp Ile
        35                  40                  45
Gly Arg Ile Asp Pro Glu Asn Gly Asn Thr Ile Tyr Asp Pro Lys Phe
    50                  55                  60
Gln Gly Lys Ala Thr Ile Thr Ala Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Ala Arg Ser Pro Tyr Asp Tyr His Ala Trp Phe Ala Tyr Trp Gly Gln
            100                 105                 110
Gly Thr Leu Val Thr Val Ser Ser
        115                 120

<210> 275
<211> 360
<212> DNA
<213> Mus musculus

<400> 275
gaggttcagc tgcagcagtc tggggctgag cttgtgaggc caggggcctc agtcaagttg 60
tcctgcaaag cttctggctt caacattaaa gactactata tgcacggggt gaaacatagg 120
cctgaacagg gcctggagtg gattggaagg attgatcctg agaatggtaa tactatatat 180

gacccgaagt tccagggcaa ggccactata acagcagaca catcctccaa cacagcctac 240
ctgcagctca gcagcctgac atctgaggac actgccgtct attactgtgc tagatcccct 300
tatgattacc acgcctggtt tgcttactgg ggccaaggga ctctggtcac tgtctcgagc 360

<210> 276
<211> 107
<212> PRT
<213> Mus musculus

<400> 276
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Val Asp Thr Ser
            20                  25                  30
Val Ala Trp Tyr Gln Gln Lys Pro Gly Lys Ala Pro Lys Leu Leu Ile
        35                  40                  45
Tyr Trp Ala Ser Thr Arg His Thr Gly Val Pro Asp Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Ser Tyr Pro Thr
                85                  90                  95
Phe Gly Gln Gly Thr Lys Leu Glu Ile Lys Arg
            100                 105

<210> 277
<211> 321
<212> DNA
<213> Mus musculus

<400> 277
gatatccaga tgacccagtc tccatcctcc ctgtctgcat ctgtaggcga ccgtgtcacc 60
atcacttgca aggcaagtca ggacgttgat acctctgtag cttggtatca gcagaaacca 120
gggaaagccc ctaagctcct gatctattgg gcatccactc gtcatactgg ggtcccagac 180
cgcttcagtg gcagtggttc tgggacagat ttcactctca ccatcagcag tctgcaacct 240
gaagattttg caacttatta ctgtcagcaa tatagcagtt accctacgtt cggtcagggg 300
accaagctgg aaattaaacg t                                           321

<210> 278
<211> 120
<212> PRT
<213> Mus musculus

<400> 278
Glu Val Gln Leu Val Gln Ser Gly Ala Glu Val Lys Lys Pro Gly Ala
1               5                   10                  15
Thr Val Lys Ile Ser Cys Lys Ala Ser Gly Phe Asn Ile Lys Asp Tyr
            20                  25                  30
Tyr Met His Gly Val Gln His Ala Pro Gly Lys Gly Leu Glu Trp Ile
        35                  40                  45
Gly Arg Ile Asp Pro Glu Asn Gly Asn Thr Ile Tyr Asp Pro Lys Phe
    50                  55                  60
Gln Gly Arg Val Thr Ile Thr Ala Asp Thr Ser Thr Asn Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Pro Tyr Asp Tyr His Ala Trp Phe Ala Tyr Trp Gly Gln
            100                 105                 110
Gly Thr Leu Val Thr Val Ser Ser
        115                 120

<210> 279
<211> 360
<212> DNA
<213> Mus musculus

<400> 279
gaggtccagc tggtacagtc tggggctgag gtgaagaagc ctggggctac agtgaaaatc 60
tcctgcaagg cttctggatt caacatcaaa gactactaca tgcacggggt gcaacatgcc 120
cctggaaaag ggcttgagtg gattggacgt attgatcctg aaaatggtaa cacaatctac 180
gacccgaagt tccagggccg cgtcaccatt accgcggaca cgtctacaaa cacagcctac 240
atggagctga gcagcctgcg ttctgaggac acggccgtgt attactgtgc acgttctccg 300
tatgattatc acgcctggtt tgcttactgg ggccaaggga ctctggtcac cgtctcgagc 360

<210> 280
<211> 112
<212> PRT
<213> Mus musculus

<400> 280
Asp Ile Val Leu Thr Gln Ser Pro Ala Ser Leu Ala Val Ser Leu Gly
1               5                   10                  15
Gln Arg Ala Thr Ile Tyr Cys Lys Ala Ser Gln Ser Val Asp Tyr Asp
            20                  25                  30
Gly Asp Ser Tyr Met Asn Trp Tyr Gln Gln Lys Pro Gly Gln Pro Pro
        35                  40                  45
Lys Val Leu Ile Phe Ala Ala Ser Asn Leu Glu Ser Gly Ile Pro Ala
    50                  55                  60
Arg Phe Ser Gly Ser Arg Ser Gly Thr Asp Phe Thr Leu Asn Ile His
65                  70                  75                  80
Pro Val Glu Glu Glu Asp Ala Ala Thr Tyr Tyr Cys Gln Gln Thr Asn
                85                  90                  95
Glu Asp Pro Pro Thr Phe Gly Gly Gly Thr Lys Leu Glu Leu Lys Arg
            100                 105                 110

<210> 281
<211> 336
<212> DNA
<213> Mus musculus

<400> 281
gacattgtgc tgacccaatc tccagcttct ttggctgtgt ctctagggca gagggccacc 60
atttactgca aggccagcca aagtgttgat tatgatggtg atagttatat gaactggtac 120
caacagaaac caggacagcc acccaaagtc ctcatctttg ctgcatccaa tctagaatct 180
gggatcccgg ccaggtttag tggcagtaga tctgggacag acttcaccct caacatccat 240
cctgtggagg aggaggatgc tgcaacctat tactgtcaac aaactaatga ggatcctccg 300
acgttcggtg gcggcaccaa gctggaactg aaacgt                            336

<210> 282
<211> 119
<212> PRT
<213> Mus musculus

<400> 282
Gln Val Gln Leu Gln Gln Ser Gly Ala Glu Leu Val Lys Pro Gly Ala
1               5                   10                  15
Ser Val Lys Leu Ser Cys Lys Ala Ser Gly Tyr Ala Phe Ile Ser Phe
            20                  25                  30
Trp Ile His Trp Val Lys Gln Arg Pro Gly Gln Gly Leu Glu Trp Ile
        35                  40                  45
Gly Glu Ile Asn Pro Ser Asn Gly Arg Thr Asp Tyr Asn Ala Lys Phe
    50                  55                  60

Lys Thr Lys Ala Thr Leu Thr Val Asp Arg Ser Ser Ser Thr Ala Tyr
65                  70                  75                  80
Met Gln Leu Ser Ser Leu Thr Ser Glu Asp Ser Ala Asp Tyr Tyr Cys
                85                  90                  95
Ala Arg Gly Gly Thr Gly Thr Trp Tyr Phe Asp Val Trp Gly Ala Gly
            100                 105                 110
Thr Thr Val Thr Val Ser Ser
        115

<210> 283
<211> 357
<212> DNA
<213> Mus musculus

<400> 283
caggtccaac tgcagcagtc tggggctgaa ctggtgaagc ctggggcttc agtgaagctg 60
tcctgcaagg cttctggcta cgcctttatt agcttctgga tacactgggt gaagcagagg 120
cctggtcaag gccttgagtg gattggagag attaatccta gcaacggtcg tactgactac 180
aatgcgaagt tcaagaccaa ggccacactg actgttgaca gatcctcctc cacagcctac 240
atgcaactca gcagcctgac atctgaggac tctgcggact attactgtgc aagaggagga 300
actgggacct ggtacttcga tgtctggggc gcagggacca cagtcaccgt ctcgagc    357

<210> 284
<211> 113
<212> PRT
<213> Mus musculus

<400> 284
Asp Val Val Met Thr Gln Thr Pro Leu Thr Leu Ser Val Thr Ile Gly
1               5                   10                  15
Gln Pro Ala Ser Ile Ser Cys Lys Ser Ser Gln Ser Leu Leu Asp Ser
            20                  25                  30
Asp Gly Glu Thr Tyr Leu Asn Trp Leu Leu Gln Arg Pro Gly Gln Ser
        35                  40                  45
Pro Lys Arg Leu Ile Tyr Leu Val Ser Lys Leu Asp Ser Gly Val Pro
    50                  55                  60
Asp Arg Phe Thr Gly Ser Gly Ser Gly Thr Asp Phe Thr Leu Lys Ile
65                  70                  75                  80
Ser Arg Val Glu Thr Glu Asp Leu Gly Val Tyr Tyr Cys Trp Gln Gly
                85                  90                  95
Thr His Phe Pro Tyr Thr Phe Gly Gly Gly Thr Lys Leu Glu Leu Lys
            100                 105                 110
Arg

<210> 285
<211> 339
<212> DNA
<213> Mus musculus

<400> 285
gatgttgtga tgacccagac tccactcact ttgtcggtta ccattggaca accagcctcc 60
atctcttgca agtcaagtca gagcctctta gatagtgatg gagagacata tttgaattgg 120
ttgttacaga ggccaggcca gtctccaaag cgcctaatct atctggtgtc taaactggac 180
tctggagtcc ctgacaggtt cactggcagt ggatcaggga cagatttcac actgaaaatc 240
agcagagtgg agactgagga tttgggagtt tattattgct ggcaaggtac acattttccg 300
tacacgttcg gaggggggac caagctggaa ctgaaacgt                        339

<210> 286
<211> 116
<212> PRT

<213> Mus musculus

<400> 286
Glu Val Lys Leu Glu Glu Ser Gly Gly Gly Leu Val Gln Pro Gly Gly
1               5                   10                  15
Ser Met Lys Leu Ser Cys Val Ala Ser Gly Phe Thr Phe Ser Asn Tyr
            20                  25                  30
Trp Met Asn Trp Val Arg Gln Ser Pro Glu Lys Gly Leu Glu Trp Val
        35                  40                  45
Ala Glu Ile Arg Leu Lys Ser Asp Asn Tyr Ala Thr His Phe Ala Glu
    50                  55                  60
Ser Val Lys Gly Arg Phe Thr Ile Ser Arg Asp Asp Ser Lys Ser Ser
65                  70                  75                  80
Val Tyr Leu Gln Met Asn Asn Leu Arg Ala Glu Asp Thr Gly Ile Tyr
                85                  90                  95
Tyr Cys Thr Gly Ile Leu Phe Gly Tyr Trp Gly Gln Gly Thr Leu Val
            100                 105                 110
Thr Val Ser Ser
        115

<210> 287
<211> 348
<212> DNA
<213> Mus musculus

<400> 287
gaagtgaaac ttgaggagtc tggaggaggc ttggtgcaac ctggaggatc catgaaactc 60
tcctgtgttg cctctggatt cactttcagt aactactgga tgaactgggt ccgccagtct 120
ccagagaagg ggcttgagtg ggttgctgaa attagattga aatctgataa ttatgcaaca 180
cattttgcgg agtctgtgaa agggaggttc accatctcaa gagatgattc caaaagtagt 240
gtctacctgc aaatgaacaa cttaagagct gaagacactg gcatttatta ctgtacgggt 300
atcctctttg gttactgggg ccaagggact ctggtcactg tctcgagc 348

<210> 288
<211> 112
<212> PRT
<213> Mus musculus

<400> 288
Asp Ile Val Leu Thr Gln Ser Pro Ala Ser Leu Ala Val Ser Leu Gly
1               5                   10                  15
Gln Arg Ala Thr Ile Ser Cys Lys Ala Ser Gln Ser Val Asp Tyr Asp
            20                  25                  30
Gly Asp Ser Tyr Met Asn Trp Tyr Gln Gln Lys Pro Gly Gln Pro Pro
        35                  40                  45
Lys Leu Leu Ile Tyr Ala Ala Ser Asn Leu Glu Ser Gly Ile Pro Ala
    50                  55                  60
Arg Phe Ser Gly Ser Gly Ser Gly Thr Asp Phe Thr Leu Asn Ile His
65                  70                  75                  80
Pro Val Glu Glu Glu Asp Ala Ala Thr Tyr Tyr Cys Gln Gln Ser Asn
                85                  90                  95
Glu Asp Pro Trp Thr Phe Gly Gly Gly Thr Lys Leu Glu Leu Lys Arg
            100                 105                 110

<210> 289
<211> 336
<212> DNA
<213> Mus musculus

<400> 289
gacattgtgc tgacccaatc tccagcttct ttggctgtgt ctctagggca gagggccacc 60

atctcctgca aggccagcca aagtgttgat tatgatggtg atagttatat gaactggtac 120
caacagaaac caggacagcc acccaaactc ctcatctatg ctgcatccaa tctagaatct 180
gggatcccag ccaggtttag tggcagtggg tctgggacag acttcaccct caacatccat 240
cctgtggagg aggaggatgc tgctacctat tactgtcagc aaagtaatga ggatccgtgg 300
acgttcggtg gaggcaccaa gctggagctg aaacgt 336

<210> 290
<211> 117
<212> PRT
<213> Mus musculus

<400> 290
Gln Val Gln Leu Leu Gln Pro Gly Ala Glu Leu Val Lys Pro Gly Ala
1               5                   10                  15
Ser Val Lys Leu Ser Cys Lys Ala Ser Gly Tyr Thr Phe Thr Ser Tyr
            20                  25                  30
Trp Met His Trp Val Lys Gln Arg Pro Gly Gln Gly Leu Glu Trp Ile
        35                  40                  45
Gly Glu Ile Asn Pro Ser Asn Gly Arg Thr Asp Tyr Asn Glu Asn Phe
    50                  55                  60
Lys Ser Lys Ala Thr Leu Thr Val Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Gly Gly Val Tyr Ala Met Asp Tyr Trp Gly Gln Gly Thr Ser
            100                 105                 110
Val Thr Val Ser Ser
        115

<210> 291
<211> 351
<212> DNA
<213> Mus musculus

<400> 291
caggtccaac tgctgcagcc tggggctgaa ctggtgaagc ctggggcttc agtgaagctg 60
tcctgcaagg cttctggcta caccttcacc agctactgga tgcactgggt gaagcagagg 120
cctgggcaag gccttgagtg gattggagag attaatccta gcaacggtcg tactgactac 180
aatgagaact tcaagagcaa ggccacactg actgtagaca aatcctccag cacagcctac 240
atgcaactca gcagcctgac atctgaggac tctgcggtct attactgtgc aagagggggg 300
gtctatgcta tggactactg gggtcaagga acctcagtca ccgtctcgag c 351

<210> 292
<211> 113
<212> PRT
<213> Mus musculus

<400> 292
Asp Val Val Met Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asp Thr Tyr Leu His Trp Tyr Leu Gln Lys Pro Gly Gln Ser
        35                  40                  45
Pro Lys Leu Leu Ile Tyr Glu Ile Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Leu Gly Val Tyr Phe Cys Ser Gln Ser
                85                  90                  95
Thr His Val Pro Phe Thr Phe Gly Gly Gly Thr Lys Leu Glu Ile Lys
            100                 105                 110

Arg

<210> 293
<211> 339
<212> DNA
<213> Mus musculus

<400> 293
gatgttgtga tgacccaaac tccactctcc ctgcctgtca gtcttggaga tcaagcctcc 60
atctcttgca gatctagtca gagccttgta cacagtaatg gagacaccta tttacattgg 120
tacctacaga agccaggcca gtctccaaaa ctcctgatct acgaaatttc caaccgattt 180
tctggggtcc cagacaggtt cagtggcagt ggatcaggga cagatttcac actcaagatc 240
agcagagtgg aggctgagga tctgggagtt tatttctgct ctcaaagtac acatgttccg 300
ttcacgttcg gaggggggac caagctggaa ataaaacgt 339

<210> 294
<211> 116
<212> PRT
<213> Mus musculus

<400> 294
Glu Ile Gln Leu Gln Gln Ser Gly Thr Glu Leu Val Arg Ser Gly Ala
1               5                   10                  15
Ser Val Lys Leu Ser Cys Thr Ala Ser Gly Phe Asn Ile Lys Asp Tyr
            20                  25                  30
Tyr Ile His Trp Val Arg Gln Arg Pro Asp Gln Gly Leu Glu Trp Ile
        35                  40                  45
Gly Trp Ile Asp Pro Glu Asn Gly Asp Thr Glu Tyr Ala Pro Lys Phe
    50                  55                  60
Gln Asp Arg Ala Thr Leu Thr Ala Asp Thr Ser Ser Asn Thr Ala Tyr
65                  70                  75                  80
Leu Gln Leu Thr Leu Leu Thr Ser Glu Asp Thr Ala Val Tyr Tyr Cys
                85                  90                  95
Asn Glu Gly Ser Gly Leu Ile Pro Tyr Trp Gly Gln Gly Thr Pro Val
            100                 105                 110
Thr Val Ser Ser
        115

<210> 295
<211> 348
<212> DNA
<213> Mus musculus

<400> 295
gagattcagc tgcagcagtc tgggacagag cttgtgaggt caggggcctc agtcaaattg 60
tcctgcacag cttctggctt caacattaaa gactactata ttcactgggt gagacagagg 120
cctgaccagg gcctggagtg gattggatgg attgatcctg agaatggtga tactgaatat 180
gccccgaagt tccaggacag ggccactttg actgcagaca catcctccaa cacagcctac 240
ctgcagctca cactcctgac atctgaggac actgccgtct attactgtaa tgagggctcg 300
ggcttgattc cttactgggg ccaagggact ccggtcaccg tctcgagc 348

<210> 296
<211> 108
<212> PRT
<213> Mus musculus

<400> 296
Asp Ile Gln Met Thr Gln Thr Thr Ser Ser Leu Ser Ala Ser Leu Gly
1               5                   10                  15
Asp Arg Val Thr Ile Arg Cys Arg Ala Ser Gln Asp Ile Ser Asn Tyr

                20                  25                  30
Leu Asn Trp Tyr Gln Gln Lys Pro Asp Gly Pro Val Lys Leu Leu Ile
        35                  40                  45
Tyr Tyr Thr Ser Thr Leu Thr Ser Gly Val Pro Ser Arg Phe Ser Gly
    50                  55                  60
Ser Gly Ser Gly Thr Asp Tyr Ser Leu Thr Ile Ser Asn Leu Asp Gln
65                  70                  75                  80
Glu Asp Ile Ala Thr Tyr Phe Cys Gln Gln Gly Lys Thr Phe Pro Phe
                85                  90                  95
Thr Phe Gly Ser Gly Thr Lys Leu Glu Ile Lys Arg
                100                 105


<210> 297
<211> 324
<212> DNA
<213> Mus musculus

<400> 297
gatatccaga tgacacagac tacatcctcc ctgtctgcct ctctgggaga cagagtcacc 60
atccgttgca gggcaagtca ggacattagc aattatttaa actggtatca gcagaaacca 120
gatggacctg ttaaactcct gatctactac acatctacat tgacctcagg agtcccatca 180
aggttcagtg gcagtgggtc tggaacagat tattctctca ccattagcaa cctggaccaa 240
gaagatattg ccacttactt ttgccaacag ggtaagacgt ttccattcac gttcggctcg 300
gggacaaagt tggaaataaa acgt                                        324

<210> 298
<211> 120
<212> PRT
<213> Mus musculus

<400> 298
Gln Val Gln Leu Gln Gln Ser Gly Ala Glu Leu Val Arg Pro Gly Thr
 1               5                  10                  15
Ser Val Lys Val Ser Cys Lys Ala Ser Gly Tyr Val Phe Ile Asn Tyr
                20                  25                  30
Phe Ile Glu Trp Val Lys Gln Arg Pro Gly Gln Gly Leu Glu Trp Ile
        35                  40                  45
Gly Val Ile Asn Pro Glu Asn Gly Gly Thr Asn Tyr Asn Glu Arg Phe
    50                  55                  60
Lys Asp Lys Ala Ala Leu Thr Ala Asp Thr Ser Ser Ser Thr Ala Tyr
65                  70                  75                  80
Met Gln Leu Ser Ser Leu Thr Ser Asp Asp Ser Ala Val Tyr Phe Cys
                85                  90                  95
Ala Arg Glu Asp Tyr Gly Asp Val Tyr Ala Met Asp Tyr Trp Gly Gln
                100                 105                 110
Gly Thr Ser Val Thr Val Ser Ser
        115                 120


<210> 299
<211> 360
<212> DNA
<213> Mus musculus

<400> 299
caggtccagc tgcagcagtc tggagctgaa ctggtcaggc ctgggacttc agtgaaggtg 60
tcctgcaagg cttctggata cgtcttcatt aattacttca tagagtgggt taaacagagg 120
cctggacagg gccttgagtg gattggagtg attaatcctg aaaatggtgg tactaactac 180
aatgagagat tcaaggacaa ggcagcactg actgcagaca catcctccag cactgcctac 240
atgcaactca gcagcctgac atctgatgac tctgcggtct atttctgtgc aagagaggac 300
tatggtgatg tctatgctat ggactactgg ggtcaaggaa cctcagtcac agtctcgagc 360

<210> 300
<211> 113
<212> PRT
<213> Mus musculus

<400> 300
Asp Val Val Met Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asp Thr Tyr Leu His Trp Tyr Leu Gln Lys Pro Gly Gln Ser
        35                  40                  45
Pro Lys Leu Leu Ile Tyr Glu Ile Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Leu Gly Val Tyr Phe Cys Ser Gln Ser
                85                  90                  95
Thr His Val Pro Phe Thr Phe Gly Gly Gly Thr Lys Leu Glu Ile Lys
            100                 105                 110
Arg

<210> 301
<211> 339
<212> DNA
<213> Mus musculus

<400> 301
gatgttgtga tgacccaaac tccactctcc ctgcctgtca gtcttggaga tcaagcctcc 60
atctcttgca gatctagtca gagccttgta cacagtaatg gagacaccta tttacattgg 120
tacctacaga agccaggcca gtctccaaaa ctcctgatct acgaaatttc caaccgattt 180
tctggggtcc cagacaggtt cagtggcagt ggatcaggga cagatttcac actcaagatc 240
agcagagtgg aggctgagga tctgggagtt tatttctgct ctcaaagtac acatgttccg 300
ttcacgttcg gaggggggac caagctggaa ataaaacgt                        339

<210> 302
<211> 116
<212> PRT
<213> Mus musculus

<400> 302
Glu Val Gln Leu Gln Gln Ser Gly Thr Glu Leu Val Arg Ser Gly Ala
1               5                   10                  15
Ser Val Arg Leu Ser Cys Thr Ala Ser Gly Phe Asn Ile Lys Asp Tyr
            20                  25                  30
Tyr Ile His Trp Val Lys Gln Arg Pro Glu Gln Gly Leu Glu Trp Ile
        35                  40                  45
Gly Trp Ile Asp Pro Glu Asn Gly Asp Ser Glu Tyr Ala Pro Lys Phe
    50                  55                  60
Gln Asp Lys Ala Thr Met Thr Ala Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Asn Glu Gly Ser Gly Leu Ile Pro Tyr Trp Gly His Gly Thr Leu Val
            100                 105                 110
Thr Val Ser Ser
        115

<210> 303
<211> 348

<212> DNA
<213> Mus musculus

<400> 303
gaggttcagc tgcagcagtc tgggacagag cttgtgaggt caggggcctc agtcaggttg 60
tcctgcacag cttctggctt caacattaaa gactactata tacactgggt gaaacagagg 120
cctgaacagg gcctggagtg gattggatgg attgatcctg agaatggtga ttctgaatat 180
gccccgaagt tccaggacaa ggccaccatg actgcagaca catcctccaa cacagcctac 240
ctgcagctca gcagcctgac atctgaggac actgccgtct attattgtaa tgaggctcgg 300
ggcttgattc cttactgggg ccacgggact ctggtcacag tctcgagc 348

<210> 304
<211> 113
<212> PRT
<213> Mus musculus

<400> 304
Asp Ile Val Leu Thr Gln Thr Pro Leu Ser Leu Pro Val Ser Leu Gly
1               5                   10                  15
Asp His Ala Ser Ile Ser Cys Arg Ser Ser Gln Ser Leu Val His Ser
            20                  25                  30
Asn Gly Asp Val Tyr Phe His Trp Tyr Leu Gln Lys Pro Gly Gln Ser
        35                  40                  45
Pro Lys Leu Leu Ile Tyr Glu Val Ser Asn Arg Phe Ser Gly Val Pro
    50                  55                  60
Asp Arg Phe Ser Gly Ser Gly Ser Gly Thr Tyr Phe Thr Leu Lys Ile
65                  70                  75                  80
Ser Arg Val Glu Ala Glu Asp Leu Gly Val Tyr Phe Cys Ser Gln Ser
                85                  90                  95
Thr His Val Pro Tyr Thr Phe Gly Gly Gly Thr Lys Leu Glu Leu Lys
            100                 105                 110
Arg

<210> 305
<211> 339
<212> DNA
<213> Mus musculus

<400> 305
gatattgtgt tgacccaaac tcctctctct ctgcctgtca gtcttggaga tcatgcctcc 60
atctcttgca gatctagtca gagccttgta cacagtaatg gcgacgtcta tttccattgg 120
tacctgcaga agccaggcca gtctccaaag ctcctgatct acgaagtttc caaccgattt 180
tctggggtcc cagacaggtt cagtggcagt ggatcaggga catatttcac actcaagatc 240
agcagagtgg aggctgagga tctgggagtt tatttctgct ctcaaagtac acatgttccg 300
tacactttcg gaggggggac caagttggag ctcaaacgt 339

<210> 306
<211> 116
<212> PRT
<213> Mus musculus

<400> 306
Glu Val Gln Leu Gln Gln Ser Gly Thr Glu Leu Val Arg Ser Gly Ala
1               5                   10                  15
Ser Val Arg Leu Ser Cys Thr Ala Ser Gly Phe Asn Ile Lys Asp Tyr
            20                  25                  30
Tyr Ile His Trp Val Lys Gln Arg Pro Glu Gln Gly Leu Glu Trp Ile
        35                  40                  45
Gly Trp Ile Asp Pro Glu Asn Gly Asp Ser Glu Tyr Ala Pro Lys Phe
    50                  55                  60
Gln Asp Lys Ala Thr Met Thr Ala Asp Thr Ser Ser Asn Thr Ala Tyr

65                  70                  75                  80
Leu Gln Leu Ser Ser Leu Thr Ser Glu Asp Thr Ala Val Tyr Tyr Cys
                85                  90                  95
Asn Glu Gly Ser Gly Leu Ile Pro Tyr Trp Gly His Gly Thr Leu Val
            100                 105                 110
Thr Val Ser Ser
        115

<210> 307
<211> 348
<212> DNA
<213> Mus musculus

<400> 307
gaggttcagc tgcagcagtc tgggacagag cttgtgaggt caggggcctc agtcaggttg 60
tcctgcacag cttctggctt caacattaaa gactactata tacactgggt gaaacagagg 120
cctgaacagg gcctggagtg gattggatgg attgatcctg agaatggtga ttctgaatat 180
gccccgaagt tccaggacaa ggccaccatg actgcagaca catcctccaa cacagcctac 240
ctgcagctca gcagcctgac atctgaggac actgccgtct attattgtaa tgagggctcg 300
ggcttgattc cttactgggg ccacgggact ctggtcacag tctcgagc             348

<210> 308
<211> 112
<212> PRT
<213> Mus musculus

<400> 308
Asp Val Val Leu Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asp Val Tyr Leu His Trp Tyr Leu Gln Lys Pro Gly Gln Ser
        35                  40                  45
Pro Lys Leu Leu Ile Tyr Glu Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Asn Arg Val Glu Ala Glu Asp Leu Gly Val Tyr Phe Cys Ser Gln Ser
                85                  90                  95
Thr His Val Pro Tyr Thr Phe Gly Gly Gly Thr Lys Leu Glu Leu Lys
            100                 105                 110

<210> 309
<211> 339
<212> DNA
<213> Mus musculus

<400> 309
gatgttgtgt tgacccaaac tccactctcc ctgcctgtca gtcttggaga tcaagcctcc 60
atctcttgca gatctagtca gagccttgta cacagtaatg gagacgtcta tttacattgg 120
tacctgcaga agccaggcca gtctccaaag ctcctgatct acgaagtttc caaccgattt 180
tctggggtcc cagacaggtt cagtggcagt ggatcaggga cagatttcac actcaagatc 240
aacagagtgg aggctgagga tctgggagtt tatttctgct ctcaaagtac acatgttccg 300
tacacgttcg gaggggggac caagctggaa ctgaaacgt                       339

<210> 310
<211> 116
<212> PRT
<213> Mus musculus

<400> 310

Glu Val Gln Leu Gln Gln Ser Gly Thr Glu Leu Val Arg Ser Gly Ala
1               5                   10                  15
Ser Val Lys Leu Ser Cys Thr Ala Ser Gly Phe Asn Ile Lys Asp Tyr
            20                  25                  30
Tyr Val His Trp Val Lys Gln Arg Pro Glu Gln Gly Leu Glu Trp Ile
        35                  40                  45
Gly Trp Ile Asp Pro Asp Asn Gly Asp Ser Glu Tyr Ala Pro Lys Phe
    50                  55                  60
Gln Asp Lys Ala Thr Met Thr Ala Asp Thr Ser Ser Asn Thr Ala Tyr
65                  70                  75                  80
Leu Gln Leu Asn Ser Leu Thr Ser Glu Asp Thr Ala Val Tyr Tyr Cys
                85                  90                  95
Asn Glu Gly Ser Gly Leu Ile Pro Tyr Trp Gly Pro Gly Thr Leu Val
            100                 105                 110
Thr Val Ser Ser
        115

<210> 311
<211> 348
<212> DNA
<213> Mus musculus

<400> 311
gaggttcagc tgcagcagtc tgggacagag cttgtgaggt caggggcctc agtcaagttg 60
tcctgcacag cttctggctt caacattaaa gactactatg tacactgggt gaagcagagg 120
cctgaacagg gcctggaatg gattggatgg attgatcctg acaatggtga tagtgaatat 180
gccccgaagt tccaggacaa ggccactatg actgcagaca catcctccaa cacagcctac 240
cttcaactca acagcctgac ttctgaggac actgccgtct attattgtaa tgagggctcg 300
ggcttgattc cttactgggg cccagggact ctggtcacag tctcgagc             348

<210> 312
<211> 113
<212> PRT
<213> Mus musculus

<400> 312
Asp Val Val Met Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asp Val Tyr Leu His Trp Tyr Leu Gln Lys Pro Gly Gln Ser
        35                  40                  45
Pro Lys Leu Leu Ile Tyr Glu Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Leu Gly Val Tyr Phe Cys Ser Gln Thr
                85                  90                  95
Thr His Val Pro Tyr Thr Phe Gly Gly Gly Thr Lys Leu Glu Leu Lys
            100                 105                 110
Arg

<210> 313
<211> 339
<212> DNA
<213> Mus musculus

<400> 313
gatgttgtga tgacccaaac tccactctcc ctgcctgtca gtcttggaga tcaagcctcc 60
atctcctgca gatctagtca gagccttgta cacagtaatg gagacgtcta tttacattgg 120

tacctgcaga agccaggcca gtctccaaaa ctcctgatct acgaagtttc caaccgattt 180
tctggggtcc cagacaggtt cagtggcagt ggttcaggga cagatttcac actcaagatc 240
agcagagtgg aggctgagga tctgggagtt tatttctgtt ctcaaactac acatgttccg 300
tacacgttcg gaggggggac caagctggag ctgaaacgt 339

<210> 314
<211> 116
<212> PRT
<213> Mus musculus

<400> 314
Glu Ile Gln Leu Gln Gln Ser Gly Thr Glu Leu Val Arg Ser Gly Ala
1               5                   10                  15
Ser Val Lys Leu Ser Cys Thr Ala Ser Gly Phe Asn Ile Lys Asp Tyr
            20                  25                  30
Tyr Ile His Trp Val Arg Gln Arg Pro Asp Gln Gly Leu Glu Trp Ile
        35                  40                  45
Gly Trp Ile Asp Pro Glu Asn Gly Asp Thr Glu Tyr Ala Pro Lys Phe
    50                  55                  60
Gln Asp Arg Ala Thr Leu Thr Ala Asp Thr Ser Ser Asn Thr Ala Tyr
65                  70                  75                  80
Leu Gln Leu Thr Leu Leu Thr Ser Glu Asp Thr Ala Val Tyr Tyr Cys
                85                  90                  95
Asn Glu Gly Ser Gly Leu Ile Pro Tyr Trp Gly Gln Gly Thr Pro Val
            100                 105                 110
Thr Val Ser Ser
        115

<210> 315
<211> 348
<212> DNA
<213> Mus musculus

<400> 315
gagattcagc tgcagcagtc tgggacagag cttgtgaggt caggggcctc agtcaaattg 60
tcctgcacag cttctggctt caacattaaa gactactata ttcactgggt gagacagagg 120
cctgaccagg gcctggagtg gattggatgg attgatcctg agaatggtga tactgaatat 180
gccccgaagt tccaggacag ggccactttg actgcagaca catcctccaa cacagcctac 240
ctgcagctca cactcctgac atctgaggac actgccgtct attactgtaa tgagggctcg 300
ggcttgattc cttactgggg ccaagggact ccggtcaccg tctcgagc 348

<210> 316
<211> 113
<212> PRT
<213> Mus musculus

<400> 316
Asp Val Val Met Thr Gln Thr Pro Leu Ser Leu Pro Val Ser Leu Gly
1               5                   10                  15
Asp Gln Ala Ser Ile Ser Cys Arg Ser Ser Gln Ser Leu Val His Ser
            20                  25                  30
Asn Gly Asp Val Tyr Leu His Trp Tyr Leu Gln Lys Pro Gly Gln Ser
        35                  40                  45
Pro Lys Leu Leu Ile Tyr Glu Val Ser Asn Arg Phe Ser Gly Val Pro
    50                  55                  60
Asp Arg Phe Thr Gly Ser Gly Ser Gly Thr Asp Phe Thr Leu Lys Ile
65                  70                  75                  80
Ser Arg Val Glu Ala Glu Asp Leu Gly Val Phe Phe Cys Ser Gln Ser
                85                  90                  95
Thr His Val Pro Tyr Thr Phe Gly Gly Gly Thr Lys Leu Glu Leu Lys
            100                 105                 110
Arg

<210> 317
<211> 339
<212> DNA
<213> Mus musculus

<400> 317
gatgttgtga tgacccaaac tccactctcc ctgcctgtca gtcttggaga tcaagcctcc 60
atctcctgca gatctagtca gagccttgta cacagtaatg gagacgtcta tttacattgg 120
tacctgcaga agccaggcca gtctccaaaa ctcctgatct acgaagtttc caaccgattt 180
tctggggtcc cagacaggtt cactggcagt ggttcaggga cagatttcac actcaagatc 240
agcagagtgg aggctgagga tctgggagtt tttttctgct ctcaaagtac acatgttccg 300
tacacgttcg gaggggggac caagctggaa ctgaaacgt 339

<210> 318
<211> 116
<212> PRT
<213> Mus musculus

<400> 318
Glu Ile Gln Leu Gln Gln Ser Gly Thr Glu Leu Val Arg Ser Gly Ala
1               5                   10                  15
Ser Val Lys Leu Ser Cys Thr Ala Ser Gly Phe Asn Ile Lys Asp Tyr
            20                  25                  30
Tyr Ile His Trp Val Lys Gln Arg Pro Asp Gln Gly Leu Glu Trp Ile
        35                  40                  45
Gly Trp Ile Asp Pro Glu Asn Gly Asp Thr Glu Tyr Ala Pro Lys Phe
    50                  55                  60
Gln Asp Arg Ala Thr Met Thr Ala Asp Thr Ser Ser Asn Thr Ala Tyr
65                  70                  75                  80
Leu Gln Leu Thr Ile Leu Thr Ser Glu Asp Thr Ala Val Tyr Tyr Cys
                85                  90                  95
Asn Glu Gly Ser Gly Leu Ile Pro Tyr Trp Gly Gln Gly Thr Leu Val
            100                 105                 110
Thr Val Ser Ser
        115

<210> 319
<211> 348
<212> DNA
<213> Mus musculus

<400> 319
gagattcagc tgcagcagtc tgggacagag cttgtgaggt caggggcctc agtcaaattg 60
tcctgcacag cttctggctt caacattaaa gactactata ttcactgggt gaaacagagg 120
cctgaccagg gcctggagtg gattggatgg attgatcctg agaatggtga tactgaatat 180
gccccgaagt tccaggacag ggccactatg actgcagaca catcctccaa cacagcctac 240
ctgcagctca ccatcctgac atctgaggac actgccgtct attactgtaa tgagggctcg 300
ggcttgattc cttactgggg ccaagggact ctggtcaccg tctcgagc 348

<210> 320
<211> 108
<212> PRT
<213> Mus musculus

<400> 320
Asp Ile Gln Met Asn Gln Ser Pro Ser Ser Leu Ser Ala Ser Leu Gly
1               5                   10                  15
Asp Thr Ile Thr Ile Thr Cys His Ala Ser Gln Asn Ile Asn Val Trp
            20                  25                  30

Leu Ser Trp Tyr Gln Gln Lys Pro Gly Asn Ile Pro Lys Leu Leu Ile
        35                  40                  45
Tyr Lys Val Ser Asn Leu His Thr Gly Val Pro Ser Arg Phe Ser Gly
    50                  55                  60
Ser Gly Ser Gly Thr Gly Phe Thr Leu Thr Ile Ser Ser Leu Gln Pro
65                  70                  75                  80
Glu Asp Ile Ala Thr Tyr Tyr Cys Gln Gln Gly Gln Ser Tyr Pro Leu
                85                  90                  95
Thr Phe Gly Gly Gly Thr Lys Leu Glu Leu Lys Arg
            100                 105

<210> 321
<211> 324
<212> DNA
<213> Mus musculus

<400> 321
gacatccaga tgaaccagtc tccatccagt ctgtctgcat cccttggaga cacaattacc 60
atcacttgcc atgccagtca gaacattaat gtttggttaa gctggtacca gcagaaacca 120
ggaaatattc ctaaactatt gatctataag gtttccaact tgcacacagg cgtcccatca 180
aggtttagtg gcagtggatc tggaacaggt ttcacattaa ccatcagcag cctgcagcct 240
gaagacattg ccacttacta ctgtcaacag ggtcaaagtt atcctctgac gttcggtgga 300
ggcaccaagt tggagctcaa acgt                                        324

<210> 322
<211> 120
<212> PRT
<213> Mus musculus

<400> 322
Glu Val Gln Leu Gln Gln Ser Gly Ala Glu Leu Val Arg Pro Gly Ala
1               5                   10                  15
Leu Val Lys Leu Ser Cys Lys Ala Ser Gly Phe Asn Ile Lys Asp Tyr
            20                  25                  30
Tyr Ile His Trp Val Lys Gln Arg Pro Glu Gln Gly Leu Glu Trp Ile
        35                  40                  45
Gly Arg Ile Asp Pro Glu Asn Gly Asn Thr Ile Tyr Asp Pro Lys Phe
    50                  55                  60
Gln Gly Lys Ala Ile Ile Thr Ala Asp Thr Ser Ser Asn Thr Ala Tyr
65                  70                  75                  80
Leu Gln Leu Ser Ser Leu Thr Ser Glu Asp Thr Ala Val Tyr Tyr Cys
                85                  90                  95
Ala Arg Cys Asp Asn Asp Pro Gly Ser Glu Met Asp Tyr Trp Gly Gln
            100                 105                 110
Gly Thr Thr Val Thr Val Ser Ser
        115                 120

<210> 323
<211> 360
<212> DNA
<213> Mus musculus

<400> 323
gaggttcagc tgcagcagtc tggggctgag cttgtgaggc caggggcctt agtcaaattg 60
tcctgcaaag cttctggatt caacattaaa gactactata tacactgggt gaagcagagg 120
cctgaacagg gcctggagtg gattggaagg attgatcctg aaaatggtaa tactatatat 180
gacccgaagt tccagggcaa ggccattata acagcagaca catcctccaa cacagcctac 240
ctgcagctca gcagcctgac atctgaggac actgccgtct attactgtgc tagatgtgat 300
aacgaccccg gctctgaaat ggactactgg ggtcaaggaa ccacggtcac cgtctcgagc 360

<210> 324
<211> 113
<212> PRT
<213> Mus musculus

<400> 324
Asp Ile Val Leu Thr Gln Thr Pro Leu Ser Leu Pro Val Ser Leu Gly
1               5                   10                  15
Asp His Ala Ser Ile Ser Cys Arg Ser Ser Gln Ser Leu Val His Ser
            20                  25                  30
Asn Gly Asp Val Tyr Phe His Trp Tyr Leu Gln Lys Pro Gly Gln Ser
        35                  40                  45
Pro Lys Leu Leu Ile Tyr Glu Val Ser Asn Arg Phe Ser Gly Val Pro
    50                  55                  60
Asp Arg Phe Ser Gly Ser Gly Ser Gly Thr Tyr Phe Thr Leu Lys Ile
65                  70                  75                  80
Ser Arg Val Glu Ala Glu Asp Leu Gly Val Tyr Phe Cys Ser Gln Ser
                85                  90                  95
Thr His Val Pro Tyr Thr Phe Gly Gly Gly Thr Lys Leu Glu Leu Lys
            100                 105                 110
Arg

<210> 325
<211> 339
<212> DNA
<213> Mus musculus

<400> 325
gatattgtgt tgacccaaac tcctctctct ctgcctgtca gtcttggaga tcatgcctcc 60
atctcttgca gatctagtca gagccttgta cacagtaatg gcgacgtcta tttccattgg 120
tacctgcaga agccaggcca gtctccaaag ctcctgatct acgaagtttc caaccgattt 180
tctggggtcc cagacaggtt cagtggcagt ggatcaggga catatttcac actcaagatc 240
agcagagtgg aggctgagga tctgggagtt tatttctgct ctcaaagtac acatgttccg 300
tacactttcg gaggggggac caagttggag ctcaaacgt                        339

<210> 326
<211> 116
<212> PRT
<213> Mus musculus

<400> 326
Glu Val Gln Leu Gln Gln Ser Gly Thr Glu Leu Val Arg Ser Gly Ala
1               5                   10                  15
Ser Val Lys Leu Ser Cys Thr Ala Ser Gly Phe Asn Ile Lys Asp Tyr
            20                  25                  30
Tyr Val His Trp Val Lys Gln Arg Pro Glu Gln Gly Leu Glu Trp Ile
        35                  40                  45
Gly Trp Ile Asp Pro Asp Asn Gly Asp Ser Glu Tyr Ala Pro Lys Phe
    50                  55                  60
Gln Asp Lys Ala Thr Met Thr Ala Asp Thr Ser Ser Asn Thr Ala Tyr
65                  70                  75                  80
Leu Gln Leu Asn Ser Leu Thr Ser Glu Asp Thr Ala Val Tyr Tyr Cys
                85                  90                  95
Asn Glu Gly Ser Gly Leu Ile Pro Tyr Trp Gly Pro Gly Thr Leu Val
            100                 105                 110
Thr Val Ser Ser
        115

<210> 327
<211> 348

<212> DNA
<213> Mus musculus

<400> 327
gaggttcagc tgcagcagtc tgggacagag cttgtgaggt caggggcctc agtcaagttg 60
tcctgcacag cttctggctt caacattaaa gactactatg tacactgggt gaagcagagg 120
cctgaacagg gcctggaatg gattggatgg attgatcctg acaatggtga tagtgaatat 180
gccccgaagt tccaggacaa ggccactatg actgcagaca catcctccaa cacagcctac 240
cttcaactca acagcctgac ttctgaggac actgccgtct attattgtaa tgaggctcgg 300
ggcttgattc cttactgggg cccagggact ctggtcacag tctcgagc 348

## Description

### TECHNICAL FIELD

The present invention relates generally to epitopes of sclerostin protein, including human sclerostin protein, and binding agents (such as antibodies) capable of binding to sclerostin or fragments thereof.

### BACKGROUND OF THE INVENTION

Two or three distinct phases of changes to bone mass occur over the life of an individual (*see* Riggs, West J. Med., 154:63-77 (1991)). The first phase occurs in both men and women and proceeds to attainment of a peak bone mass. This first phase is achieved through linear growth of the endochondral growth plates and radial growth due to a rate of periosteal apposition. The second phase begins around age 30 for trabecular bone (flat bones such as the vertebrae and pelvis) and about age 40 for cortical bone (e.g., long bones found in the limbs) and continues to old age. This phase is characterized by slow bone loss and occurs in both men and women. In women, a third phase of bone loss also occurs, most likely due to postmenopausal estrogen deficiencies. During this phase alone, women may lose an additional bone mass from the cortical bone and from the trabecular compartment (*see* Riggs, *supra*)*.*

Loss of bone mineral content can be caused by a wide variety of conditions and may result in significant medical problems. For example, osteoporosis is a debilitating disease in humans and is characterized by marked decreases in skeletal bone mass and mineral density, structural deterioration of bone, including degradation of bone microarchitecture and corresponding increases in bone fragility (i.e., decreases in bone strength), and susceptibility to fracture in afflicted individuals. Osteoporosis in humans is generally preceded by clinical osteopenia (bone mineral density that is greater than one standard deviation but less than 2.5 standard deviations below the mean value for young adult bone), a condition found in approximately 25 million people in the United States. Another 7-8 million patients in the United States have been diagnosed with clinical osteoporosis (defined as bone mineral content greater than 2.5 standard deviations below that of mature young adult bone). The frequency of osteoporosis in the human population increases with age. Among Caucasians, osteoporosis is predominant in women who, in the United States, comprise 80% of the osteoporosis patient pool. The increased fragility and susceptibility to fracture of skeletal bone in the aged is aggravated by the greater risk of accidental falls in this population. Fractured hips, wrists, and vertebrae are among the most common injuries associated with osteoporosis. Hip fractures in particular are extremely uncomfortable and expensive for the patient, and for women, correlate with high rates of mortality and morbidity.

Although osteoporosis has been regarded as an increase in the risk of fracture due to decreased bone mass, few of the presently available treatments for skeletal disorders can increase the bone density of adults, and most of the presently available treatments work primarily by inhibiting further bone resorption rather than stimulating new bone formation. Estrogen is now being prescribed to retard bone loss. However, some controversy exists over whether patients gain any long-term benefit and whether estrogen has any effect on patients over 75 years old. Moreover, use of estrogen is believed to increase the risk of breast and endometrial cancer. Calcitonin, osteocalcin with vitamin K, or high doses of dietary calcium, with or without vitamin D, have also been suggested for postmenopausal women. High doses of calcium, however, often have undesired gastrointestinal side effects, and serum and urinary calcium levels must be continuously monitored (*e.g*., Khosla and Riggs, Mayo Clin. Proc., 70:978982 (1995)).

Other current therapeutic approaches to osteoporosis include bisphosphonates (*e.g*., Fosamax^{™}, Actonel^{™}, Bonviva^{™}, Zometa^{™}, olpadronate, neridronate, skelid, bonefos), parathyroid hormone, calcilytics, calcimimetics (*e.g*., cinacalcet), statins, anabolic steroids, lanthanum and strontium salts, and sodium fluoride. Such therapeutics, however, are often associated with undesirable side effects *(see* Khosla and Riggs, *supra*)*.*

Sclerostin, the product of the SOST gene, is absent in sclerosteosis, a skeletal disease characterized by bone overgrowth and strong dense bones (Brunkow et al., Am. J. Hum. Genet., 68:577-589 (2001); Balemans et al., Hum. Mol. Genet., 10:537-543 (2001)). The amino acid sequence of human sclerostin is reported by *Brunkow et al. ibid* and is disclosed herein as SEQ ID NO:1.

### BRIEF SUMMARY OF THE INVENTION

The invention relates to an isolated antibody selected from the group consisting of antibodies AA, BB, CC, DD, EE, FF, GG, HH, II, JJ, KK, LL, MM, NN, OO, PP, QQ, RR, SS, TT, UU, VV, and WW; the isolated antibody, or an antigen-binding fragment thereof, may be a polyclonal antibody, a monoclonal antibody, a humanized antibody, a human antibody, or a human/non-human chimeric antibody, such as a mouse/human or rabbit/human chimeric antibody.

The invention further relates to a methods for detecting, diagnosing, and determining the progression or regression of a bone disorder associated with at least one of low bone mass, low bone mineral density, and poor bone quality in a mammalian subject which comprises obtaining a biological sample from a subject suspected of suffering from the disorder, contacting the biological sample with an agent capable of detecting sclerostin, and identifying or quantitating a binding complex between the agent and sclerostin, wherein the agent comprises an anti-sclerostin antibody, or sclerostin-binding fragment thereof.

Provided herein are antibodies that specifically bind to human sclerostin. The antibodies can be characterized by their ability to bind to human sclerostin or a fragment thereof.

Also provided is an isolated antibody, or an antigen-binding fragment thereof, that specifically binds to human sclerostin and has at least one CDR sequence selected from SEQ ID NO:101, 104, 110, 116, 122, 128, 134, 140, 146, 152, 158, 164, 170, 176, 182, 188, 194, 200, 206, 212, 218, 224, and 230 for CDR-L1;SEQ ID NO:102, 105, 111, 117, 123, 129, 135, 141, 147, 153, 159, 165, 171, 177, 183, 189, 195, 201, 207, 213, 219, 225, 231 for CDR-L2; SEQ ID NO:103, 106, 112, 118, 124, 130, 136, 142, 148, 154, 160, 166, 172, 178, 184, 190, 196, 202, 208, 214, 220, 226, and 232 for CDR-L3; SEQ ID NO: 98, 107, 113, 119, 125, 131, 137, 143, 149, 155, 161, 167, 173, 179, 185, 191, 197, 203, 209, 215, 221, 227, and 233 for CDR-H1; SEQ ID NO:99, 108, 114, 120, 132, 144, 150, 156, 162, 168, 174, 180, 186, 192, 198, 204, 210, 216, 222, 228, and 234 for CDR-H2; SEQ ID NO:100, 109, 115, 121, 127, 133, 139, 145, 151, 157, 163, 169, 175, 181, 187, 193, 199, 205, 211, 217, 223, 229, and 235 for CDR-H3; and variants thereof.

Further provided is a method for treating a bone disorder associated with at least one of low bone mass, low bone mineral density, and poor bone quality in a mammalian subject which comprises providing to a subject in need of such treatment an amount of an anti-sclerostin agent sufficient to modulate at least one of low bone mass, low bone mineral density, and poor bone quality wherein the anti-sclerostin agent comprises an antibody, or sclerostin-binding fragment thereof.

These and other aspects of the present invention will become apparent upon reference to the following detailed description and attached drawings. All references disclosed herein are hereby incorporated by reference in their entireties as if each was incorporated individually.

### BRIEF DESCRIPTION OF THE DRAWINGS

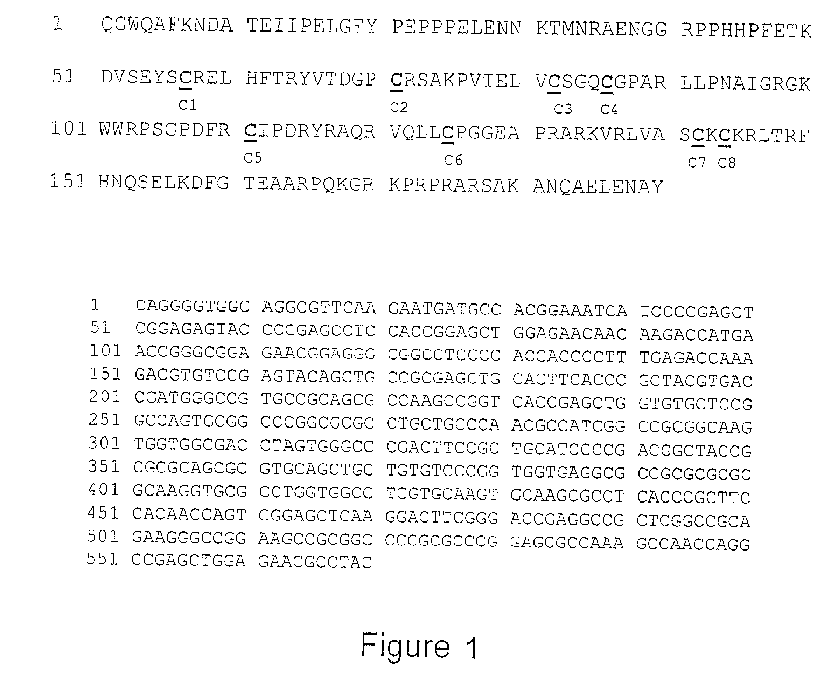

Figure 1 depicts the amino acid sequence of the mature form (signal peptide cleaved off) of human sclerostin (SEQ ID NO:1). Also depicted is the nucleotide sequence of the human sclerostin coding region that encodes the mature form of human sclerostin. The eight cysteines are numbered C1 through C8. The cystine-knot is formed by three disulfide bonds (C1-C5; C3-C7; C4-C8). C2 and C6 also form a disulfide bond, however this disulfide is not part of the cystine-knot.

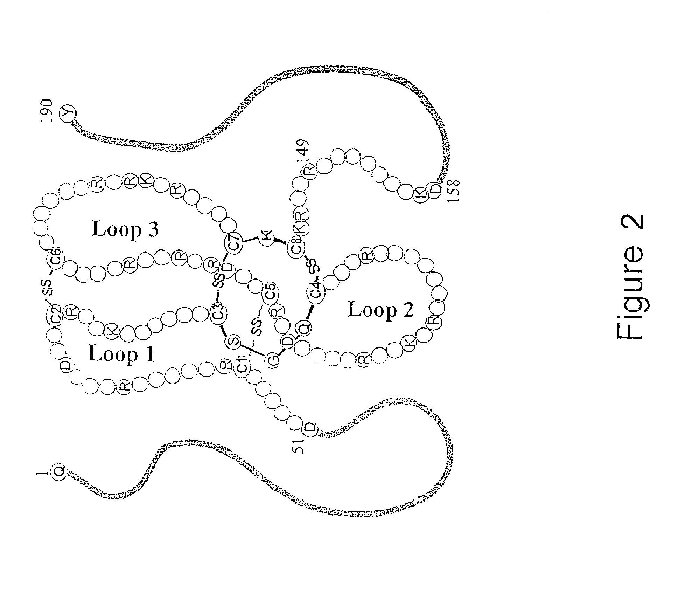

Figure 2 depicts a schematic of the basic structure of human sclerostin. There is an N-terminal arm (from the first Q to C1) and a C-terminal arm (from C8 to the terminal Y). In between these arms there is the cystine-knot structure (formed by three disulfides: C1-C5; C3-C7; C4-C8) and three loops which are designated Loop 1, Loop 2 and Loop 3. The distal regions of Loop 1 and Loop 3 are linked by the C2-C6 disulfide. Potential trypsin cleavage sites are indicated (arginine=R and lysine=K). Some of the potential AspN cleavage sites are indicated [only aspartic acid (D) residues are shown].

### DETAILED DESCRIPTION

The present invention relates to regions of the human sclerostin protein that contain epitopes recognized by antibodies that also bind to full-length sclerostin, and methods of making and using these epitopes. The invention also provides binding agents (such as antibodies) that specifically bind to sclerostin or portions of sclerostin, and methods for using such binding agents. The binding agents are useful to block or impair binding of human sclerostin to one or more ligand.

Recombinant human sclerostin/SOST is commercially available from R&D Systems (Minneapolis, MN, USA; 2006 cat# 1406-ST-025). Additionally, recombinant mouse sclerostin/SOST is commercially available from R&D Systems (Minneapolis, MN, USA; 2006 cat# 1589-ST-025). Research grade sclerostin binding monoclonal antibodies are commercially available from R&D Systems (Minneapolis, MN, USA; mouse monoclonal: 2006 cat# MAB1406; rat monoclonal: 2006 cat# MAB1589). U.S. Patent Nos. 6,395,511 and 6,803,453, and U.S. Patent Publications 2004/0009535 and 2005/0106683 refer to anti-sclerostin antibodies generally.

As used herein, the term human sclerostin is intended to include the protein of SEQ ID NO:1 and allelic variants thereof. Sclerostin can be purified from 293T host cells that have been transfected by a gene encoding sclerostin by elution of filtered supernatant of host cell culture fluid using a Heparin HP column, using a salt gradient. The preparation and further purification using cation exchange chromatography are described in Examples 1 and 2.

Binding agents of the invention are preferably antibodies, as defined herein. The term "antibody" refers to an intact antibody, or a binding fragment thereof. An antibody may comprise a complete antibody molecule (including polyclonal, monoclonal, chimeric, humanized, or human versions having full length heavy and/or light chains), or comprise an antigen binding fragment thereof. Antibody fragments include F(ab')₂, Fab, Fab', Fv, Fc, and Fd fragments, and can be incorporated into single domain antibodies, single-chain antibodies, maxibodies, minibodies, intrabodies, diabodies, triabodies, tetrabodies, v-NAR and bis-scFv (*see e.g.,,* Hollinger and Hudson, Nature Biotechnology, 23(9):1126-1136 (2005)). Antibody polypeptides are also disclosed in U. S. Patent No. 6,703,199, including fibronectin polypeptide monobodies. Other antibody polypeptides are disclosed in U.S. Patent Publication 2005/0238646, which are single-chain polypeptides.

Antigen binding fragments derived from an antibody can be obtained, for example, by proteolytic hydrolysis of the antibody, for example, pepsin or papain digestion of whole antibodies according to conventional methods. By way of example, antibody fragments can be produced by enzymatic cleavage of antibodies with pepsin to provide a 5S fragment termed F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using papain produces two monovalent Fab fragments and an Fc fragment directly. These methods are described, for example, by Goldenberg, U.S. Patent No. 4,331,647, Nisonoff et al., Arch. Biochem. Biophys., 89:230 (1960); Porter, Biochem. J., 73:119 (1959); Edelman et al., in Methods in Enzymology, 1:422 (Academic Press 1967); and by Andrews, S.M. and Titus, J.A. in Current Protocols in Immunology (Coligan J.E., et al., eds.), John Wiley & Sons, New York (2003), pages 2.8.1-2.8.10 and 2.10A.1-2.10A.5. Other methods for cleaving antibodies, such as separating heavy chains to form monovalent light-heavy chain fragments (Fd), further cleaving of fragments, or other enzymatic, chemical, or genetic techniques may also be used, so long as the fragments bind to the antigen that is recognized by the intact antibody.

An antibody fragment may also be any synthetic or genetically engineered protein. For example, antibody fragments include isolated fragments consisting of the light chain variable region, "Fv" fragments consisting of the variable regions of the heavy and light chains, and recombinant single chain polypeptide molecules in which light and heavy variable regions are connected by a peptide linker (scFv proteins).

Another form of an antibody fragment is a peptide comprising one or more complementarity determining regions (CDRs) of an antibody. CDRs (also termed "minimal recognition units," or "hypervariable region") can be obtained by constructing polynucleotides that encode the CDR of interest. Such polynucleotides are prepared, for example, by using the polymerase chain reaction to synthesize the variable region using mRNA of antibody-producing cells as a template (*see,* for example, Larrick et al., Methods: A Companion to Methods in Enzymology 2:106, 1991; Courtenay-Luck, "Genetic Manipulation of Monoclonal Antibodies," in Monoclonal Antibodies: Production, Engineering and Clinical Application, Ritter et al. (eds.), page 166 (Cambridge University Press 1995); and Ward et al., "Genetic Manipulation and Expression of Antibodies," in Monoclonal Antibodies: Principles and Applications, Birch et al., (eds.), page 137 (Wiley-Liss, Inc. 1995)).

Thus, in one embodiment, the binding agent comprises at least one CDR as described herein. The binding agent may comprise at least two, three, four, five or six CDR's as described herein. The binding agent further may comprise at least one variable region domain of an antibody described herein. The variable region domain may be of any size or amino acid composition and will generally comprise at least one CDR sequence responsible for binding to human sclerostin, for example CDR-H1, CDR-H2, CDR-H3 and/or the light chain CDRs specifically described herein and which is adjacent to or in frame with one or more framework sequences. In general terms, the variable (V) region domain may be any suitable arrangement of immunoglobulin heavy (V_{H}) and/or light (V_{L}) chain variable domains. Thus, for example, the V region domain may be monomeric and be a V_{H} or V_{L} domain, which is capable of independently binding human sclerostin with an affinity at least equal to 1 x 10⁻⁷M or less as described below. Alternatively, the V region domain may be dimeric and contain V_{H}-V_{H}, V_{H}-V_{L}, or V_{L}-V_{L}, dimers. The V region dimer comprises at least one V_{H} and at least one V_{L} chain that may be non-covalently associated (hereinafter referred to as Fᵥ). If desired, the chains may be covalently coupled either directly, for example, via a disulfide bond between the two variable domains, or through a linker, for example, a peptide linker, to form a single chain Fv (scFv).

The variable region domain may be any naturally occurring variable domain or an engineered version thereof. By engineered version is meant a variable region domain that has been created using recombinant DNA engineering techniques. Such engineered versions include those created, for example, from a specific antibody variable region by insertions, deletions, or changes in or to the amino acid sequences of the specific antibody. Particular examples include engineered variable region domains containing at least one CDR and optionally one or more framework amino acids from a first antibody and the remainder of the variable region domain from a second antibody.

The variable region domain may be covalently attached at a C-terminal amino acid to at least one other antibody domain or a fragment thereof. Thus, for example, a V_{H} domain that is present in the variable region domain may be linked to an immunoglobulin CH1 domain, or a fragment thereof. Similarly a V_{L} domain may be linked to a C_{K} domain or a fragment thereof. In this way, for example, the antibody may be an Fab fragment wherein the antigen binding domain contains associated V_{H} and V_{L} domains covalently linked at their C-termini to a CH1 and C_{K} domain, respectively. The CH1 domain may be extended with further amino acids, for example, to provide a hinge region or a portion of a hinge region domain as found in a Fab' fragment, or to provide further domains, such as antibody CH2 and CH3 domains.

As described herein, binding agents comprise at least one of these CDRs. For example, one or more CDR may be incorporated into known antibody framework regions (IgG1, IgG2, etc.), or conjugated to a suitable vehicle to enhance the half-life thereof. Suitable vehicles include, but are not limited to Fc, polyethylene glycol (PEG), albumin, transferrin, and the like. These and other suitable vehicles are known in the art. Such conjugated CDR peptides may be in monomeric, dimeric, tetrameric, or other form. In one embodiment, one or more water-soluble polymer is bonded at one or more specific position, for example at the amino terminus, of a binding agent.

In certain preferred embodiments, a binding agent comprises one or more water soluble polymer attachments, including, but not limited to, polyethylene glycol, polyoxyethylene glycol, or polypropylene glycol. *See, e.g.,* U.S. Patent Nos. 4,640,835, 4,496,689, 4,301,144, 4,670,417, 4,791,192 and 4,179,337. In certain embodiments, a derivative binding agent comprises one or more of monomethoxy-polyethylene glycol, dextran, cellulose, or other carbohydrate based polymers, poly-(N-vinyl pyrrolidone)-polyethylene glycol, propylene glycol homopolymers, a polypropylene oxide/ethylene oxide co-polymer, polyoxyethylated polyols (*e.g*., glycerol) and polyvinyl alcohol, as well as mixtures of such polymers. In certain embodiments, one or more water-soluble polymers are randomly attached to one or more side chains. In certain embodiments, PEG can act to improve the therapeutic capacity for a binding agent, such as an antibody. Certain such methods are discussed, for example, in U.S. Patent No. 6,133,426, which is hereby incorporated by reference for any purpose.

It will be appreciated that a binding agent of the present invention may have at least one amino acid substitution, providing that the binding agent retains binding specificity. Therefore, modifications to the binding agent structures are encompassed within the scope of the invention. These may include amino acid substitutions, which may be conservative or non-conservative, that do not destroy the sclerostin binding capability of a binding agent. Conservative amino acid substitutions may encompass non-naturally occurring amino acid residues, which are typically incorporated by chemical peptide synthesis rather than by synthesis in biological systems. These include peptidomimetics and other reversed or inverted forms of amino acid moieties. A conservative amino acid substitution may also involve a substitution of a native amino acid residue with a normative residue such that there is little or no effect on the polarity or charge of the amino acid residue at that position.

Non-conservative substitutions may involve the exchange of a member of one class of amino acids or amino acid mimetics for a member from another class with different physical properties (e.g., size, polarity, hydrophobicity, and/or charge). Such substituted residues may be introduced into regions of the human antibody that are homologous with non-human antibodies, or into the non-homologous regions of the molecule.

Moreover, one skilled in the art may generate test variants containing a single amino acid substitution at each desired amino acid residue. The variants can then be screened using activity assays known to those skilled in the art. Such variants could be used to gather information about other suitable variants. For example, if one discovered that a change to a particular amino acid residue resulted in destroyed, undesirably reduced, or unsuitable activity, variants with such a change may be avoided. In other words, based on information gathered from such routine experiments, one skilled in the art can readily determine the amino acids where further substitutions should be avoided either alone or in combination with other mutations.

A skilled artisan will be able to determine suitable variants of the polypeptide as set forth herein using well-known techniques. In certain embodiments, one skilled in the art may identify suitable areas of the molecule that may be changed without destroying activity by targeting regions not believed to be important for activity. In certain embodiments, one can identify residues and portions of the molecules that are conserved among similar polypeptides. In certain embodiments, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in a protein that correspond to amino acid residues which are important for activity or structure in similar proteins. One skilled in the art may opt for chemically similar amino acid substitutions for such predicted important amino acid residues.

One skilled in the art can also analyze the three-dimensional structure and amino acid sequence in relation to that structure in similar polypeptides. In view of such information, one skilled in the art may predict the alignment of amino acid residues of an antibody with respect to its three dimensional structure. In certain embodiments, one skilled in the art may choose not to make radical changes to amino acid residues predicted to be on the surface of the protein, since such residues may be involved in important interactions with other molecules.

A number of scientific publications have been devoted to the prediction of secondary structure. *See* Moult J., Curr. Op. in Biotech., 7(4):422-427 (1996), Chou et al., Biochemistry, 13(2):222-245 (1974); Chou et al., Biochemistry, 113(2):211-222 (1974); Chou et al., Adv. Enzymol. Relat. Areas Mol. Biol., 47:45-148 (1978); Chou et al., Ann. Rev. Biochem., 47:251-276 and Chou et al., Biophys. J., 26:367-384 (1979). Moreover, computer programs are currently available to assist with predicting secondary structure. One method of predicting secondary structure is based upon homology modeling. For example, two polypeptides or proteins which have a sequence identity of greater than 30%, or similarity greater than 40% often have similar structural topologies. The recent growth of the protein structural database (PDB) has provided enhanced predictability of secondary structure, including the potential number of folds within a polypeptide's or protein's structure. *See* Holm et al., Nucl. Acid. Res., 27(1):244-247 (1999). It has been suggested (Brenner et al., Curr. Op. Struct. Biol., 7(3):369-376 (1997)) that there are a limited number of folds in a given polypeptide or protein and that once a critical number of structures have been resolved, structural prediction will become dramatically more accurate.

Additional methods of predicting secondary structure include "threading" (Jones, D., Curr. Opin. Struct. Biol., 7(3):377-87 (1997); Sippl et al., Structure, 4(1):15-19 (1996)), "profile analysis" (Bowie et al., Science, 253:164-170 (1991); Gribskov et al., Meth. Enzym., 183:146-159 (1990); Gribskov et al., Proc. Nat. Acad. Sci., 84(13):4355-4358 (1987)), and "evolutionary linkage" (see Holm, *supra* (1999), and Brenner, *supra* (1997)).

In certain embodiments, variants of binding agents include glycosylation variants wherein the number and/or type of glycosylation site has been altered compared to the amino acid sequences of a parent polypeptide. In certain embodiments, variants comprise a greater or a lesser number of N-linked glycosylation sites than the native protein. An N-linked glycosylation site is characterized by the sequence: Asn-X-Ser or Asn-X-Thr, wherein the amino acid residue designated as X may be any amino acid residue except proline. The substitution of amino acid residues to create this sequence provides a potential new site for the addition of an N-linked carbohydrate chain. Alternatively, substitutions which eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N-linked sites are created. Additional preferred antibody variants include cysteine variants wherein one or more cysteine residues are deleted from or substituted for another amino acid (e.g., serine) as compared to the parent amino acid sequence. Cysteine variants may be useful when antibodies must be refolded into a biologically active conformation such as after the isolation of insoluble inclusion bodies. Cysteine variants generally have fewer cysteine residues than the native protein, and typically have an even number to minimize interactions resulting from unpaired cysteines.

Desired amino acid substitutions (whether conservative or non-conservative) can be determined by those skilled in the art at the time such substitutions are desired. In certain embodiments, amino acid substitutions can be used to identify important residues of antibodies to sclerostin, or to increase or decrease the affinity of the antibodies to sclerostin described herein.

According to certain embodiments, preferred amino acid substitutions are those which: (1) reduce susceptibility to proteolysis, (2) reduce susceptibility to oxidation, (3) alter binding affinity for forming protein complexes, (4) alter binding affinities, and/or (5) confer or modify other physiochemical or functional properties on such polypeptides. According to certain embodiments, single or multiple amino acid substitutions (in certain embodiments, conservative amino acid substitutions) may be made in the naturally-occurring sequence (in certain embodiments, in the portion of the polypeptide outside the domain(s) forming intermolecular contacts). In certain embodiments, a conservative amino acid substitution typically may not substantially change the structural characteristics of the parent sequence (*e.g.*, a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et al., Nature, 354:105 (1991), which are each incorporated herein by reference.

In certain embodiments, binding agents of the invention may be chemically bonded with polymers, lipids, or other moieties.

The binding agents may comprise at least one of the CDRs described herein incorporated into a biocompatible framework structure. In one example, the biocompatible framework structure comprises a polypeptide or portion thereof that is sufficient to form a conformationally stable structural support, or framework, or scaffold, which is able to display one or more sequences of amino acids that bind to an antigen (*e.g*., CDRs, a variable region, etc.) in a localized surface region. Such structures can be a naturally occurring polypeptide or polypeptide "fold" (a structural motif), or can have one or more modifications, such as additions, deletions or substitutions of amino acids, relative to a naturally occurring polypeptide or fold. These scaffolds can be derived from a polypeptide of any species (or of more than one species), such as a human, other mammal, other vertebrate, invertebrate, plant, bacteria, or virus.

Typically the biocompatible framework structures are based on protein scaffolds or skeletons other than immunoglobulin domains. For example, those based on fibronectin, ankyrin, lipocalin, neocarzinostain, cytochrome b, CP1 zinc finger, PST1, coiled coil, LACI-D1, Z domain and tendramisat domains may be used (see*, e.g.,* Nygren and Uhlen, Current Opinion in Structural Biology, 7:463-469 (1997)).

In preferred embodiments, it will be appreciated that the binding agents of the invention include the humanized antibodies described herein. Humanized antibodies such as those described herein can be produced using techniques known to those skilled in the art (Zhang, W., et al., Molecular Immunology, 42(12):1445-1451 (2005); Hwang W. et al., Methods, 36(1):35-42 (2005); Dall'Acqua WF, et al., Methods, 36(1):43-60 (2005); and Clark, M., Immunology Today, 21(8):397-402 (2000)).

Additionally, one skilled in the art will recognize that suitable binding agents include portions of these antibodies, such as one or more of CDR-H1, CDR-H2, CDR-H3, CDR-L1, CDR-L2 and CDR-L3 as specifically disclosed herein. At least one of the regions of CDR-H1, CDR-H2, CDR-H3, CDR-L1, CDR-L2 and CDR-L3 may have at least one amino acid substitution, provided that the binding agent retains the binding specificity of the non-substituted CDR. The non-CDR portion of the binding agent may be a non-protein molecule, wherein the binding agent cross-blocks the binding of an antibody disclosed herein to sclerostin and/or neutralizes sclerostin. The non-CDR portion of the binding agent may be a non-protein molecule in which the binding agent exhibits a similar binding pattern to human sclerostin peptides in a "human sclerostin peptide epitope competition binding assay" as that exhibited by at least one of antibodies AA-WW, and/or neutralizes sclerostin. The non-CDR portion of the binding agent may be composed of amino acids, wherein the binding agent is a recombinant binding protein or a synthetic peptide, and the recombinant binding protein cross-blocks the binding of an antibody disclosed herein to sclerostin and/or neutralizes sclerostin. The non-CDR portion of the binding agent may be composed of amino acids, wherein the binding agent is a recombinant binding protein, and the recombinant binding protein exhibits a similar binding pattern to human sclerostin peptides in the human sclerostin peptide epitope competition binding assay (described hereinbelow) as that exhibited by at least one of the antibodies AA-WW, and/or neutralizes sclerostin.

Where an antibody comprises one or more of CDR-H1, CDR-H2, CDR-H3, CDR-L1, CDR-L2 and CDR-L3 as described above, it may be obtained by expression from a host cell containing DNA coding for these sequences. A DNA coding for each CDR sequence may be determined on the basis of the amino acid sequence of the CDR and synthesized together with any desired antibody variable region framework and constant region DNA sequences using oligonucleotide synthesis techniques, site-directed mutagenesis and polymerase chain reaction (PCR) techniques as appropriate. DNA coding for variable region frameworks and constant regions is widely available to those skilled in the art from genetic sequences databases such as GenBank®. Each of the above-mentioned CDRs will be typically located in a variable region framework at positions 31-35 (CDR-H1), 50-65 (CDR-H2) and 95-102 (CDR-H3) of the heavy chain and positions 24-34 (CDR-L1), 50-56 (CDR-L2) and 89-97 (CDR-L3) of the light chain according to the Kabat numbering system (Kabat *et al.,* 1987 in *Sequences of Proteins of Immunological Interest,* U.S. Department of Health and Human Services, NIH, USA).

The present invention therefore relates to an isolated antibody, exemplified by antibody AA but also applicable to all antibodies disclosed herein, or an antigen binding fragment thereof, which specifically binds to sclerostin and wherein the variable domain of the heavy chain comprises at least one CDR having the sequences given in SEQ ID NO:101, 107, 113, 119, 125, 131, 137, 143, 149, 155, 161, 167, 173, 179, 185, 191, 197, 203, 209, 215, 221, 227, and 233 for CDR-H1; SEQ ID NO:102, 108, 114, 120, 132, 144, 150, 156, 162, 168, 174, 180, 186, 192, 198, 204, 210, 216, 222, 228, and 234 for CDR-H2; and SEQ ID NO:103,109,115,121,127,133,139,145,151,157,163,169,175,181,187,193,199, 205, 211, 217, 223, 229, and 235 for CDR-H3. The antibody or antigen binding fragment thereof may comprise a heavy chain variable domain in which the CDRs consist of at least one of the peptides of SEQ ID NO:101, 107, 113, 119, 125, 131, 137, 143, 149, 155, 161, 167, 173, 179, 185, 191, 197, 203, 209, 215, 221, 227, and 233 for CDR-H1; SEQ ID NO:102, 108, 114, 120, 132, 144, 150, 156, 162, 168, 174, 180, 186, 192, 198, 204, 210, 216, 222, 228, and 234 for CDR-H2; and SEQ ID NO: 103, 109, 115, 121, 127, 133, 139, 145, 151, 157, 163, 169, 175, 181, 187, 193, 199, 205, 211, 217, 223, 229, and 235 for CDR-H3.

When a light chain is present in antibodies of the invention the light chain may be any suitable complementary chain and may in particular be selected from a light chain wherein the variable domain comprises at least one or two or all of the CDRs consisting of (or comprising) at least one of the peptides of SEQ ID NO:98, 104, 110, 116, 122, 128, 134, 140, 146, 152, 158, 164, 170, 176, 182, 188, 194, 200, 206, 212, 218, 224, and 230 for CDR-L1; SEQ ID NO:99, 105, 111, 117, 123, 129, 135, 141, 147, 153, 159, 165, 171, 177, 183, 189, 195, 201, 207, 213, 219, 225, 231 for CDR-L2; and SEQ ID NO:100, 106, 112, 118, 124, 130, 136, 142, 148, 154, 160, 166, 172, 178, 184, 190, 196, 202, 208, 214, 220, 226, and 232 for CDR-L3.

Once synthesized, the DNA encoding an antibody of the invention or fragment thereof may be propagated and expressed according to any of a variety of well-known procedures for nucleic acid excision, ligation, transformation, and transfection using any number of known expression vectors. Thus, in certain embodiments, expression of an antibody fragment may be preferred in a prokaryotic host, such as *Escherichia coli* (*see, e.g.,* Pluckthun et al., Methods Enzymol., 178:497-515 (1989). In certain other embodiments, expression of the antibody or a fragment thereof may be preferred in a eukaryotic host cell, including yeast (*e.g., Saccharomyces cerevisiae, Schizosaccharomyces pombe,* and *Pichia pastoris*)*,* animal cells (including mammalian cells) or plant cells. Examples of suitable animal cells include, but are not limited to, myeloma (such as a mouse NSO line), COS, CHO, or hybridoma cells. Examples of plant cells include tobacco, corn, soybean, and rice cells.

One or more replicable expression vectors containing DNA encoding an antibody variable and/or constant region may be prepared and used to transform an appropriate cell line, for example, a non-producing myeloma cell line, such as a mouse NSO line or a bacteria, such as *E*. *coli,* in which production of the antibody will occur. In order to obtain efficient transcription and translation, the DNA sequence in each vector should include appropriate regulatory sequences, particularly a promoter and leader sequence operatively linked to the variable domain sequence. Particular methods for producing antibodies in this way are generally well-known and routinely used. For example, basic molecular biology procedures are described by Maniatis et al., Molecular Cloning, A Laboratory Manual, 2d ed., Cold Spring Harbor Laboratory, New York, 1989. *See also* Maniatis *et al,* 3d ed., Cold Spring Harbor Laboratory, New York, (2001). DNA sequencing can be performed as described in Sanger et al., PNAS, 74:5463 (1977), and the Amersham International plc sequencing handbook, and site directed mutagenesis can be carried out according to methods known in the art (Kramer et al., Nucleic Acids Res., 12:9441 (1984); Kunkel, Proc. Natl. Acad. Sci. USA, 82:488-92 (1985); Kunkel et al., Methods in Enzymol., 154:367-82 (1987); the Anglian Biotechnology Ltd handbook). Additionally, numerous publications describe techniques suitable for the preparation of antibodies by manipulation of DNA, creation of expression vectors, and transformation and culture of appropriate cells (Mountain, A. and Adair, J. R., in Biotechnology and Genetic Engineering Reviews, (Tombs, M. P. (ed.), 10, Chapter 1, Intercept, Andover, UK (1992)); Current Protocols in Molecular Biology, F.M. Ausubel (ed.), Wiley Interscience, New York (1999)).

Antibodies with improved affinities containing one or more of the above-mentioned CDRs can be obtained by a number of affinity maturation protocols including maintaining the CDRs (Yang et al., J. Mol. Biol., 254:392-403 (1995), chain shuffling (Marks et al., BiolTechnology, 10:779-783 (1992), use of mutation strains of *E*. *coli.* (Low et al., J. Mol. Biol., 250:350-368 (1996), DNA shuffling (Patten et al., Curr. Opin. Biotechnol., 8:724-733 (1997)), phage display (Thompson et al., J. Mol. Biol., 256:7-88 (1996)) and sexual PCR (Crameri, et al., Nature, 391:288-291 (1998)). All of these methods of affinity maturation are discussed by Vaughan et al., (Nature Biotechnology, 16:535-539 (1998)).

Other antibodies according to the invention may be obtained by conventional immunization and cell fusion procedures as described herein and known in the art. Monoclonal antibodies of the invention may be generated using a variety of known techniques. In general, monoclonal antibodies that bind to specific antigens may be obtained by methods known to those skilled in the art (*see,* for example, Kohler et al., Nature, 256:495 (1975); Coligan et al. (eds.), Current Protocols in Immunology, 1:2.5.12.6.7 (John Wiley & Sons 1991); U.S. Patent Nos. RE 32,011, 4,902,614, 4,543,439, and 4,411,993; Monoclonal Antibodies, Hybridomas: A New Dimension in Biological Analyses, Plenum Press, Kennett, McKearn, and Bechtol (eds.) (1980); and Antibodies: A Laboratory Manual, Harlow and Lane (eds.), Cold Spring Harbor Laboratory Press (1988); Picksley et al., "Production of monoclonal antibodies against proteins expressed in E. coli," in DNA Cloning 2: Expression Systems, 2d Ed., Glover et al. (eds.), page 93 (Oxford University Press 1995)). Antibody fragments may be derived therefrom using any suitable standard technique such as proteolytic digestion, or optionally, by proteolytic digestion (for example, using papain or pepsin) followed by mild reduction of disulfide bonds and alkylation. Alternatively, such fragments may also be generated by recombinant genetic engineering techniques as described herein.

Monoclonal antibodies can be obtained by injecting an animal, for example, a rat, hamster, a rabbit, or preferably a mouse, including for example a transgenic or a knockout, as known in the art, with an immunogen comprising human sclerostin of SEQ ID N0:1, or a fragment thereof, according to methods known in the art and described herein. Specific antibody production may be monitored after the initial injection and/or after a booster injection by obtaining a serum sample and detecting the presence of an antibody that binds to human sclerostin (or fragment thereof) using any one of several immunodetection methods known in the art and described herein. From animals producing the desired antibodies, lymphoid cells, most commonly cells from the spleen or lymph node, are removed to obtain B-lymphocytes. The B-lymphocytes are then fused with a drug-sensitized myeloma cell fusion partner, preferably one that is syngeneic with the immunized animal and that optionally has other desirable properties (*e.g.*, inability to express endogenous Ig gene products, *e.g*., P3X63 - Ag 8.653 (ATCC No. CRL 1580); NSO, SP20) to produce hybridomas, which are immortal eukaryotic cell lines. The lymphoid (*e.g.*, spleen) cells and the myeloma cells may be combined for a few minutes with a membrane fusion-promoting agent, such as polyethylene glycol or a nonionic detergent, and then plated at low density on a selective medium that supports the growth of hybridoma cells but not unfused myeloma cells. A preferred selection media is HAT (hypoxanthine, aminopterin, thymidine). After a sufficient time, usually about one to two weeks, colonies of cells are observed. Single colonies are isolated, and antibodies produced by the cells may be tested for binding activity to human sclerostin, using any one of a variety of immunoassays known in the art and described herein. The hybridomas are cloned (*e.g.*, by limited dilution cloning or by soft agar plaque isolation) and positive clones that produce an antibody specific to sclerostin are selected and cultured. The monoclonal antibodies from the hybridoma cultures may be isolated from the supernatants of hybridoma cultures. An alternative method for production of a murine monoclonal antibody is to inject the hybridoma cells into the peritoneal cavity of a syngeneic mouse, for example, a mouse that has been treated (*e.g*., pristane-primed) to promote formation of ascites fluid containing the monoclonal antibody. Monoclonal antibodies can be isolated and purified by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, Vol. 10, pages 79-104, Humana Press, Inc. (1992)). Monoclonal antibodies may be purified by affinity chromatography using an appropriate ligand selected based on particular properties of the antibody (e.g., heavy or light chain isotype, binding specificity, etc.). Examples of a suitable ligand, immobilized on a solid support, include Protein A, Protein G, an anticonstant region (light chain or heavy chain) antibody, an anti-idiotype antibody, and a TGF-beta binding protein, or fragment or variant thereof.

An antibody of the present invention may also be a human monoclonal antibody. Human monoclonal antibodies may be generated by any number of techniques with which those having ordinary skill in the art will be familiar. Such methods include, but are not limited to, Epstein Barr Virus (EBV) transformation of human peripheral blood cells (*e.g.*, containing B lymphocytes), *in vitro* immunization of human B cells, fusion of spleen cells from immunized transgenic mice carrying inserted human immunoglobulin genes, isolation from human immunoglobulin V region phage libraries, or other procedures as known in the art and based on the disclosure herein. For example, human monoclonal antibodies may be obtained from transgenic mice that have been engineered to produce specific human antibodies in response to antigenic challenge. Methods for obtaining human antibodies from transgenic mice are described, for example, by Green et al., Nature Genet., 7:13 (1994); Lonberg et al., Nature, 368:856 (1994); Taylor et al., Int. Immun., 6:579 (1994); U.S. Patent No. 5,877,397; Bruggemann et al., Curr. Opin. Biotechnol., 8:455-58 (1997); Jakobovits et al., Ann. N. Y. Acad. Sci., 764:525-35 (1995). In this technique, elements of the human heavy and light chain locus are introduced into strains of mice derived from embryonic stem cell lines that contain targeted disruptions of the endogenous heavy chain and light chain loci (*see also* Bruggemann et al., Curr. Opin. Biotechnol., 8:455-58 (1997)). For example, human immunoglobulin transgenes may be mini-gene constructs, or transloci on yeast artificial chromosomes, which undergo B cell-specific DNA rearrangement and hypermutation in the mouse lymphoid tissue. Human monoclonal antibodies may be obtained by immunizing the transgenic mice, which may then produce human antibodies specific for sclerostin. Lymphoid cells of the immunized transgenic mice can be used to produce human antibody-secreting hybridomas according to the methods described herein. Polyclonal sera containing human antibodies may also be obtained from the blood of the immunized animals.

Another method for generating human antibodies of the invention includes immortalizing human peripheral blood cells by EBV transformation. *See, e.g.,* U.S. Patent No. 4,464,456. Such an immortalized B cell line (or lymphoblastoid cell line) producing a monoclonal antibody that specifically binds to sclerostin can be identified by immunodetection methods as provided herein, for example, an ELISA, and then isolated by standard cloning techniques. The stability of the lymphoblastoid cell line producing an anti-sclerostin antibody may be improved by fusing the transformed cell line with a murine myeloma to produce a mouse-human hybrid cell line according to methods known in the art (*see, e.g.,* Glasky et al., Hybridoma 8:377-89 (1989)). Still another method to generate human monoclonal antibodies is *in vitro* immunization, which includes priming human splenic B cells with human sclerostin, followed by fusion of primed B cells with a heterohybrid fusion partner. *See, e.g.,* Boerner et al., J. Immunol., 147:86-95 (1991).

In certain embodiments, a B cell that is producing an anti-human sclerostin antibody is selected and the light chain and heavy chain variable regions are cloned from the B cell according to molecular biology techniques known in the art (WO 92/02551; U.S. Patent No. 5,627,052; Babcook et al., Proc. Natl. Acad. Sci. USA, 93:7843-48 (1996)) and described herein. B cells from an immunized animal may be isolated from the spleen, lymph node, or peripheral blood sample by selecting a cell that is producing an antibody that specifically binds to sclerostin. B cells may also be isolated from humans, for example, from a peripheral blood sample. Methods for detecting single B cells that are producing an antibody with the desired specificity are well known in the art, for example, by plaque formation, fluorescence-activated cell sorting, *in vitro* stimulation followed by detection of specific antibody, and the like. Methods for selecting specific antibody-producing B cells include, for example, preparing a single cell suspension of B cells in soft agar that contains human sclerostin. Binding of the specific antibody produced by the B cell to the antigen results in the formation of a complex, which may be visible as an immunoprecipitate. After the B cells producing the desired antibody are selected, the specific antibody genes may be cloned by isolating and amplifying DNA or mRNA according to methods known in the art and described herein.

An additional method for obtaining antibodies of the invention is by phage display. *See, e.g.,* Winter et al., Annu. Rev. Immunol., 12:433-55 (1994); Burton et al., Adv. Immunol., 57:191-280 (1994). Human or murine immunoglobulin variable region gene combinatorial libraries may be created in phage vectors that can be screened to select Ig fragments (Fab, Fv, sFv, or multimers thereof) that bind specifically to TGF-beta binding protein or variant or fragment thereof. *See, e.g.,* U.S. Patent No. 5,223,409; Huse et al., Science, 246:1275-81 (1989); Sastry et al., Proc. Natl. Acad. Sci. USA, 86:5728-32 (1989); Alting-Mees et al., Strategies in Molecular Biology, 3:1-9 (1990); Kang et al., Proc. Natl. Acad. Sci. USA, 88:4363-66 (1991); Hoogenboom et al., J. Molec. Biol., 227:381-388 (1992); Schlebusch et al., Hybridoma, 16:47-52 (1997) and references cited therein. For example, a library containing a plurality of polynucleotide sequences encoding Ig variable region fragments may be inserted into the genome of a filamentous bacteriophage, such as M13 or a variant thereof, in frame with the sequence encoding a phage coat protein. A fusion protein may be a fusion of the coat protein with the light chain variable region domain and/or with the heavy chain variable region domain. According to certain embodiments, immunoglobulin Fab fragments may also be displayed on a phage particle (*see, e.g.,* U.S. Patent No. 5,698,426).

Heavy and light chain immunoglobulin cDNA expression libraries may also be prepared in lambda phage, for example, using λlmmunoZap^{™}(H) and λImmunoZap^{™}(L) vectors (Stratagene, La Jolla, California). Briefly, mRNA is isolated from a B cell population, and used to create heavy and light chain immunoglobulin cDNA expression libraries in the λImmunoZap(H) and λImmunoZap(L) vectors. These vectors may be screened individually or co-expressed to form Fab fragments or antibodies (*see* Huse *et al., supra; see also* Sastry *et al., supra*)*.* Positive plaques may subsequently be converted to a non-lytic plasmid that allows high level expression of monoclonal antibody fragments from *E. coli.*

In one embodiment, in a hybridoma the variable regions of a gene expressing a monoclonal antibody of interest are amplified using nucleotide primers. These primers may be synthesized by one of ordinary skill in the art, or may be purchased from commercially available sources. *See, e.g.,* Stratagene (La Jolla, California), which sells primers for mouse and human variable regions including, among others, primers for V_{Ha}, V_{Hb}, V_{Hc}, V_{Hd}, C_{H1}, V_{L} and C_{L} regions. These primers may be used to amplify heavy or light chain variable regions, which may then be inserted into vectors such as ImmunoZAP^{™}H or ImmunoZAP^{™}L (Stratagene), respectively. These vectors may then be introduced into *E. coli,* yeast, or mammalian-based systems for expression. Large amounts of a single-chain protein containing a fusion of the V_{H} and V_{L} domains may be produced using these methods (*see* Bird et al., Science, 242:423-426, (1988)).

Once cells producing antibodies according to the invention have been obtained using any of the above-described immunization and other techniques, the specific antibody genes may be cloned by isolating and amplifying DNA or mRNA therefrom according to standard procedures as described herein. The antibodies produced therefrom may be sequenced and the CDRs identified and the DNA coding for the CDRs may be manipulated as described previously to generate other antibodies according to the invention.

Preferably the binding agents specifically bind to sclerostin. As with all binding agents and binding assays, one of skill in this art recognizes that the various moieties to which a binding agent should not detectably bind in order to be therapeutically effective and suitable would be exhaustive and impractical to list. Therefore, for a binding agent disclosed herein, the term "specifically binds" refers to the ability of a binding agent to bind to sclerostin, preferably human sclerostin, with greater affinity than it binds to an unrelated control protein. Preferably the control protein is hen egg white lysozyme. Preferably the binding agents bind to sclerostin with an affinity that is at least, 50, 100, 250, 500, 1000, or 10,000 times greater than the affinity for a control protein. A binding agent may have a binding affinity for human sclerostin of less than or equal to 1 x 10⁻⁷ M, less than or equal to 1 x 10⁻⁸ M, less than or equal to 1 x 10⁻⁹ M, less than or equal to 1 x 10⁻¹⁰ M, less than or equal to 1 x 10⁻¹¹ M, or less than or equal to 1 x 10⁻¹² M.

Affinity may be determined by an affinity ELISA assay. In certain embodiments, affinity may be determined by a BIAcore assay. In certain embodiments, affinity may be determined by a kinetic method. In certain embodiments, affinity may be determined by an equilibrium/solution method. Such methods are described in further detail herein or known in the art.

Sclerostin binding agents of the present invention preferably modulate sclerostin function in the cell-based assay described herein and/or the *in vivo* assay described herein and/or bind to one or more of the epitopes described herein and/or cross-block the binding of one of the antibodies described in this application and/or are cross-blocked from binding sclerostin by one of the antibodies described in this application. Accordingly such binding agents can be identified using the assays described herein.

In certain embodiments, binding agents are generated by first identifying antibodies that bind to one more of the epitopes provided herein and/or neutralize in the cell-based and/or *in vivo* assays described herein and/or cross-block the antibodies described in this application and/or are cross-blocked from binding sclerostin by one of the antibodies described in this application. The CDR regions from these antibodies are then used to insert into appropriate biocompatible frameworks to generate sclerostin binding agents. The non-CDR portion of the binding agent may be composed of amino acids, or may be a non-protein molecule. The assays described herein allow the characterization of binding agents. Preferably the binding agents of the present invention are antibodies as defined herein.

It will be understood by one skilled in the art that some proteins, such as antibodies, may undergo a variety of posttranslational modifications. The type and extent of these modifications often depends on the host cell line used to express the protein as well as the culture conditions. Such modifications may include variations in glycosylation, methionine oxidation, diketopiperizine formation, aspartate isomerization and asparagine deamidation. A frequent modification is the loss of a carboxy-terminal basic residue (such as lysine or arginine) due to the action of carboxypeptidases (as described in Harris, R.J., Journal of Chromatography, 705:129-134 (1995)).

The Kappa Constant region for all VK regions of antibodies AA-CC, EE-JJ, and LL-WW disclosed herein is as follows:

TDAAPTVSIFPPSSEQLTSGGASVVCFLNNFYPKDINVKWKIDGSERQNGVLNSWTD
QDSKDSTYSMSSTLTLTKDEYERHNSYTCEATHKTSTSPIVKSFNRNEC (SEQ ID
NO:2)

The Heavy Constant Region for all VH regions of antibodies AA-WW in this Example is as follows:

AKTTPPSVYPLAPGSAAQTNSMVTLGCLVKGYFPEPVTVTWNSGSLSSGVHTFPAVL
QSDLYTLSSSVTVPSSTWPSETVTCNVAHPASSTKVDKKIVPRDCGCKPCICTVPEVS
SVFIFPPKPKDVLTITLTPKVTCVVVDISKDDPEVQFSWFVDDVEVHTAQTQPREEQF
NSTFRSVSELPIMHQDWLNGKEFKCRVNSAAFPAPIEKTISKTKGRPKAPQVYTIPPPK
EQMAKDKVSLTCMITDFFPEDITVEWQWNGQPAENYKNTQPIMDTDGSYFVYSKLN
VQKSNWEAGNTFTCSVLHEGLHNHHTEKSLSHSPGK (SEQ ID NO:3)

In the following antibody amino acid sequences, the boxed-shaded amino acids represent complement-determining regions (CDRs) and the underlined amino acids represent signal peptide.

### Antibody AA

VK 
METDTILLWVLLLWVPGSTGDIVLTQSPASLAVSLGQRATISCKASQSVDYAGDNYM
NWYQQKPGQPPKLLIYTASNLESGIPARFSGSGSGTDFTLNIHPVEEEDAATYYCQQS
NEDPPTFGGGTKLELKR (SEQ ID NO:4)
 
ATGGAGACAGACACAATCCTGCTATGGGTGCTGCTGCTCTGGGTTCCAGGCTCCA
CTGGTGACATTGTGCTGACCCAATCTCCAGCTTCTTTGGCTGTGTCTCTAGGGCAG
AGGGCCACCATCTCCTGCAAGGCCAGCCAAAGTGTTGATTATGCTGGTGATAATT
ATATGAACTGGTACCAACAGAAACCAGGACAGCCACCCAAACTCCTCATCTATA
CTGCATCCAATCTAGAGTCTGGGATCCCAGCCAGGTTTAGTGGCAGTGGGTCTGG
GACAGACTTCACCCTCAACATTCATCCTGTGGAGGAGGAGGATGCTGCAACCTAT
TACTGTCAGCAAAGTAATGAGGATCCTCCGACGTTCGGTGGAGGCACCAAGTTG
GAGCTCAAACGT (SEQ ID NO:5)
VH

MGWNWIFILILSVTTGVHSEVQLQQSGPELVKPGASVKISCKASGYPFTGYFMHWVK
QSPENSLEWIGEINPSTGGTTYNQRFKGKATLTVDKSSSTAYMQLKSLTSEDSAVYY
CTRWGYNPYALDYWGQGTSVTVSS (SEQ ID NO:6)

ATGGGATGGAACTGGATCTTTATTTTAATCCTGTCAGTAACTACAGGTGTCCACT
CTGAGGTCCAACTGCAGCAGTCTGGACCTGAGCTGGTGAAGCCTGGGGCTTCAGT
GAAGATATCCTGCAAGGCTTCTGGTTACCCATTCACTGGCTACTTCATGCACTGG
GTGAAACAAAGTCCTGAAAATAGTCTTGAGTGGATTGGAGAGATTAATCCTAGC
ACTGGGGGTACTACCTACAACCAGAGGTTCAAGGGCAAGGCCACATTAACTGTA
GATAAATCCTCCAGCACAGCCTACATGCAGCTCAAGAGCCTGACATCTGAAGAC
TCTGCAGTCTATTACTGTACAAGATGGGGATATAACCCCTATGCTTTGGACTACT
GGGGTCAAGGAACCTCAGTCACCGTCTCGAGC (SEQ ID NO:7)

### Antibody

VK 
MRPSIQFLGLLLFWLHGAQCDIQMTQSPSSLSASLGGKVTITCKASQDINKYIAWYQH
KPGKGPRLLIHYTSTLQPGIPSRFSGSGSGRDYSFSISNLEPEDIATYYCLQYDNLYTFG
GGTKLELKR (SEQ ID NO:8)
 
ATGAGACCGTCTATTCAGTTCCTGGGGCTCTTGTTGTTCTGGCTTCATGGTGCTCA
GTGTGACATCCAGATGACACAGTCTCCATCCTCACTGTCTGCATCTCTGGGAGGC
AAAGTCACCATCACTTGCAAGGCAAGCCAAGACATTAACAAGTATATAGCTTGG
TACCAACACAAGCCTGGAAAAGGTCCTAGGCTGCTCATACATTACACATCTACAT
TACAGCCAGGCATCCCATCAAGGTTCAGTGGAAGTGGGTCTGGGAGAGATTATT
CCTTCAGCATCAGCAACCTGGAGCCTGAAGATATTGCAACTTATTATTGTCTACA
GTATGATAATCTATACACGTTCGGAGGGGGGACCAAGCTGGAACTGAAACGT
(SEQ ID NO:9)
VH

MEWSWVFLFLLSVTAGVHSQVQLQQSGAELMKPGASVKISCKATGYTFSSYWIEWV
KQRPGHGPEWIGEIFPRNGSTYYNEKFKGKATFTADTSSNTAYMQLSSLTSDDSAVY
YCAIINTLDYWGQGTTLTVSS (SEQ ID NO:10)

ATGGAATGGAGCTGGGTCTTTCTCTTCCTCCTGTCAGTAACTGCAGGTGTCCACTC
CCAGGTTCAGCTGCAGCAGTCTGGAGCTGAGCTGATGAAGCCTGGGGCCTCAGT
GAAGATATCCTGCAAGGCTACTGGCTACACATTCAGTAGTTATTGGATAGAGTGG
GTAAAGCAGAGGCCTGGACATGGCCCTGAGTGGATTGGAGAGATTTTTCCTAGA
AATGGTAGTACTTACTACAATGAGAAATTCAAGGGCAAGGCCACATTCACTGCA
GATACATCCTCCAACACAGCCTACATGCAACTCAGCAGCCTGACATCTGACGACT
CTGCCGTCTATTACTGTGCAATTATTAATACGCTTGACTACTGGGGCCAAGGCAC
CACTCTCACAGTCTCGAGC (SEQ ID NO:11)

### Antibody CC

VK 
MKLPVRLLVLMFWIPVSSSDVVMTQTPLSLPVSLGDQASISCRSSQSLVHSNGDTYL
HWYLQKPGQSPKLLIYEISNRFSGVPDRFSGSGSGTDFTLKISRVEAEDLGVYFCSQST
HVPFTFGGGTKLEIKR (SEQ ID NO:12)
 
ATGAAGTTGCCTGTTAGGCTGTTGGTGCTGATGTTCTGGATTCCTGTTTCCAGTAG
TGATGTTGTGATGACCCAAACTCCACTCTCCCTGCCTGTCAGTCTTGGAGATCAA
GCCTCCATCTCTTGCAGATCTAGTCAGAGCCTTGTACACAGTAATGGAGACACCT
ATTTACATTGGTACCTACAGAAGCCAGGCCAGTCTCCAAAACTCCTGATCTACGA
AATTTCCAACCGATTTTCTGGGGTCCCAGACAGGTTCAGTGGCAGTGGATCAGGG
ACAGATTTCACACTCAAGATCAGCAGAGTGGAGGCTGAGGATCTGGGAGTTTAT
TTCTGCTCTCAAAGTACACATGTTCCGTTCACGTTCGGAGGGGGGACCAAGCTGG
AAATAAAACGT (SEQ ID NO:13)
VH

MGWSWVIFFLMAVVIGINSEVQLQQSGAELVRSGASVRLSCTASGFNIKDYYMHWV
RQRPEQGLEWIGWNDPETGDTEYAPKFQGKATMTSDTSSNTAYLQLSSLTSEDTAV
YYCNEGSGLIPYWGPGTLVTVSS (SEQ ID NO:14)

ATGGGATGGAGCTGGGTCATCTTCTTCCTGATGGCAGTGGTTATAGGAATCAATT
CAGAGGTTCAGCTGCAGCAGTCTGGGGCAGAACTTGTGAGGTCAGGGGCCTCAG
TCAGGTTGTCCTGCACAGCTTCTGGCTTCAACATTAAAGACTACTATATGCACTG
GGTGAGGCAGCGGCCTGAACAGGGCCTGGAGTGGATTGGATGGAATGATCCTGA
GACTGGTGATACTGAATATGCCCCGAAGTTCCAGGGCAAGGCCACTATGACTTCA
GACACATCCTCCAACACAGCCTACCTGCAGCTCAGCAGCCTGACATCTGAGGAC
ACTGCCGTCTATTACTGTAATGAGGGCTCGGGCTTGATTCCTTACTGGGGCCCAG
GGACTCTGGTCACAGTCTCGAGC (SEQ ID NO:15)

### Antibody DD Humanized

VK 
MKLPVRLLVLMFWIPVSSSDIVMTQTPLSLSVTPGQPASISCRSSQSLVHSNGDTYLH
WYLQKPGQPPQLLIYEISNRFSGVPDRFSGSGSGTDFTLKISRVEAEDVGVYYCSQST
HVPFTFGQGTKLEIKR (SEQ ID NO:16)
 
ATGAAGTTGCCTGTTAGGCTGTTGGTGCTGATGTTCTGGATTCCTGTTTCCAGTAG
TGATATCGTGATGACCCAGACTCCACTCTCCCTGTCTGTCACTCCGGGTCAACCG
GCCTCCATCTCTTGCCGTTCTAGTCAGAGCCTTGTACACAGTAATGGTGACACCT
 
ATTTACATTGGTACTTACAGAAGCCAGGCCAGCCTCCACAACTCCTGATCTACGA
AATTTCCAACCGCTTTTCTGGGGTCCCAGACCGTTTCAGTGGCAGTGGTTCAGGG
ACAGATTTCACACTCAAGATCAGCCGCGTGGAGGCTGAGGATGTGGGTGTTTATT
ACTGCTCTCAAAGTACACATGTTCCGTTCACGTTCGGTCAGGGGACCAAGCTGGA
AATTAAACGT (SEQ ID NO:17)
VH

MKCSWVIFFLMAVVIGITSQVQLVQSGAEVKKPGSSVKVSCKASGFNIKDYYMHWV
RQAPGQGLEWMGWNDPETGDTEYAPKFQGRVTITADESTNTAYMELSSLRSEDTAV
YYCNEGSGLIPYWGQGTLVTVSS (SEQ ID NO:18)

ATGAAATGCAGCTGGGTCATCTTCTTCCTGATGGCAGTGGTTATAGGAATCACTT
CACAGGTTCAGCTGGTCCAGTCTGGGGCAGAGGTTAAAAAGCCAGGGTCCTCAG
TCAAAGTGTCCTGCAAGGCTTCTGGCTTCAACATTAAAGACTACTATATGCACTG
GGTGAGACAGGCGCCTGGTCAGGGCCTGGAGTGGATGGGATGGAATGATCCTGA
GACTGGTGATACTGAATATGCCCCGAAGTTCCAGGGCAGGGTCACTATTACTGCA
GACGAATCCACCAACACAGCCTACATGGAGCTCTCATCCCTGCGTTCTGAGGACA
CTGCCGTCTATTACTGTAATGAGGGCTCGGGCTTGATTCCTTACTGGGGCCAAGG
GACTCTGGTCACCGTCTCGAGC (SEQ ID NO:19)

### Antibody EE

VK 
MKLPVRLLVLMFWIPVSSSDVVLTQTPLSLPVSLGDQASISCRSSQSLVHSNGDVYLH
WYLQKPGQSPKLLIYEVSNRFSGVPDRFSGSGSGTDFTLKISRVEAEDLGVYFCSQST
HVPFTFGGGTKLEIKR (SEQ ID NO:20)
 
ATGAAGTTGCCTGTTAGGCTGTTGGTGCTGATGTTCTGGATTCCTGTTTCCAGCAG
TGATGTTGTATTGACCCAAACTCCACTCTCCCTGCCTGTCAGTCTTGGAGATCAA
GCCTCCATCTCTTGTAGATCTAGTCAGAGCCTTGTACACAGTAATGGAGACGTCT
ATTTACATTGGTACCTACAGAAGCCAGGCCAGTCTCCAAAGCTCCTGATCTACGA
AGTTTCCAACCGATTTTCTGGGGTCCCAGACAGGTTCAGTGGCAGTGGATCAGGG
ACAGATTTCACACTCAAGATCAGCAGAGTGGAGGCTGAGGATCTGGGAGTTTAT
TTCTGCTCTCAAAGTACACATGTTCCGTTCACGTTCGGAGGGGGGACCAAGCTGG
AAATAAAACGT (SEQ ID NO:21)
VH

MGWSWVIFFLMAVVIGINSEVQLQQSGTELVRSGASVKLACTASGFNIKNYYMHWV
RQRPEQGLEWIGWIDPENGDTEYAPKFQDKATMTADTSSNTAYLQLSSLTSEDTAVY
YCNEGSGLIPYWGPGTLVTVSS (SEQ ID NO:22)

ATGGGATGGAGCTGGGTCATCTTCTTCCTGATGGCAGTGGTTATAGGAATCAATT
CAGAGGTTCAGCTGCAGCAGTCTGGGACAGAGCTTGTGAGGTCAGGGGCCTCAG
TCAAGTTGGCCTGCACAGCTTCTGGCTTCAACATTAAAAACTACTATATGCACTG
GGTGAGGCAGAGGCCTGAACAGGGCCTGGAGTGGATTGGATGGATTGATCCTGA
GAATGGTGATACTGAGTATGCCCCGAAGTTCCAGGACAAGGCCACTATGACTGC
AGACACATCCTCCAACACAGCCTACCTGCAGCTCAGCAGCCTGACATCTGAGGA

CACTGCCGTCTATTACTGTAATGAGGGCTCGGGCTTGATTCCTTACTGGGGCCCA
GGGACTCTGGTCACTGTCTCGAGC (SEQ ID NO:23)

### Antibody FF

VK 
MKLPVRLLVLMFWIPASSSGVVMTQTPLSLPVSLGDQASISCRSSQSLVHSNGNTYL
YWYLQKPGQSPKLLLFKVSTRFSGVPDRFSGSGSGTDFTLKITRVEAEDLGIYFCSQS
SHIPPTFGGGTKLELKR (SEQ ID NO:24)
 
ATGAAGTTGCCTGTTAGGCTGTTGGTGCTGATGTTCTGGATTCCTGCTTCCAGCAG
TGGTGTTGTGATGACCCAAACTCCACTCTCCCTGCCTGTCAGTCTTGGAGATCAA
GCCTCCATCTCGTGCAGATCTAGTCAGAGCCTTGTTCACAGTAATGGAAACACCT
ATTTATATTGGTACCTACAGAAGCCAGGCCAGTCTCCAAAGCTCCTGCTCTTCAA
AGTTTCCACCCGATTTTCTGGGGTCCCAGACAGGTTCAGTGGCAGTGGATCAGGG
ACAGATTTCACACTCAAGATCACCAGAGTAGAGGCTGAGGATCTGGGAATTTATT
TCTGCTCTCAAAGTTCACATATTCCTCCGACGTTCGGTGGAGGCACCAAGTTGGA
GCTCAAACGT (SEQ ID NO:25)
VH

MDWLWTLLFLMAAAQSAQAQIQLVQSGPELKKPGETVKISCKASGYTVTDYGMNW
VKQSPGKDLKWMGWIDTYTEKPTYADDFKGRFAFSLETSASTAYLQISNLKNEDSA
AYFCLRSNFDFWGQGTTLTVSS (SEQ ID NO:26)

ATGGATTGGCTGTGGACCTTGCTATTCCTGATGGCAGCTGCCCAAAGTGCCCAAG
CACAGATCCAGTTGGTGCAGTCTGGACCTGAGCTGAAGAAGCCTGGAGAGACAG
TCAAGATCTCCTGCAAGGCTTCTGGGTATACCGTCACAGACTATGGAATGAACTG
GGTGAAGCAGAGTCCAGGAAAGGATTTAAAGTGGATGGGCTGGATAGACACCTA
CACTGAAAAGCCAACATATGCTGATGACTTCAAGGACGATTTGCCTTCTCTTTG
GAAACCTCTGCCAGCACTGCCTACTTGCAGATCAGCAACCTCAAAAATGAGGAC
TCGGCTGCATATTTCTGTCTAAGATCCAATTTTGACTTTTGGGGCCAAGGCACCA
CTCTCACAGTCTCGAGC (SEQ ID NO:27)

### Antibody GG

VK 
MESQIQVFVYMLLWLSGVEGDIVMTQSHKFMSTSVGGRVSITCKASQDVDTSVAWY
QQKPGQAPKLLVYWASTRHTGVPDRFTGSGSGTDFTLTISNVQSEDLADYFCQQYSN
YPTFGAGTKLELKR (SEQ ID NO:28)
 
ATGGAGTCACAGATTCAGGTCTTTGTATACATGTTGCTGTGGTTGTCTGGTGTTGA
AGGAGACATTGTGATGACCCAGTCTCACAAATTCATGTCCACATCAGTAGGAGG
CAGGGTCAGCATCACCTGCAAGGCCAGTCAGGATGTGGATACTTCTGTAGCCTGG
TATCAACAGAAACCAGGGCAAGCTCCTAAACTTCTGGTTTACTGGGCATCCACCC
GCCACACTGGAGTCCCTGATCGCTTCACAGGCAGTGGATCTGGGACAGATTTCAC
TCTCACCATTAGCAATGTGCAGTCTGAAGACTTGGCAGATTATTTCTGTCAGCAA
TATAGCAACTATCCCACGTTCGGTGCTGGGACCAAGCTGGAACTGAAACGT (SEQ
ID NO:29)
VH

MKCSWVIFFLMAVVTGVNSEVQLQQSGAELVRPGASVKLSCKASGFNIKDYYMHG
VKHRPEQGLEWIGRIDPENGNTIYDPKFQGKATITADTSSNTAYLQLSSLTSEDTAVY
YCARSPYDYHAWFAYWGQGTLVTVSS (SEQ ID NO:30)

ATGAAATGCAGCTGGGTCATCTTCTTCCTGATGGCAGTGGTTACAGGGGTCAATT
CAGAGGTTCAGCTGCAGCAGTCTGGGGCTGAGCTTGTGAGGCCAGGGGCCTCAG
TCAAGTTGTCCTGCAAAGCTTCTGGCTTCAACATTAAAGACTACTATATGCACGG
GGTGAAACATAGGCCTGAACAGGGCCTGGAGTGGATTGGAAGGATTGATCCTGA
AAATGGTAATACTATATATGACCCGAAGTTCCAGGGCAAGGCCACTATAACAGC
AGACACATCCTCCAACACAGCCTACCTGCAGCTCAGCAGCCTGACATCTGAGGA
CACTGCCGTCTATTACTGTGCTAGATCCCCTTATGATTACCACGCCTGGTTTGCTT
ACTGGGGCCAAGGGACTCTGGTCACCGTCTCGAGC (SEQ ID NO:31)

### Antibody HH

VK 
MMSSAQFLGILLLWFPGIKCDIKMTQSPSSMYTSLGERVTITCKASQDISNYFTWFQQ
KPGKSPKTLIYRANRLVDGVPSRFSGSGSGQDFSLTISSLEYEDMGIYYCLQYDEFPY
TFGGGTKLELKR (SEQ ID NO:32)
 
ATGATGTCCTCTGCTCAGTTTCTTGGAATCTTGTTGCTCTGGTTTCCAGGTATCAA
ATGTGACATCAAGATGACCCAGTCTCCATCTTCCATGTATACATCTCTAGGCGAG
AGAGTCACTATCACTTGCAAGGCGAGTCAGGACATTAGTAACTATTTACCTGGT
TCCAGCAGAAACCAGGGAAATCTCCTAAGACCCTGATCTATCGTGCAAACAGAT
TGGTAGATGGGGTCCCATCAAGGTTCAGTGGCAGTGGATCTGGGCAAGATTTTTC
TCTCACCATCAGCAGCCTCGAATATGAAGATATGGGAATTTATTATTGTCTACAG
TATGATGAGTTTCCGTACACGTTCGGAGGGGGGACCAAGTTGGAGCTCAAACGT
(SEQ ID NO:33)
VH

MNFGLSLIFLVLVLKGVLCEVKLVESGGGVMQPGGSLKLSCAASGFTFRTYTMSWV
RQTPEKRLEWVAYISDGGGSSYFPDTVKGRFTVSRDNAKNTLYLQMSSLRSEDTAM
YYCTRHSNWYFDVWGAGTSVTVSS (SEQ ID NO:34)

ATGAACTTCGGGCTCAGCCTGATTTTCCTTGTCCTTGTTTTAAAAGGTGTCCTGTG
TGAAGTGAAGTTGGTGGAGTCTGGGGGAGGTGTAATGCAGCCTGGAGGGTCCCT
GAAACTCTCCTGTGCAGCCTCTGGGTTCACTTTCAGAACCTATACCATGTCTTGG
GTTCGCCAGACTCCAGAGAAGAGGCTGGAGTGGGTCGCATATATTAGTGATGGT
GGTGGTAGTTCCTACTTTCCAGACACTGTCAAGGGCCGATTCACCGTCTCCAGGG
ACAATGCCAAGAACACCCTATACCTGCAAATGAGCAGTCTGAGGTCTGAGGACA
CGGCCATGTATTACTGTACAAGACATTCTAACTGGTACTTCGATGTCTGGGGCGC
AGGGACCTCAGTCACCGTCTCGAGC (SEQ ID NO:35)

### Antibody II

VK 
MMSSAQFLGLLLLCFQGTRCDIQMTQTTSSLSASLGDRVTIRCRASQDISNYLNWYQ
QKPDGPVKLLIYYTSTLTSGVPSRFSGSGSGTDYSLTISNLDQEDIATYFCQQGKTFPF
TFGSGTKLEIKR (SEQ ID NO:35)
 
ATGATGTCCTCTGCTCAGTTCCTTGGTCTCCTGTTGCTCTGTTTTCAAGGTACCAG
ATGTGATATCCAGATGACACAGACTACATCCTCCCTGTCTGCCTCTCTGGGAGAC
AGAGTCACCATCCGTTGCAGGGCAAGTCAGGACATTAGCAATTATTTAAACTGGT
ATCAGCAGAAACCAGATGGACCTGTTAAACTCCTGATCTACTACACATCTACATT
GACCTCAGGAGTCCCATCAAGGTTCAGTGGCAGTGGGTCTGGAACAGATTATTCT
CTCACCATTAGCAACCTGGACCAAGAAGATATTGCCACTTACTTTTGCCAACAGG
GTAAGACGTTTCCATTCACGTTCGGCTCGGGGACAAAGTTGGAAATAAAACGT
(SEQ ID NO:37)
VH

MEWSGVFIFLLSVTAGVHSQVQLQQSGAELVRPGTSVKVSCKASGYAFTNYFIEWV
KQRPGQGLEWIGAINPGSGGTNYNERFKGKATLSADKSSSTAYMQLSSLTSDDSAVY
FCAREDYGDVYAMDYWGQGTSVTVSS (SEQ ID NO:38)

ATGGAATGGAGCGGGGTCTTTATCTTTCTCCTGTCAGTAACTGCAGGTGTTCACT
CCCAGGTCCAGCTGCAGCAGTCTGGAGCTGAGCTGGTCAGGCCTGGGACTTCAGT
GAAGGTGTCCTGCAAGGCTTCTGGATACGCCTTCACTAATTACTTCATAGAGTGG
GTAAAACAGAGGCCTGGACAGGGCCTTGAGTGGATTGGAGCGATTAATCCTGGA
AGTGGTGGTACTAACTACAATGAGAGGTTCAAGGGCAAGGCAACTCTGTCTGCA
GACAAATCCTCCAGCACTGCCTACATGCAGCTCAGCAGCCTGACATCTGATGACT
CTGCGGTCTATTTCTGTGCAAGAGAGGACTATGGTGATGTCTATGCTATGGACTA
CTGGGGTCAAGGAACCTCGGTCACAGTCTCGAGC (SEQ ID NO:39)

### Antibody JJ

VK 
MGIKMESHSQVFVYMLLWLSGVEGDIVMTQSHKFMSTSVGDRVSITCKASQDVDTS
VAWYQQKPGQAPKLLIYWASTRHTGVPDRFTGSGSGTDFTLTISNVQSEDLADYFCQ
QYSSYPTFGAGTKLELKR (SEQ ID NO:40)
 
ATGGGCATCAAGATGGAGTCACATTCTCAGGTCTTTGTATACATGTTGCTGTGGT
TGTCTGGTGTTGAAGGAGACATTGTGATGACCCAGTCTCACAAATTCATGTCCAC
ATCAGTAGGAGACAGGGTCAGCATCACCTGCAAGGCCAGTCAGGATGTGGATAC
TTCTGTAGCCTGGTATCAACAGAAACCAGGGCAAGCTCCTAAACTACTGATTTAC
TGGGCATCCACCCGGCACACTGGAGTCCCTGATCGCTTCACAGGCAGTGGATCTG
GGACAGATTTCACTCTCACCATTAGCAATGTGCAGTCTGAAGACTTGGCAGATTA
TTTCTGTCAGCAATATAGCAGCTATCCCACGTTCGGTGCTGGGACCAAGCTGGAA
CTGAAACGT (SEQ ID NO:41)
VH

MEWSGVIFFLMAVVTGVNSEVQLQQSGAELVRPGASVKLSCKASGFNIKDYYMHG
VKHRPEQGLEWIGRIDPENGNTIYDPKFQGKATITADTSSNTAYLQLSSLTSEDTAVY
YCARSPYDYHAWFAYWGQGTLVTVSS (SEQ ID NO:42)

ATGGAATGGAGCGGAGTCATCTTCTTCCTGATGGCAGTGGTTACAGGGGTCAATT
CAGAGGTTCAGCTGCAGCAGTCTGGGGCTGAGCTTGTGAGGCCAGGGGCCTCAG
TCAAGTTGTCCTGCAAAGCTTCTGGCTTCAACATTAAAGACTACTATATGCACGG
GGTGAAACATAGGCCTGAACAGGGCCTGGAGTGGATTGGAAGGATTGATCCTGA
GAATGGTAATACTATATATGACCCGAAGTTCCAGGGCAAGGCCACTATAACAGC
AGACACATCCTCCAACACAGCCTACCTGCAGCTCAGCAGCCTGACATCTGAGGA
CACTGCCGTCTATTACTGTGCTAGATCCCCTTATGATTACCACGCCTGGTTTGCTT
ACTGGGGCCAAGGGACTCTGGTCACTGTCTCGAGC (SEQ ID NO:43)

### Antibody KK Humanized

VK 
MGIKMESHSQVFVYMLLWLSGVEGDIQMTQSPSSLSASVGDRVTITCKASQDVDTS
VAWYQQKPGKAPKLLIYWASTRHTGVPDRFSGSGSGTDFTLTISSLQPEDFATYYCQ
QYSSYPTFGQGTKLEIKR (SEQ ID NO:44)
 
ATGGGCATCAAGATGGAGTCACATTCTCAGGTCTTTGTATACATGTTGCTGTGGT
TGTCTGGTGTTGAAGGAGATATCCAGATGACCCAGTCTCCATCCTCCCTGTCTGC
ATCTGTAGGCGACCGTGTCACCATCACTTGCAAGGCAAGTCAGGACGTTGATACC
TCTGTAGCTTGGTATCAGCAGAAACCAGGGAAAGCCCCTAAGCTCCTGATCTATT
GGGCATCCACTCGTCATACTGGGGTCCCAGACCGCTTCAGTGGCAGTGGTTCTGG
GACAGATTTCACTCTCACCATCAGCAGTCTGCAACCTGAAGATTTTGCAACTTAT
TACTGTCAGCAATATAGCAGTTACCCTACGTTCGGTCAGGGGACCAAGCTGGAA
ATTAAACGT (SEQ ID NO:45)
VH

MEWSGVIFFLMAVVTGVNSEVQLVQSGAEVKKPGATVKISCKASGFNIKDYYMHG
VQHAPGKGLEWIGRIDPENGNTIYDPKFQGRVTITADTSTNTAYMELSSLRSEDTAV
YYCARSPYDYHAWFAYWGQGTLVTVSS (SEQ ID NO:46)

ATGGAATGGAGCGGAGTCATCTTCTTCCTGATGGCAGTGGTTACAGGGGTCAATT
CAGAGGTCCAGCTGGTACAGTCTGGGGCTGAGGTGAAGAAGCCTGGGGCTACAG
TGAAAATCTCCTGCAAGGCTTCTGGATTCAACATCAAAGACTACTACATGCACGG
GGTGCAACATGCCCCTGGAAAAGGGCTTGAGTGGATTGGACGTATTGATCCTGA
AAATGGTAACACAATCTACGACCCGAAGTTCCAGGGCCGCGTCACCATTACCGC
GGACACGTCTACAAACACAGCCTACATGGAGCTGAGCAGCCTGCGTTCTGAGGA
CACGGCCGTGTATTACTGTGCACGTTCTCCGTATGATTATCACGCCTGGTTTGCTT
ACTGGGGCCAAGGGACTCTGGTCACCGTCTCGAGC (SEQ ID NO:47)

### Antibody LL

VK 
MKLPVLLWVLLLWVPGSTGDIVLTQSPASLAVSLGQRATIYCKASQSVDYDGDSYM
NWYQQKPGQPPKVLIFAASNLESGIPARFSGSRSGTDFTLNIHPVEEEDAATYYCQQT
NEDPPTFGGGTKLELKR (SEQ ID NO:48)
 
ATGAAGCTGCCTGTTCTGCTATGGGTGCTGCTGCTCTGGGTTCCAGGCTCCACTG
GTGACATTGTGCTGACCCAATCTCCAGCTTCTTTGGCTGTGTCTCTAGGGCAGAG
GGCCACCATTTACTGCAAGGCCAGCCAAAGTGTTGATTATGATGGTGATAGTTAT
ATGAACTGGTACCAACAGAAACCAGGACAGCCACCCAAAGTCCTCATCTTTGCT
GCATCCAATCTAGAATCTGGGATCCCGGCCAGGTTTAGTGGCAGTAGATCTGGGA
CAGACTTCACCCTCAACATCCATCCTGTGGAGGAGGAGGATGCTGCAACCTATTA
CTGTCAACAAACTAATGAGGATCCTCCGACGTTCGGTGGCGGCACCAAGCTGGA
ACTGAAACGT (SEQ ID NO:49)
VH

MEWSWIILFLVATATDVHSQVQLQQSGAELVKPGASVKLSCKASGYAFISFWIHWV
KQRPGQGLEWIGEINPSNGRTDYNAKFKTKATLTVDRSSSTAYMQLSSLTSEDSADY
YCARGGTGTWYFDVWGAGTTVTVSS (SEQ ID NO:50)

ATGGAATGGAGCTGGATCATCCTCTTTTTGGTAGCAACAGCTACAGATGTCCACT
CCCAGGTCCAACTGCAGCAGTCTGGGGCTGAACTGGTGAAGCCTGGGGCTTCAG
TGAAGCTGTCCTGCAAGGCTTCTGGCTACGCCTTTATTAGCTTCTGGATACACTG
GGTGAAGCAGAGGCCTGGTCAAGGCCTTGAGTGGATTGGAGAGATTAATCCTAG
CAACGGTCGTACTGACTACAATGCGAAGTTCAAGACCAAGGCCACACTGACTGT
TGACAGATCCTCCTCCACAGCCTACATGCAACTCAGCAGCCTGACATCTGAGGAC
TCTGCGGACTATTACTGTGCAAGAGGAGGAACTGGGACCTGGTACTTCGATGTCT
GGGGCGCAGGGACCACAGTCACCGTCTCGAGC (SEQ ID NO:51)

### Antibody MM

VK 
MMSPAQFLFLLVLWIRETNGDVVMTQTPLTLSVTIGQPASISCKSSQSLLDSDGETYL
NWLLQRPGQSPKRLIYLVSKLDSGVPDRFTGSGSGTDFTLKISRVETEDLGVYYCWQ
GTHFPYTFGGGTKLELKR (SEQ ID NO:52)
 
ATGATGAGTCCTGCCCAGTTCCTGTTTCTGTTAGTGCTCTGGATTCGGAAACCA
ACGGTGATGTTGTGATGACCCAGACTCCACTCACTTTGTCGGTTACCATTGGACA
ACCAGCCTCCATCTCTTGCAAGTCAAGTCAGAGCCTCTTAGATAGTGATGGAGAG
ACATATTTGAATTGGTTGTTACAGAGGCCAGGCCAGTCTCCAAAGCGCCTAATCT
ATCTGGTGTCTAAACTGGACTCTGGAGTCCCTGACAGGTTCACTGGCAGTGGATC
AGGGACAGATTTCACACTGAAAATCAGCAGAGTGGAGACTGAGGATTTGGGAGT
TTATTATTGCTGGCAAGGTACACATTTTCCGTACACGTTCGGAGGGGGGACCAAG
CTGGAACTGAAACGT (SEQ ID NO:53)
VH

MNLGLNCVFIVFLLKGVQSEVKLEESGGGLVQPGGSMKLSCVASGFTFSNYWMNW
VRQSPEKGLEWVAEIRLKSDNYATHFAESVKGRFTISRDDSKSSVYLQMNNLRAEDT
GIYYCTGILFGYWGQGTLVTVSS (SEQ ID NO:54)

ATGAACTTGGGACTGAACTGTGTATTCATAGTTTTTCTCTTAAAAGGTGTCCAGA
GTGAAGTGAAACTTGAGGAGTCTGGAGGAGGCTTGGTGCAACCTGGAGGATCCA
TGAAACTCTCCTGTGTTGCCTCTGGATTCACTTTCAGTAACTACTGGATGAACTGG

GTCCGCCAGTCTCCAGAGAAGGGGCTTGAGTGGGTTGCTGAAATTAGATTGAAA
TCTGATAATTATGCAACACATTTTGCGGAGTCTGTGAAAGGGAGGTTCACCATCT
CAAGAGATGATTCCAAAAGTAGTGTCTACCTGCAAATGAACAACTTAAGAGCTG
AAGACACTGGCATTTATTACTGTACGGGTATCCTCTTTGGTTACTGGGGCCAAGG
GACTCTGGTCACTGTCTCGAGC (SEQ ID NO:55)

### Antibody

VK 
MESDTILLWVLLLWVPGSTGDIVLTQSPASLAVSLGQRATISCKASQSVDYDGDSYM
NWYQQKPGQPPKLLIYAASNLESGIPARFSGSGSGTDFTLNIHPVEEEDAATYYCQQS
NEDPWTFGGGTKLELKR (SEQ ID NO:56)
 
ATGGAGTCAGACACAATCCTGCTATGGGTGCTGCTGCTCTGGGTTCCAGGCTCCA
CTGGTGACATTGTGCTGACCCAATCTCCAGCTTCTTTGGCTGTGTCTCTAGGGCAG
AGGGCCACCATCTCCTGCAAGGCCAGCCAAAGTGTTGATTATGATGGTGATAGTT
ATATGAACTGGTACCAACAGAAACCAGGACAGCCACCCAAACTCCTCATCTATG
CTGCATCCAATCTAGAATCTGGGATCCCAGCCAGGTTTAGTGGCAGTGGGTCTGG
GACAGACTTCACCCTCAACATCCATCCTGTGGAGGAGGAGGATGCTGCTACCTAT
TACTGTCAGCAAAGTAATGAGGATCCGTGGACGTTCGGTGGAGGCACCAAGCTG
GAGCTGAAACGT (SEQ ID NO:57)
VH

MEWSWIILFLVATATDVHSQVQLLQPGAELVKPGASVKLSCKASGYTFTSYWMHW
VKQRPGQGLEWIGEINPSNGRTDYNENFKSKATLTVDKSSSTAYMQLSSLTSEDSAV
YYCARGGVYAMDYWGQGTSVTVSS (SEQ ID NO:58)

ATGGAATGGAGCTGGATCATCCTCTTTTTGGTAGCAACAGCTACAGATGTCCACT
CCCAGGTCCAACTGCTGCAGCCTGGGGCTGAACTGGTGAAGCCTGGGGCTTCAGT
GAAGCTGTCCTGCAAGGCTTCTGGCTACACCTTCACCAGCTACTGGATGCACTGG
GTGAAGCAGAGGCCTGGGCAAGGCCTTGAGTGGATTGGAGAGATTAATCCTAGC
AACGGTCGTACTGACTACAATGAGAACTTCAAGAGCAAGGCCACACTGACTGTA
GACAAATCCTCCAGCACAGCCTACATGCAACTCAGCAGCCTGACATCTGAGGAC
TCTGCGGTCTATTACTGTGCAAGAGGGGGGGTCTATGCTATGGACTACTGGGGTC
AAGGAACCTCAGTCACCGTCTCGAGC (SEQ ID NO:59)

### Antibody OO

VK 
MKLPVRLLVLMFWIPVSSSDVVMTQTPLSLPVSLGDQASISCRSSQSLVHSNGDTYL
HWYLQKPGQSPKLLIYEISNRFSGVPDRFSGSGSGTDFTLKISRVEAEDLGVYFCSQST
HVPFTFGGGTKLEIKR (SEQ ID NO:60)
 
ATGAAGTTGCCTGTTAGGCTGTTGGTGCTGATGTTCTGGATTCCTGTTTCCAGTAG
TGATGTTGTGATGACCCAAACTCCACTCTCCCTGCCTGTCAGTCTTGGAGATCAA
GCCTCCATCTCTTGCAGATCTAGTCAGAGCCTTGTACACAGTAATGGAGACACCT
ATTTACATTGGTACCTACAGAAGCCAGGCCAGTCTCCAAAACTCCTGATCTACGA
AATTTCCAACCGATTTTCTGGGGTCCCAGACAGGTTCAGTGGCAGTGGATCAGGG
ACAGATTTCACACTCAAGATCAGCAGAGTGGAGGCTGAGGATCTGGGAGTTTAT
 
TTCTGCTCTCAAAGTACACATGTTCCGTTCACGTTCGGAGGGGGACCAAGCTGG
AAATAAAACGT (SEQ ID NO:61)
VH

MKCSWVIFFLMAVVIGITSEIQLQQSGTELVRSGASVKLSCTASGFNIKDYYIHWVRQ
RPDQGLEWIGWIDPENGDTEYAPKFQDRATLTADTSSNTAYLQLTLLTSEDTAVYYC
NEGSGLIPYWGQGTPVTVSS (SEQ ID NO:62)

ATGAAATGCAGCTGGGTCATCTTCTTCCTGATGGCAGTGGTTATAGGAATCACTT
CAGAGATTCAGCTGCAGCAGTCTGGGACAGAGCTTGTGAGGTCAGGGGCCTCAG
TCAAATTGTCCTGCACAGCTTCTGGCTTCAACATTAAAGACTACTATATTCACTG
GGTGAGACAGAGGCCTGACCAGGGCCTGGAGTGGATTGGATGGATTGATCCTGA
GAATGGTGATACTGAATATGCCCCGAAGTTCCAGGACAGGGCCACTTTGACTGC
AGACACATCCTCCAACACAGCCTACCTGCAGCTCACACTCCTGACATCTGAGGAC
ACTGCCGTCTATTACTGTAATGAGGGCTCGGGCTTGATTCCTTACTGGGGCCAAG
GGACTCCGGTCACCGTCTCGAGC (SEQ ID NO:63)

### Antibody PP

VK 
MMSSAQFLGLLLLCFQGTRCDIQMTQTTSSLSASLGDRVTIRCRASQDISNYLNWYQ
QKPDGPVKLLIYYTSTLTSGVPSRFSGSGSGTDYSLTISNLDQEDIATYFCQQGKTFPF
TFGSGTKLEIKR (SEQ ID NO:64)
 
ATGATGTCCTCTGCTCAGTTCCTTGGTCTCCTGTTGCTCTGTTTTCAAGGTACCAG
ATGTGATATCCAGATGACACAGACTACATCCTCCCTGTCTGCCTCTCTGGGAGAC
AGAGTCACCATCCGTTGCAGGGCAAGTCAGGACATTAGCAATTATTTAAACTGGT
ATCAGCAGAAACCAGATGGACCTGTTAAACTCCTGATCTACTACACATCTACATT
GACCTCAGGAGTCCCATCAAGGTTCAGTGGCAGTGGGTCTGGAACAGATTATTCT
CTCACCATTAGCAACCTGGACCAAGAAGATATTGCCACTTACTTTTGCCAACAGG
GTAAGACGTTTCCATTCACGTTCGGCTCGGGGACAAAGTTGGAAATAAAACGT
(SEQ ID NO:65)
VH

MEWSWVFIFLLSVTAGVHSQVQLQQSGAELVRPGTSVKVSCKASGYVFINYFIEWV
KQRPGQGLEWIGVINPENGGTNYNERFKDKAALTADTSSSTAYMQLSSLTSDDSAV
YFCAREDYGDVYAMDYWGQGTSVTVSS (SEQ ID NO:66)

ATGGAATGGAGCTGGGTCTTTATCTTTCTCCTGTCAGTTACTGCAGGTGTTCACTC
CCAGGTCCAGCTGCAGCAGTCTGGAGCTGAACTGGTCAGGCCTGGGACTTCAGT
GAAGGTGTCCTGCAAGGCTTCTGGATACGTCTTCATTAATTACTTCATAGAGTGG
GTTAAACAGAGGCCTGGACAGGGCCTTGAGTGGATTGGAGTGATTAATCCTGAA
AATGGTGGTACTAACTACAATGAGAGATTCAAGGACAAGGCAGCACTGACTGCA
GACACATCCTCCAGCACTGCCTACATGCAACTCAGCAGCCTGACATCTGATGACT
CTGCGGTCTATTTCTGTGCAAGAGAGGACTATGGTGATGTCTATGCTATGGACTA
CTGGGGTCAAGGAACCTCAGTCACAGTCTCGAGC (SEQ ID NO:67)

### Antibody QQ

VK 
MKLPVRLLVLMFWIPVSSSDVVMTQTPLSLPVSLGDQASISCRSSQSLVHSNGDTYL
HWYLQKPGQSPKLLIYEISNRFSGVPDRFSGSGSGTDFTLKISRVEAEDLGVYFCSQST
HVPFTFGGGTKLEIKR (SEQ ID NO:68)
 
ATGAAGTTGCCTGTTAGGCTGTTGGTGCTGATGTTCTGGATTCCTGTTTCCAGTAG
TGATGTTGTGATGACCCAAACTCCACTCTCCCTGCCTGTCAGTCTTGGAGATCAA
GCCTCCATCTCTTGCAGATCTAGTCAGAGCCTTGTACACAGTAATGGAGACACCT
ATTTACATTGGTACCTACAGAAGCCAGGCCAGTCTCCAAAACTCCTGATCTACGA
AATTTCCAACCGATTTTCTGGGGTCCCAGACAGGTTCAGTGGCAGTGGATCAGGG
ACAGATTTCACACTCAAGATCAGCAGAGTGGAGGCTGAGGATCTGGGAGTTTAT
TTCTGCTCTCAAAGTACACATGTTCCGTTCACGTTCGGAGGGGGGACCAAGCTGG
AAATAAAACGT (SEQ ID NO:69)
VH

MKCSWVIFFLMAVVIGINSEVQLQQSGTELVRSGASVRLSCTASGFNIKDYYIHWVK
QRPEQGLEWIGWIDPENGDSEYAPKFQDKATMTADTSSNTAYLQLSSLTSEDTAVYY
CNEGSGLIPYWGHGTLVTVSS (SEQ ID NO:70)

ATGAAATGCAGCTGGGTCATCTTCTTCCTGATGGCAGTGGTTATAGGAATCAATT
CAGAGGTTCAGCTGCAGCAGTCTGGGACAGAGCTTGTGAGGTCAGGGGCCTCAG
TCAGGTTGTCCTGCACAGCTTCTGGCTTCAACATTAAAGACTACTATATACACTG
GGTGAAACAGAGGCCTGAACAGGGCCTGGAGTGGATTGGATGGATTGATCCTGA
GAATGGTGATTCTGAATATGCCCCGAAGTTCCAGGACAAGGCCACCATGACTGC
AGACACATCCTCCAACACAGCCTACCTGCAGCTCAGCAGCCTGACATCTGAGGA
CACTGCCGTCTATTATTGTAATGAGGGCTCGGGCTTGATTCCTTACTGGGGCCAC
GGGACTCTGGTCACAGTCTCGAGC (SEQ ID NO:71)

### Antibody RR

VK 
MSVPTQVLGLLLLWLTDARCDIVLTQTPLSLPVSLGDHASISCRSSQSLVHSNGDVYF
HWYLQKPGQSPKLLIYEVSNRFSGVPDRFSGSGSGTYFTLKISRVEAEDLGVYFCSQS
THVPYTFGGGTKLELKR (SEQ ID NO:72)
 
ATGTCTGTCCCCACCCAAGTCCTCGGACTCCTGCTGCTGTGGCTTACAGATGCCA
GATGCGATATTGTGTTGACCCAAACTCCTCTCTCTCTGCCTGTCAGTCTTGGAGAT
CATGCCTCCATCTCTTGCAGATCTAGTCAGAGCCTTGTACACAGTAATGGCGACG
TCTATTTCCATTGGTACCTGCAGAAGCCAGGCCAGTCTCCAAAGCTCCTGATCTA
CGAAGTTTCCAACCGATTTTCTGGGGTCCCAGACAGGTTCAGTGGCAGTGGATCA
GGGACATATTTCACACTCAAGATCAGCAGAGTGGAGGCTGAGGATCTGGGAGTT
TATTTCTGCTCTCAAAGTACACATGTTCCGTACACTTTCGGAGGGGGGACCAAGT
TGGAGCTCAAACGT (SEQ ID NO:73)
VH

MKCSWVIFFLMAVVIGINSEVQLQQSGTELVRSGASVRLSCTASGFNIKDYYIHWVK
QRPEQGLEWIGWIDPENGDSEYAPKFQDKATMTADTSSNTAYLQLSSLTSEDTAVYY
CNEGSGLIPYWGHGTLVTVSS (SEQ ID NO:74)

ATGAAATGCAGCTGGGTCATCTTCTTCCTGATGGCAGTGGTTATAGGAATCAATT
CAGAGGTTCAGCTGCAGCAGTCTGGGACAGAGCTTGTGAGGTCAGGGGCCTCAG
TCAGGTTGTCCTGCACAGCTTCTGGCTTCAACATTAAAGACTACTATATACACTG
GGTGAAACAGAGGCCTGAACAGGGCCTGGAGTGGATTGGATGGATTGATCCTGA
GAATGGTGATTCTGAATATGCCCCGAAGTTCCAGGACAAGGCCACCATGACTGC
AGACACATCCTCCAACACAGCCTACCTGCAGCTCAGCAGCCTGACATCTGAGGA
CACTGCCGTCTATTATTGTAATGAGGGCTCGGGCTTGATTCCTTACTGGGGCCAC
GGGACTCTGGTCACAGTCTCGAGC (SEQ ID NO:75)

### Antibody SS

VK 
MSVPTQVLGLLLLWLTDARCDVVLTQTPLSLPVSLGDQASISCRSSQSLVHSNGDVY
LHWYLQKPGQSPKLLIYEVSNRFSGVPDRFSGSGSGTDFTLKINRVEAEDLGVYFCSQ
STHVPYTFGGGTKLELK (SEQ ID NO:76)
 
ATGTCTGTCCCCACCCAAGTCCTCGGACTCCTGCTGCTGTGGCTTACAGATGCCA
GATGCGATGTTGTGTTGACCCAAACTCCACTCTCCCTGCCTGTCAGTCTTGGAGA
TCAAGCCTCCATCTCTTGCAGATCTAGTCAGAGCCTTGTACACAGTAATGGAGAC
GTCTATTTACATTGGTACCTGCAGAAGCCAGGCCAGTCTCCAAAGCTCCTGATCT
ACGAAGTTTCCAACCGATTTTCTGGGGTCCCAGACAGGTTCAGTGGCAGTGGATC
AGGGACAGATTTCACACTCAAGATCAACAGAGTGGAGGCTGAGGATCTGGGAGT
TTATTTCTGCTCTCAAAGTACACATGTTCCGTACACGTTCGGAGGGGGGACCAAG
CTGGAACTGAAACGT (SEQ ID NO:77)
VH

MEWSWVIFFLMAVVIGINSEVQLQQSGTELVRSGASVKLSCTASGFNIKDYYVHWV
KQRPEQGLEWIGWIDPDNGDSEYAPKFQDKATMTADTSSNTAYLQLNSLTSEDTAV
YYCNEGSGLIPYWGPGTLVTVSS (SEQ ID NO:78)

ATGGAATGGAGCTGGGTCATCTTCTTCCTGATGGCAGTGGTTATAGGAATCAATT
CTGAGGTTCAGCTGCAGCAGTCTGGGACAGAGCTTGTGAGGTCAGGGGCCTCAG
TCAAGTTGTCCTGCACAGCTTCTGGCTTCAACATTAAAGACTACTATGTACACTG
GGTGAAGCAGAGGCCTGAACAGGGCCTGGAATGGATTGGATGGATTGATCCTGA
CAATGGTGATAGTGAATATGCCCCGAAGTTCCAGGACAAGGCCACTATGACTGC
AGACACATCCTCCAACACAGCCTACCTTCAACTCAACAGCCTGACTTCTGAGGAC
ACTGCCGTCTATTATTGTAATGAGGGCTCGGGCTTGATTCCTTACTGGGGCCCAG
GGACTCTGGTCACAGTCTCGAGC (SEQ ID NO:79)

### Antibody TT

VK 
MSVPTQVLGLLLLWLTDARCDVVMTQTPLSLPVSLGDQASISCRSSQSLVHSNGDVY
LHWYLQKPGQSPKLLIYEVSNRFSGVPDRFSGSGSGTDFTLKISRVEAEDLGVYFCSQ
TTHVPYTFGGGTKLELKR (SEQ ID NO:80)
 
ATGTCTGTCCCCACCCAAGTCCTCGGACTCCTGCTGCTGTGGCTTACAGATGCCA
GATGCGATGTTGTGATGACCCAAACTCCACTCTCCCTGCCTGTCAGTCTTGGAGA
TCAAGCCTCCATCTCCTGCAGATCTAGTCAGAGCCTTGTACACAGTAATGGAGAC
GTCTATTTACATTGGTACCTGCAGAAGCCAGGCCAGTCTCCAAAACTCCTGATCT
ACGAAGTTTCCAACCGATTTTCTGGGGTCCCAGACAGGTTCAGTGGCAGTGGTTC
AGGGACAGATTTCACACTCAAGATCAGCAGAGTGGAGGCTGAGGATCTGGGAGT
TTATTTCTGTTCTCAAACTACACATGTTCCGTACACGTTCGGAGGGGGGACCAAG
CTGGAGCTGAAACGT (SEQ ID NO:81)
VH

MKCSWVIFFLMAVVIGITSEIQLQQSGTELVRSGASVKLSCTASGFNIKDYYIHWVRQ
RPDQGLEWIGWIDPENGDTEYAPKFQDRATLTADTSSNTAYLQLTLLTSEDTAVYYC
NEGSGLIPYWGQGTPVTVSS (SEQ ID NO:82)

ATGAAATGCAGCTGGGTCATCTTCTTCCTGATGGCAGTGGTTATAGGAATCACTT
CAGAGATTCAGCTGCAGCAGTCTGGGACAGAGCTTGTGAGGTCAGGGGCCTCAG
TCAAATTGTCCTGCACAGCTTCTGGCTTCAACATTAAAGACTACTATATTCACTG
GGTGAGACAGAGGCCTGACCAGGGCCTGGAGTGGATTGGATGGATTGATCCTGA
GAATGGTGATACTGAATATGCCCCGAAGTTCCAGGACAGGGCCACTTTGACTGC
AGACACATCCTCCAACACAGCCTACCTGCAGCTCACACTCCTGACATCTGAGGAC
ACTGCCGTCTATTACTGTAATGAGGGCTCGGGCTTGATTCCTTACTGGGGCCAAG
GGACTCCGGTCACCGTCTCGAGC (SEQ ID NO:83)

### Antibody UU

VK 
MSVPTQVLGLLLLWLTDARCDVVMTQTPLSLPVSLGDQASISCRSSQSLVHSNGDVY
LHWYLQKPGQSPKLLIYEVSNRFSGVPDRFTGSGSGTDFTLKISRVEAEDLGVFFCSQ
STHVPYTFGGGTKLELKR (SEQ ID NO:84)
 
ATGTCTGTCCCCACCCAAGTCCTCGGACTCCTGCTGCTGTGGCTTACAGATGCCA
GATGCGATGTTGTGATGACCCAAACTCCACTCTCCCTGCCTGTCAGTCTTGGAGA
TCAAGCCTCCATCTCCTGCAGATCTAGTCAGAGCCTTGTACACAGTAATGGAGAC
GTCTATTTACATTGGTACCTGCAGAAGCCAGGCCAGTCTCCAAAACTCCTGATCT
ACGAAGTTTCCAACCGATTTTCTGGGGTCCCAGACAGGTTCACTGGCAGTGGTTC
AGGGACAGATTTCACACTCAAGATCAGCAGAGTGGAGGCTGAGGATCTGGGAGT
TTTTTTCTGCTCTCAAAGTACACATGTTCCGTACACGTTCGGAGGGGGGACCAAG
CTGGAACTGAAACGT (SEQ ID NO:85)
VH

MKCSWVIFFLMAVVIGITSEIQLQQSGTELVRSGASVKLSCTASGFNIKDYYIHWVKQ
RPDQGLEWIGWIDPENGDTEYAPKFQDRATMTADTSSNTAYLQLTILTSEDTAVYYC
NEGSGLIPYWGQGTLVTVSS (SEQ ID NO:86)

ATGAAATGCAGCTGGGTCATCTTCTTCCTGATGGCAGTGGTTATAGGAATCACTT
CAGAGATTCAGCTGCAGCAGTCTGGACAGAGCTTGTGAGGTCAGGGGCCTCAG
TCAAATTGTCCTGCACAGCTTCTGGCTTCAACATTAAAGACTACTATATTCACTG

GGTGAAACAGAGGCCTGACCAGGGCCTGGAGTGGATTGGATGGATTGATCCTGA
GAATGGTGATACTGAATATGCCCCGAAGTTCCAGGACAGGGCCACTATGACTGC
AGACACATCCTCCAACACAGCCTACCTGCAGCTCACCATCCTGACATCTGAGGAC
ACTGCCGTCTATTACTGTAATGAGGGCTCGGGCTTGATTCCTTACTGGGGCCAAG
GGACTCTGGTCACCGTCTCGAGC (SEQ ID NO:87)

### Antibody VV

VK 
MSVPTQVLGLLLLWLTDARCDIQMNQSPSSLSASLGDTITITCHASQNINVWLSWYQ
QKPGNIPKLLIYKVSNLHTGVPSRFSGSGSGTGFTLTISSLQPEDIATYYCQQGQSYPL
TFGGGTKLELKR (SEQ ID NO:88)
 
ATGTCTGTCCCCACCCAAGTCCTCGGACTCCTGCTGCTGTGGCTTACAGATGCCA
GATGCGACATCCAGATGAACCAGTCTCCATCCAGTCTGTCTGCATCCCTTGGAGA
CACAATTACCATCACTTGCCATGCCAGTCAGAACATTAATGTTTGGTTAAGCTGG
TACCAGCAGAAACCAGGAAATATTCCTAAACTATTGATCTATAAGGTTTCCAACT
TGCACACAGGCGTCCCATCAAGGTTTAGTGGCAGTGGATCTGGAACAGGTTTCAC
ATTAACCATCAGCAGCCTGCAGCCTGAAGACATTGCCACTTACTACTGTCAACAG
GGTCAAAGTTATCCTCTGACGTTCGGTGGAGGCACCAAGTTGGAGCTCAAACGT
(SEQ ID NO:89)
VH

MGWNWIIFFLMAVVTGVNSEVQLQQSGAELVRPGALVKLSCKASGFNIKDYYIHWV
KQRPEQGLEWIGRIDPENGNTIYDPKFQGKAIITADTSSNTAYLQLSSLTSEDTAVYY
CARCDNDPGSEMDYWGQGTTVTVSS (SEQ ID NO:90)

ATGGGATGGAACTGGATCATCTTCTTCCTGATGGCAGTGGTTACAGGGGTCAATT
CAGAGGTTCAGCTGCAGCAGTCTGGGGCTGAGCTTGTGAGGCCAGGGGCCTTAG
TCAAATTGTCCTGCAAAGCTTCTGGATTCAACATTAAAGACTACTATATACACTG
GGTGAAGCAGAGGCCTGAACAGGGCCTGGAGTGGATTGGAAGGATTGATCCTGA
AAATGGTAATACTATATATGACCCGAAGTTCCAGGGCAAGGCCATTATAACAGC
AGACACATCCTCCAACACAGCCTACCTGCAGCTCAGCAGCCTGACATCTGAGGA
CACTGCCGTCTATTACTGTGCTAGATGTGATAACGACCCCGGCTCTGAAATGGAC
TACTGGGGTCAAGGAACCACGGTCACCGTCTCGAGC (SEQ ID NO:91)

### Antibody WW

VK 
MSVPTQVLGLLLLWLTDARCDIVLTQTPLSLPVSLGDHASISCRSSQSLVHSNGDVYF
HWYLQKPGQSPKLLIYEVSNRFSGVPDRFSGSGSGTYFTLKISRVEAEDLGVYFCSQS
THVPYTFGGGTKLELKR (SEQ ID NO:92)
 
ATGTCTGTCCCCACCCAAGTCCTCGGACTCCTGCTGCTGTGGCTTACAGATGCCA
GATGCGATATTGTGTTGACCCAAACTCCTCTCTCTCTGCCTGTCAGTCTTGGAGAT
CATGCCTCCATCTCTTGCAGATCTAGTCAGAGCCTTGTACACAGTAATGGCGACG
TCTATTTCCATTGGTACCTGCAGAAGCCAGGCCAGTCTCCAAAGCTCCTGATCTA
CGAAGTTTCCAACCGATTTTCTGGGGTCCCAGACAGGTTCAGTGGCAGTGGATCA
GGGACATATTTCACACTCAAGATCAGCAGAGTGGAGGCTGAGGATCTGGGAGTT
 
TATTTCTGCTCTCAAAGTACACATGTTCCGTACACTTTCGGAGGGGGACCAAGT
TGGAGCTCAAACGT (SEQ ID NO:93)
VH

MEWSWVIFFLMAVVIGINSEVQLQQSGTELVRSGASVKLSCTASGFNIKDYYVHWV
KQRPEQGLEWIGWIDPDNGDSEYAPKFQDKATMTADTSSNTAYLQLNSLTSEDTAV
YYCNEGSGLIPYWGPGTLVTVSS (SEQ ID NO:94)

ATGGAATGGAGCTGGGTCATCTTCTTCCTGATGGCAGTGGTTATAGGAATCAATT
CTGAGGTTCAGCTGCAGCAGTCTGGGACAGAGCTTGTGAGGTCAGGGGCCTCAG
TCAAGTTGTCCTGCACAGCTTCTGGCTTCAACATTAAAGACTACTATGTACACTG
GGTGAAGCAGAGGCCTGAACAGGGCCTGGAATGGATTGGATGGATTGATCCTGA
CAATGGTGATAGTGAATATGCCCCGAAGTTCCAGGACAAGGCCACTATGACTGC
AGACACATCCTCCAACACAGCCTACCTTCAACTCAACAGCCTGACTTCTGAGGAC
ACTGCCGTCTATTATTGTAATGAGGGCTCGGGCTTGATTCCTTACTGGGGCCCAG
GGACTCTGGTCACAGTCTCGAGC (SEQ ID NO:95)

For humanized antibodies DD and KK, light chain human kappa constant regions are: 
TVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC* (SEQ ID
NO:96)
 and heavy chain human gamma-4 constant regions are:

ASTKGPSVFPLAPCSRSTSESTAALGCLVKDYFPEPVTVSWNSGALTSGVHTFPAVLQ
SSGLYSLSSVVTVPSSSLGTKTYTCNVDHKPSNTKVDKRVESKYGPPCPPCPAPEFLG
GPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDSDGSFFLYS
RLTVDKSRWQEGNVFSCSVMHEALHNHYTQKSLSLSLGK*(SEQ ID NO:97)

The hinge region contains the Ser-241-Pro mutation to improve hinge stability (Angal S et al, Mol. Immunol, 30(1):105-108 (1993)).

The following table, Table 1, provides the SEQ ID NOs for the antibody polynucleotides and polypeptides disclosed above, without leader sequences.

**Table 1**

| Antibody | POLYPEPTIDES | | POLYNUCLEOTIDES | |
|---|---|---|---|---|
| | SEQ ID NO with leader | SEQ ID NO without leader | SEQ ID NO with leader | SEQ ID NO without leader |
| AA VK | 4 | 236 | 5 | 237 |
| AA VH | 6 | 238 | 7 | 239 |
| BB VK | 8 | 240 | 9 | 241 |
| BB VH | 10 | 242 | 11 | 243 |
| CC VK | 12 | 244 | 13 | 245 |
| CC VH | 14 | 246 | 15 | 247 |
| DD VK | 16 | 248 | 17 | 249 |
| DD VH | 18 | 250 | 19 | 251 |
| EE VK | 20 | 252 | 21 | 253 |
| EE VH | 22 | 254 | 23 | 255 |
| FF VK | 24 | 256 | 25 | 257 |
| FF VH | 26 | 258 | 27 | 259 |
| GG VK | 28 | 260 | 29 | 261 |
| GG VH | 30 | 262 | 31 | 263 |
| HH VK | 32 | 264 | 33 | 265 |
| HH VH | 34 | 266 | 35 | 267 |
| II VK | 36 | 268 | 37 | 269 |
| II VH | 38 | 270 | 39 | 271 |
| JJ VK | 40 | 272 | 41 | 273 |
| JJ VH | 42 | 274 | 43 | 275 |
| KK VK | 44 | 276 | 45 | 277 |
| KK VH | 46 | 278 | 47 | 279 |
| LL VK | 48 | 280 | 49 | 281 |
| LL VH | 50 | 282 | 51 | 283 |
| MM VK | 52 | 284 | 53 | 285 |
| MM VH | 54 | 286 | 55 | 287 |
| NN VK | 56 | 288 | 57 | 289 |
| NN VH | 58 | 290 | 59 | 291 |
| OO VK | 60 | 292 | 61 | 293 |
| OO VH | 62 | 294 | 63 | 295 |
| PP VK | 64 | 296 | 65 | 297 |
| PP VH | 66 | 298 | 67 | 299 |
| QQ VK | 68 | 300 | 69 | 301 |
| QQ VH | 70 | 302 | 71 | 303 |
| RR VK | 72 | 304 | 73 | 305 |
| RR VH | 74 | 306 | 75 | 307 |
| SS VK | 76 | 308 | 77 | 309 |
| SS VH | 78 | 310 | 79 | 311 |
| TT VK | 80 | 312 | 81 | 313 |
| TT VH | 82 | 314 | 83 | 315 |
| UU VK | 84 | 316 | 85 | 317 |
| UU VH | 86 | 318 | 87 | 319 |
| VV VK | 88 | 320 | 89 | 321 |
| VV VH | 90 | 322 | 91 | 323 |
| WW VK | 92 | 324 | 93 | 325 |
| WW VH | 94 | 326 | 95 | 327 |

Table 2 below provides the SEQ ID NOs and amino acid sequences of the CDR's of AA-WW. L1, L2, and L3 refer to light chain CDR's 1, 2, and 3, and H1, H2, and H3 refer to heavy chain CDR's 1, 2, and 3 according to the Kabat numbering system (Kaba et al., 1987 in Sequences of Proteins of Immunological Interest, U.S. Department of Health and Human Services, NIH, USA).

**Table 2**

| **Antibody** | **CDR 1** | **CDR 2** | **CDR 3** |
|---|---|---|---|
| AA VK | KASQSVDYAGDNYMN (SEQ ID NO:98) | TASNLES (SEQ ID NO:99) | QQSNEDPPT (SEQ ID NO:100) |
| AA VH | GYFMH (SEQ ID NO:101) | EINPSTGGTTYNQRFKG (SEQ ID NO:102) | WGYNPYALDY (SEQ ID NO:103) |
| BB VK | KASQDINKYIA (SEQ ID NO:104) | YTSTLQP (SEQ ID NO:105) | LQYDNLYT (SEQ ID NO:106) |
| BB VH | SYWIE (SEQ ID NO:107) | EIFPRNGSTYYNEKFKG (SEQ ID NO:108) | INTLDY (SEQ ID NO:109) |
| CC VK | RSSQSLVHSNGDTYLH (SEQ ID NO:110) | EISNRFS (SEQ ID NO:111) | SQSTHVPFT (SEQ ID NO:112) |
| CC VH | DYYMH (SEQ ID NO:113) | WNDPETGDTEYAPKFQG (SEQ ID NO:114) | GSGLIPY (SEQ ID NO:115) |
| DD VK | RSSQSLVHSNGDTYLH (SEQ ID NO:116) | EISNRFS (SEQ ID NO:117) | SQSTHVPFT (SEQ ID NO:118) |
| DD VH | DYYMH (SEQ ID NO:119) | WNDPETGDTEYAPKFQG (SEQ ID NO:120) | GSGLIPY (SEQ ID NO:121) |
| EE VK | RSSQSLVHSNGDVYLH (SEQ ID NO:122) | EVSNRFS (SEQ ID NO:123) | SQSTHVPFT (SEQ ID NO:124) |
| EE VH | NYYMH (SEQ ID NO:125) | WNDPETGDTEYAPKFQG (SEQ ID NO:126) | GSGLIPY (SEQ ID NO:127) |
| FF K | RSSQSLVHSNGNTYLY (SEQ ID NO:128) | KVSTRFS (SEQ ID NO:129) | SQSSHIPPT (SEQ ID NO:130) |
| FF VH | DYGMN (SEQ ID NO:131) | WIDTYTEKPTYADDFKG (SEQ ID NO:132) | SNFDF (SEQ ID NO:133) |
| GG VK | KASQDVDTSVA (SEQ ID NO:134) | WASTRHT (SEQ ID NO:135) | QQYSNYPT (SEQ ID NO:136) |
| GG VH | DYYMH (SEQ ID NO:137) | RIDPENGNTIYDPKFQG (SEQ ID NO:138) | SPYDYHAWFAY (SEQ ID NO:139) |
| HH VK | KASQDISNYFT (SEQ ID NO:140) | RANRLVD (SEQ ID NO:141) | LQYDEFPYT (SEQ ID NO:142) |
| HH VH | TYTMS (SEQ ID NO:143) | YISDGGGSSYFPDTVKG (SEQ ID NO:144) | HSNWYFDV (SEQ ID NO:145) |
| II VK | RASQDISNYLN (SEQ ID NO:146) | YTSTLTS (SEQ ID NO:147) | QQGKTFPFT (SEQ ID NO:148) |
| II VH | NYFIE (SEQ ID NO:149) | AINPGSGGTNYNERFKG (SEQ ID NO:150) | EDYGDVYAMDY (SEQ ID NO:151) |
| JJ VK | KASQDVDTSVA | WASTRHT | QQYSSYPT |

| **Antibody** | **CDR1** | **CDR2** | **CDR3** |
|---|---|---|---|
| | (SEQ ID NO:152) | (SEQ ID NO:153) | (SEQ ID NO:154) |
| JJ VH | DYYMH (SEQ ID NO155) | RIDPENGNTIYDPKFQG (SEQ ID NO:156) | SPYDYHAWFAY (SEQ ID NO:157) |
| KK VK | KASQDVDTSVA (SEQ ID NO:158) | WASTRHT (SEQ ID NO: 159) | QQYSSYPT (SEQ ID NO:160) |
| KK VH | DYYMH (SEQ ID NO:161) | RIDPENGNTIYDPKFQG (SEQ ID NO:162) | SPYDYHAWFAY (SEQ ID NO:163) |
| LL VK | KASQSVDYDGDSYMN (SEQ ID NO:164) | AASNLES (SEQ ID NO:165) | QQTNEDPPT (SEQ ID NO:166) |
| LL VH | SFWIH (SEQ ID NO:167) | EINPSNGRTDYNAKFKT (SEQ ID NO:168) | GGTGTWYFDV (SEQ ID NO:169) |
| MM VK | KSSQSLLDSDGETYLN (SEQ ID NO:170) | LVSKLDS (SEQ ID NO:171) | WQGTHFPYT (SEQ ID NO:172) |
| MMVH | NYWMN (SEQ ID NO:173) | EIRLKSDNYATHFAESVK G (SEQ ID NO:174) | ILFGY (SEQ ID NO:175) |
| NN VK | KASQSVDYDGDSYMN (SEQ ID NO:176) | AASNLES (SEQ ID NO:177) | QQSNEDPWT (SEQ ID NO:178) |
| NN VH | SYWMH (SEQ ID NO:179) | EINPSNGRTDYNENFKS (SEQ ID NO:180) | GGVYAMDY (SEQ ID NO:181) |
| OO VK | RSSQSLVHSNGDTYLH (SEQ ID NO:182) | EISNRFS (SEQ ID NO:183) | SQSTHVPFT (SEQ ID NO:184) |
| OO VH | DYYIH (SEQ ID NO:185) | WIDPENGDTEYAPKFQD (SEQ ID NO:186) | GSGLIPY (SEQ ID NO:187) |
| PP VK | RASQDISNYLN (SEQ ID NO:188) | YTSTLTS (SEQ ID NO:189) | QQGKTFPFT (SEQ ID NO:190) |
| PP VH | NYFIE (SEQ ID NO:191) | VINPENGGTNYNERFKD (SEQ ID NO:192) | EDYGDVYAMDY (SEQ ID NO:193) |
| QQ VK | RSSQSLVHSNGDTYLH (SEQ ID NO:194) | EISNRFS (SEQ ID NO:195) | SQSTHVPFT (SEQ ID NO:196) |
| QQ VH | DYYIH (SEQ ID NO:197) | WIDPENGDSEYAPKFQD (SEQ ID NO:198) | GSGLIPY (SEQ ID NO:199) |
| RR VK | RSSQSLVHSNGDVYFH (SEQ ID NO:200) | EVSNRFS (SEQ ID NO:201) | SQSTHVPYT (SEQ ID NO:202) |
| RR VH | DYYIH (SEQ ID NO:203) | WIDPENGDSEYAPKFQD (SEQ ID NO:204) | GSGLIPY (SEQ ID NO:205) |
| SS VK | RSSQSLVHSNGDVYFH (SEQ ID NO:206) | EVSNRFS (SEQ ID NO:207) | SQSTHVPYT (SEQ ID NO:208) |
| SS VH | DYYVH (SEQ ID NO:209) | WIDPDNGDSEYAPKFQD (SEQ ID NO:210) | GSGLIPY (SEQ ID NO:211) |
| TT VK | RSSQSLVHSNGDVYLH (SEQ ID NO:212) | EVSNRFS (SEQ ID NO:213) | SQTTHVPYT (SEQ ID NO:214) |
| TT VH | DYYIH (SEQ ID NO:215) | WIDPENGDTEYAPKFQD (SEQ ID NO:216) | GSGLIPY (SEQ ID NO:217) |
| UU VK | RSSQSLVHSNGDVYLH (SEQ ID NO:218) | EVSNRFS (SEQ ID NO:219) | SQSTHVPYT (SEQ ID NO:220) |
| UU VH | DYYIH (SEQ ID NO:221) | WIDPENGDTEYAPKFQD (SEQ ID NO:222) | GSGLIPY (SEQ ID NO:223) |
| VV VK | HASQNINVWLS (SEQ ID NO:224) | KVSNLHT (SEQ ID NO:225) | QQGQSYPLT (SEQ ID NO:226) |
| VV VH | DYYIH (SEQ ID NO:227) | RIDPENGNTIYDPKFQG (SEQ ID NO:228) | CDNDPGSEMDY (SEQ ID NO:229) |
| WW VK | RSSQSLVHSNGDVYFH (SEQ ID NO:230) | EVSNRFS (SEQ ID NO:231) | SQSTHVPYT (SEQ ID NO:232) |
| WW VH | DYYVH (SEQ ID NO:233) | WIDPDNGDSEYAPKFQD (SEQ ID NO:234) | GSGLIPY (SEQ ID NO:235) |

An oligopeptide or polypeptide is within the scope of the invention if it has an amino acid sequence that is at least 75%, 76%. 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to least one of the CDR's of Table 2 above; and/or to a CDR of a sclerostin binding agent that cross-blocks the binding of at least one of antibodies AA-WW to sclerostin; and/or is cross-blocked from binding to sclerostin by at least one of antibodies and/or to a CDR of a sclerostin binding agent wherein the binding agent can block the inhibitory effect of sclerostin in a cell based mineralization assay *(i.e.,* a sclerostin neutralizing binding agent).

Sclerostin binding agent polypeptides and antibodies are within the scope of the invention if they have amino acid sequences that are at least 85%, 86%, 87%. 88%, 89%, 90%, 91%, 92%, 93%, 94%, 95%, 96%, 97%, 98% or 99% identical to a variable region of at least one of antibodies AA-WW and cross-block the binding of at least one of antibodies AA-WW to sclerostin; and/or are cross-blocked from binding to sclerostin by at least one of antibodies AA-WW; and/or can block the inhibitory effect of sclerostin in a cell based mineralization assay *(i.e.,* a sclerostin neutralizing binding agent).

Polynucleotides encoding sclerostin binding agents are within the scope of the invention if they have polynucleotide sequences that are at least 85%, 86%, 87%, 88%, 89%, 90%, 91%, 92%, 93%, 94%, 95%, 96%, 97%, 98% or 99% identical to a polynucleotide encoding a variable region of at least one of antibodies, and wherein the encoded sclerostin binding agents cross-block the binding of at least one of antibodies to sclerostin; and/or are cross-blocked from binding to sclerostin by at least one of antibodies AA-WW; and/or can block the inhibitory effect of sclerostin in a cell based mineralization assay (*i.e*., a sclerostin neutralizing binding agent).

Antibodies according to the invention may have a binding affinity for human sclerostin of less than or equal to 1 x 10⁻⁷ M, less than or equal to 1 x 10⁻⁸ M, less than or equal to 1 x 10⁻⁹ M, less than or equal to 1 x 10⁻¹⁰ M, less than or equal to 1 x 10⁻¹¹ M, or less than or equal to 1 x 10⁻¹² M.

The affinity of a binding agent such as an antibody or binding partner, as well as the extent to which a binding agent (such as an antibody) inhibits binding, can be determined by one of ordinary skill in the art using conventional techniques, for example, those described by Scatchard et al., Ann. N. Y. Acad. Sci., 51:660-672 (1949)) or by surface plasmon resonance (SPR; BIAcore, Biosensor, Piscataway, NJ). For surface plasmon resonance, target molecules are immobilized on a solid phase and exposed to ligands in a mobile phase running along a flow cell. If ligand binding to the immobilized target occurs, the local refractive index changes, leading to a change in SPR angle, which can be monitored in real time by detecting changes in the intensity of the reflected light. The rates of change of the SPR signal can be analyzed to yield apparent rate constants for the association and dissociation phases of the binding reaction. The ratio of these values gives the apparent equilibrium constant (affinity) (*see, e.g.,* Wolff et al., Cancer Res., 53:2560-65 (1993)).

An antibody according to the present invention may belong to any immunoglobin class, for example IgG, IgE, IgM, IgD, or IgA. It may be obtained from or derived from an animal, for example, fowl (*e.g*., chicken) and mammals, which includes but, is not limited, to a mouse, rat, hamster, rabbit, or other rodent, cow, horse, sheep, goat, camel, human, or other primate. The antibody may be an internalizing antibody. Production of antibodies is disclosed generally in U.S. Patent Publication No. 2004/0146888 A1.

### Characterization Assays

In the methods described above to generate antibodies according to the invention, including the manipulation of the specific antibody AA-WW CDRs into new frameworks and/or constant regions, appropriate assays are available to select the desired antibodies or binding agents (i.e., assays for determining binding affinity to sclerostin; cross-blocking assays; Biacore-based "human sclerostin peptide epitope competition binding assay;" MC3T3-E1 cell based assay; and/or *in vivo* assays).

### CROSS-BLOCKING ASSAYS

The terms "cross-block," "cross-blocked" and "cross-blocking" are used interchangeably herein to mean the ability of an antibody or other binding agent to interfere with the binding of other antibodies or binding agents to sclerostin.

The extent to which an antibody or other binding agent is able to interfere with the binding of another to sclerostin, and therefore whether it can be said to cross-block according to the invention, can be determined using competition binding assays. One particularly suitable quantitative assay uses a Biacore machine which can measure the extent of interactions using surface plasmon resonance technology. Another suitable quantitative cross-blocking assay uses an ELISA-based approach to measure competition between antibodies or other binding agents in terms of their binding to sclerostin.

### BIACORE CROSS-BLOCKING ASSAY

The following generally describes a suitable Biacore assay for determining whether an antibody or other binding agent cross-blocks or is capable of cross-blocking according to the invention. For convenience, reference is made to two antibodies, but it will be appreciated that the assay can be used with any of the sclerostin binding agents described herein. The Biacore machine (for example the Biacore 3000) is operated in line with the manufacturer's recommendations.

Thus, in one cross-blocking assay, sclerostin is coupled to a CM5 Biacore chip using standard amine coupling chemistry to generate a sclerostin-coated surface. Typically 200-800 resonance units of sclerostin would be coupled to the chip (an amount that gives easily measurable levels of binding but that is readily saturable by the concentrations of test reagent being used).

The two antibodies (termed 1* and 2*) to be assessed for their ability to cross-block each other are mixed at a one to one molar ratio of binding sites in a suitable buffer to create the test mixture. When calculating the concentrations on a binding site basis the molecular weight of an antibody is assumed to be the total molecular weight of the antibody divided by the number of sclerostin binding sites on that antibody.

The concentration of each antibody in the test mix should be high enough to readily saturate the binding sites for that antibody on the sclerostin molecules captured on the Biacore chip. The antibodies in the mixture are at the same molar concentration (on a binding basis) and that concentration would typically be between 1.00 and 1.5 micromolar (on a binding site basis).

Separate solutions containing antibody 1* alone and antibody 2* alone are also prepared. Antibody 1* and antibody 2* in these solutions should be in the same buffer and at the same concentration as in the test mix.

The test mixture is passed over the sclerostin-coated Biacore chip and the total amount of binding recorded. The chip is then treated in such a way as to remove the bound antibodies without damaging the chip-bound sclerostin. Typically this is done by treating the chip with 30 mM HCl for 60 seconds.

The solution of antibody 1* alone is then passed over the sclerostin-coated surface and the amount of binding recorded. The chip is again treated to remove all of the bound antibody without damaging the chip-bound sclerostin.

The solution of antibody 2* alone is then passed over the sclerostin-coated surface and the amount of binding recorded.

The maximum theoretical binding of the mixture of antibody 1* and antibody 2* is next calculated, and is the sum of the binding of each antibody when passed over the sclerostin surface alone. If the actual recorded binding of the mixture is less than this theoretical maximum then the two antibodies are cross-blocking each other.

Thus, in general, a cross-blocking antibody or other binding agent according to the invention is one which will bind to sclerostin in the above Biacore cross-blocking assay such that during the assay and in the presence of a second antibody or other binding agent of the invention the recorded binding is between 80% and 0.1% (*e.g*., between 80% and 4%) of the maximum theoretical binding, specifically between 75% and 0.1% (*e.g*., between 75% and 4%) of the maximum theoretical binding, and more specifically between 70% and 0.1% (e.g., between 70% and 4%) of maximum theoretical binding (as just defined above) of the two antibodies or binding agents in combination.

The Biacore assay described above is a primary assay used to determine if antibodies or other binding agents cross-block each other according to the invention. On rare occasions particular antibodies or other binding agents may not bind to sclerostin coupled via amine chemistry to a CM5 Biacore chip (this usually occurs when the relevant binding site on sclerostin is masked or destroyed by the coupling to the chip). In such cases cross-blocking can be determined using a tagged version of Sclerostin, for example N-terminal His-tagged Sclerostin (R & D Systems, Minneapolis, MN, USA; 2005 cat# 1406-ST-025). In this particular format, an anti-His antibody would be coupled to the Biacore chip and then the His-tagged sclerostin would be passed over the surface of the chip and captured by the anti-His antibody. The cross blocking analysis would be carried out essentially as described above, except that after each chip regeneration cycle, new His-tagged sclerostin would be loaded back onto the anti-His antibody coated surface. In addition to the example given using N-terminal His-tagged sclerostin, C-terminal His-tagged sclerostin could alternatively be used. Furthermore, various other tags and tag binding protein combinations that are known in the art could be used for such a cross-blocking analysis (*e.g*., HA tag with anti-HA antibodies; FLAG tag with anti-FLAG antibodies; and/or biotin tag with streptavidin).

### ELISA-BASED CROSS-BLOCKING ASSAY

The following generally describes an ELISA assay for determining whether an anti-sclerostin antibody or other sclerostin binding agent cross-blocks or is capable of cross-blocking according to the invention. For convenience, reference is made to two antibodies (Ab-1 and Ab-2), but it will be appreciated that the assay can be used with any of the sclerostin binding agents described herein.

The general principle of the assay is to have an anti-sclerostin antibody coated onto the wells of an ELISA plate. An excess amount of a second, potentially cross-blocking, anti-sclerostin antibody is added in solution (i.e., not bound to the ELISA plate). A limited amount of sclerostin is then added to the wells. The coated antibody and the antibody in solution compete for binding of the limited number of sclerostin molecules. The plate is washed to remove sclerostin that has not been bound by the coated antibody and to also remove the second, solution phase antibody as well as any complexes formed between the second, solution phase antibody and sclerostin. The amount of bound sclerostin is then measured using an appropriate sclerostin detection reagent. An antibody in solution that is able to cross-block the coated antibody will be able to cause a decrease in the number of sclerostin molecules that the coated antibody can bind relative to the number of sclerostin molecules that the coated antibody can bind in the absence of the second, solution phase, antibody.

This assay is described below in more detail for Ab-1 and Ab-2. In the instance where Ab-1 is chosen to be the immobilized antibody, it is coated onto the wells of the ELISA plate, after which the plates are blocked with a suitable blocking solution to minimize non-specific binding of reagents that are subsequently added. An excess amount of Ab-2 is then added to the ELISA plate such that the moles of Ab-2 sclerostin binding sites per well are at least 10 fold higher than the moles of Ab-1 sclerostin binding sites that were used, per well, during the coating of the ELISA plate. Sclerostin is then added such that the moles of sclerostin added per well are at least 25-fold lower than the moles of Ab-1 sclerostin binding sites that were used for coating each well. Following a suitable incubation period, the ELISA plate is washed and a sclerostin detection reagent is added to measure the amount of sclerostin specifically bound by the coated anti-sclerostin antibody (in this case Ab-1). The background signal for the assay is defined as the signal obtained in wells with the coated antibody (in this case Ab-1), second solution phase antibody (in this case Ab-2), sclerostin buffer only *(i.e.,* no sclerostin) and sclerostin detection reagents. The positive control signal for the assay is defined as the signal obtained in wells with the coated antibody (in this case Ab-1), second solution phase antibody buffer only *(i.e.,* no second solution phase antibody), sclerostin and sclerostin detection reagents. The ELISA assay needs to be run in such a manner so as to have the positive control signal be at least 6 times the background signal.

To avoid any artifacts resulting from the choice of which antibody to use as the coating antibody and which to use as the second (competitor) antibody (*e.g*., significantly different affinities between Ab-1 and Ab-2 for sclerostin), the cross-blocking assay can be run in two formats:
1) format 1 is where Ab-1 is the antibody that is coated onto the ELISA plate and Ab-2 is the competitor antibody that is in solution
   and
2) format 2 is where Ab-2 is the antibody that is coated onto the ELISA plate and Ab-1 is the competitor antibody that is in solution.

Ab-1 and Ab-2 are defined as cross-blocking if, either in format 1 or in format 2, the solution phase anti-sclerostin antibody is able to cause a reduction of between 60% and 100%, specifically between 70% and 100%, and more specifically between 80% and 100%, of the sclerostin detection signal (*i.e*., the amount of sclerostin bound by the coated antibody) as compared to the sclerostin detection signal obtained in the absence of the solution phase anti-sclerostin antibody (*i.e*., the positive control wells).

### CELL-BASED NEUTRALIZATION ASSAY

Mineralization by osteoblast-lineage cells in culture, either primary cells or cell lines, is used as an *in vitro* model of bone formation. Mineralization takes from about one to six weeks to occur beginning with the induction of osteoblast-lineage cell differentiation by one or more differentiation agents. The overall sequence of events involves cell proliferation, differentiation, extracellular matrix production, matrix maturation, and finally deposition of mineral, which refers to crystallization and/or deposition of calcium phosphate. This sequence of events starting with cell proliferation and differentiation, and ending with deposition of mineral, is referred to herein as mineralization. Measurement of calcium (mineral) is the output of the assay.

MC3T3-E1 cells (Sudo et al. "In vitro differentiation and calcification in a new clonal osteogenic cell line derived from newborn mouse calvaria. " J. Cell Biol., 96:191-198 (1983)) and subclones of the original cell line can form mineral in culture upon growth in the presence of differentiating agents. Such subclones include MC3T3-E1-BF (Smith et al., "Glucocorticoids inhibit developmental stage-specific osteoblast cell cycle. " J. Biol. Chem., 275:19992-20001 (2000)). For both the MC3T3-E1-BF subclone as well as the original MC3T3-E1 cells, sclerostin can inhibit one or more of the sequence of events leading up to and including mineral deposition (i.e., sclerostin inhibits mineralization). Anti-sclerostin antibodies that are able to neutralize sclerostin's inhibitory activity allow for mineralization of the culture in the presence of sclerostin such that there is a statistically significant increase in deposition of calcium phosphate (measured as calcium) as compared to the amount of calcium measured in the sclerostin-only (*i.e*., no antibody) treatment group.

When running the assay with the goal of determining whether a particular anti-sclerostin antibody or anti-sclerostin binding agent can neutralize sclerostin (*i.e*., is a sclerostin neutralizing antibody or derivative thereof, or is a sclerostin neutralizing binding agent), the amount of sclerostin used in the assay can be the minimum amount of sclerostin that causes at least a 70%, statistically significant, reduction in deposition of calcium phosphate (measured as calcium) in the sclerostin-only group, as compared to the amount of calcium measured in the no sclerostin group. An anti-sclerostin neutralizing antibody or an anti-sclerostin neutralizing binding agent is defined as one that causes a statistically significant increase in deposition of calcium phosphate (measured as calcium) as compared to the amount of calcium measured in the sclerostin-only (*i.e*., no antibody, no binding agent) treatment group. To determine whether an anti-sclerostin antibody or an anti-sclerostin binding agent is neutralizing or not, the amount of anti-sclerostin antibody or anti-sclerostin binding agent used in the assay needs to be such that there is an excess of moles of sclerostin binding sites per well as compared to the number of moles of sclerostin per well. Depending on the potency of the antibody, the fold excess that may be required can be 24, 18, 12, 6, 3, or 1.5, and one of skill is familiar with the routine practice of testing more than one concentration of binding agent. For example, a very potent anti-sclerostin neutralizing antibody or anti-sclerostin neutralizing binding agent will be able to neutralize sclerostin even when there is less than a 6-fold excess of moles of sclerostin binding sites per well as compared to the number of moles of sclerostin per well. A less potent anti-sclerostin neutralizing antibody or anti-sclerostin neutralizing binding agent will be able to neutralize sclerostin only at a 12, 18, or 24 fold excess. Sclerostin binding agents within this full range of potencies are suitable as neutralizing sclerostin binding agents.

Anti-sclerostin antibodies and derivatives thereof that can neutralize human sclerostin, and sclerostin binding agents that can neutralize human sclerostin, may be of use in the treatment of human conditions/disorders that are caused by, associated with, or result in at least one of low bone formation, low bone mineral density, low bone mineral content, low bone mass, low bone quality and low bone strength.

### IN VIVO NEUTRALIZATION ASSAY

Increases in various parameters associated with, or that result from, the stimulation of new bone formation can be measured as an output from *in vivo* testing of sclerostin binding agents in order to identify those binding agents that are able to neutralize sclerostin and thus able to cause stimulation of new bone formation. Such parameters include various serum anabolic markers (*e.g*., osteocalcin and P1NP (n-terminal propeptide of type 1 procollagen)), histomorphometric markers of bone formation (*e.g*., osteoblast surface/bone surface; bone formation rate/bone surface; and trabecular thickness), bone mineral density, bone mineral content, bone mass, bone quality, and bone strength. A sclerostin neutralizing binding agent is defined as one capable of causing a statistically significant increase, as compared to vehicle treated animals, in any parameter associated with, or that results from, the stimulation of new bone formation. Such *in vivo* testing can be performed in any suitable mammal (e.g. mouse, rat, and/or monkey).

Although the amino acid sequence of sclerostin is not 100% identical across mammalian species (*e.g*., mouse sclerostin is not 100% identical to human sclerostin), it will be appreciated by one skilled in the art that a sclerostin binding agent that can neutralize, *in vivo,* the sclerostin of a certain species (*e.g*., mouse) and that also can bind human sclerostin *in vitro* is very likely to be able to neutralize human sclerostin *in vivo.* Thus, such a human sclerostin binding agent (*e.g*., anti-human sclerostin antibody) may be of use in the treatment of human conditions/disorders that are caused by, associated with, or result in at least one of low bone formation, low bone mineral density, low bone mineral content, low bone mass, low bone quality, and low bone strength. Mice in which homologous recombination had been used to delete the mouse sclerostin gene and insert the human sclerostin gene in its place *(i.e.,* human sclerostin gene knock-in mice or human SOST knock-in mice) would be an example of an additional *in vivo* system.

Pharmaceutical compositions are provided, comprising one of the above-described binding agents such as at least one of antibody AA-WW to human sclerostin, along with a pharmaceutically or physiologically acceptable carrier, excipient, or diluent. Pharmaceutical compositions and methods of treatment are disclosed in copending Application Serial No. 10/868,497, filed June 16, 2004, published as U.S. 2005/0106683, which claims priority to Serial No. 60/478,977, both of which are incorporated by reference herein.

The development of suitable dosing and treatment regimens for using the particular compositions described herein in a variety of treatment regimens, including *e.g*., subcutaneous, oral, parenteral, intravenous, intranasal, and intramuscular administration and formulation, is well known in the art, some of which are briefly discussed below for general purposes of illustration.

In certain applications, the pharmaceutical compositions disclosed herein may be delivered via oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

In certain circumstances it will be desirable to deliver the pharmaceutical compositions disclosed herein subcutaneously, parenterally, intravenously, intramuscularly, or intraperitoneally. Such approaches are well known to the skilled artisan, some of which are further described, for example, in U.S. Patent No. 5,543,158; U.S. Patent No. 5,641,515; and U.S. Patent No. 5,399,363. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U.S. Patent No. 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e.g*., glycerol, propylene glycol, liquid polyethylene glycol, and the like), suitable mixtures thereof, and/or vegetable oils. Proper fluidity may be maintained, for example, by the use of a coating, such as lecithin, by the maintenance of the required particle size in the case of dispersion, and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art. For example, one dosage may be dissolved in 1 ml of isotonic NaCl solution and either added to 1000 ml of hypodermoclysis fluid or injected at the proposed site of infusion (see, for example, Remington's Pharmaceutical Sciences, 15th ed., pp. 1035-1038 and 1570-1580). Some variation in dosage will necessarily occur depending on the condition of the subject being treated. Moreover, for human administration, preparations will preferably meet sterility, pyrogenicity, and the general safety and purity standards as required by FDA Office of Biologics standards.

In another embodiment of the invention, the compositions disclosed herein may be formulated in a neutral or salt form. Illustrative pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine, and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, liposomes, nanocapsules, microparticles, lipid particles, vesicles, and the like, are used for the introduction of the compositions of the present invention into suitable host cells/organisms. In particular, the compositions of the present invention may be formulated for delivery either encapsulated in a lipid particle, a liposome, a vesicle, a nanosphere, or a nanoparticle or the like. Alternatively, compositions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol., 16(7):307-21 (1998); Takakura, Nippon Rinsho, 56(3):691-95 (1998); Chandran et al., Indian J. Exp. Biol., 35(8):801-09 (1997); Margalit, Crit. Rev. Ther. Drug Carrier Syst., 12(2-3):233-61 (1995); U.S. Patent No. 5,567,434; U.S. Patent No. 5,552,157; U.S. Patent No. 5,565,213; U.S. Patent No. 5,738,868, and U.S. Patent No. 5,795,587, each specifically incorporated herein by reference in its entirety). The use of liposomes does not appear to be associated with autoimmune responses or unacceptable toxicity after systemic delivery. In certain embodiments, liposomes are formed from phospholipids that are dispersed in an aqueous medium and spontaneously form multilamellar concentric bilayer vesicles (also termed multilamellar vesicles (MLVs)).

Alternatively, in other embodiments, the invention provides for pharmaceutically-acceptable nanocapsule formulations of the compositions of the present invention. Nanocapsules can generally entrap compounds in a stable and reproducible way (see, for example, Quintanar-Guerrero et al., Drug Dev. Ind. Pharm., 24(12):1113-28 (1998)). To avoid side effects due to intracellular polymeric overloading, such ultrafine particles (sized around 0.1µm) may be designed using polymers able to be degraded *in vivo.* Such particles can be made as described, for example, by Couvreur et al., Crit. Rev. Ther. Drug Carrier Syst., 5(1):1-20 (1988); zur Muhlen et al., Eur. J. Pharm. Biopharm., 45(2):149-55 (1998); Zambaux et al., J. Controlled Release, 50(1-3):31-40 (1998); and U.S. Patent No. 5,145,684.

In addition, pharmaceutical compositions of the present invention may be placed within containers, along with packaging material that provides instructions regarding the use of such pharmaceutical compositions. Generally, such instructions will include a tangible expression describing the reagent concentration, as well as within certain embodiments, relative amounts of excipient ingredients or diluents (*e.g*., water, saline or PBS) that may be necessary to reconstitute the pharmaceutical composition.

The dose administered may range from 0.01 mg/kg to 100 mg/kg of body weight. As will be evident to one of skill in the art, the amount and frequency of administration will depend, of course, on such factors as the nature and severity of the indication being treated, the desired response, the condition of the patient, and so forth. Typically, the compositions may be administered by a variety of techniques, as noted above.

Increases in bone mineral content and/or bone mineral density may be determined directly through the use of X-rays (*e.g*., Dual Energy X-ray Absorptometry or "DEXA"), or by inference through the measurement of (1) markers of bone formation and/or osteoblast activity, such as, but not limited to, osteoblast specific alkaline phosphatase, osteocalcin, type 1 procollagen C' propeptide (PICP), total alkaline phosphatase (*see* Comier, Curr. Opin. in Rheu., 7:243 (1995)) and serum procollagen 1 N-terminal propeptide (P1NP) and/or (2) markers of bone resorption and/or osteoclast activity including, but not limited to, pyridinoline, deoxypryridinoline, N-telopeptide, urinary hydroxyproline, plasma tartrate-resistant acid phosphatases, and galactosyl hydroxylysine *(see* Comier, *id),* serum TRAP 5b (tartrate-resistant acid phosphatase isoform 5b) and serum cross-linked C-telopeptide (sCTXI). The amount of bone mass may also be calculated from body weights or by using other methods (see Guinness-Hey, Metab. Bone Dis. Relat. Res., 5:177-181 (1984). Animals and particular animal models are used in the art for testing the effect of the compositions and methods of the invention on, for example, parameters of bone loss, bone resorption, bone formation, bone strength or bone mineralization that mimic conditions of human disease such as osteoporosis and osteopenias. Examples of such models include the ovariectomized rat model (Kalu, D.N., "The ovariectomized rat model of postmenopausal bone loss." Bone and Mineral, 15:175-192 (1991); Frost, H.M. and Jee, W.S.S., "On the rat model of human osteopenias and osteoporosis." Bone and Mineral, 18:227-236 (1992); and Jee, W.S.S. and Yao, W., "Overview: animal models of osteopenia and osteoporosis." J. Musculoskel. Neuron. Interact., 1:193-207 (2001)).

Particular conditions which may be treated by the compositions of the present invention include dysplasias, wherein growth or development of bone is abnormal and a wide variety of causes of osteopenia, osteoporosis, and bone loss. Representative examples of such conditions include achondroplasia, cleidocranial dysostosis, enchondromatosis, fibrous dysplasia, Gaucher's Disease, hypophosphatemic rickets, Marfan's syndrome, multiple hereditary exotoses, neurofibromatosis, osteogenesis imperfecta, osteopetrosis, osteopoikilosis, sclerotic lesions, pseudoarthrosis, and pyogenic osteomyelitis, periodontal disease, anti-epileptic drug induced bone loss, primary and secondary hyperparathyroidism, familial hyperparathyroidism syndromes, weightlessness induced bone loss, osteoporosis (e.g., osteoporosis in men), postmenopausal bone loss, osteoarthritis, renal osteodystrophy, infiltrative disorders of bone, oral bone loss, osteonecrosis of the jaw, juvenile Paget's disease, melorheostosis, metabolic bone diseases, mastocytosis, sickle cell anemia/disease, organ transplant related bone loss, kidney transplant related bone loss, systemic lupus erythematosus, ankylosing spondylitis, epilepsy, juvenile arthritides, thalassemia, mucopolysaccharidoses, fabry disease, Turner syndrome, Down Syndrome, Klinefelter Syndrome, leprosy, Perthes' Disease, adolescent idiopathic scoliosis, infantile onset multi-system inflammatory disease, Winchester Syndrome, Menkes Disease, Wilson's Disease, ischemic bone disease (such as Legg-Calve-Perthes disease, regional migratory osteoporosis), anemic states, conditions caused by steroids, glucocorticoid-induced bone loss, heparin-induced bone loss, bone marrow disorders, scurvy, malnutrition, calcium deficiency, idiopathic osteopenia or osteoporosis, congenital osteopenia or osteoporosis, alcoholism, chronic liver disease, postmenopausal state, chronic inflammatory conditions, rheumatoid arthritis, inflammatory bowel disease, ulcerative colitis, inflammatory colitis, Crohn's disease, oligomenorrhea, amenorrhea, pregnancy, diabetes mellitus, hyperthyroidism, thyroid disorders, parathyroid disorders, Cushing's disease, acromegaly, hypogonadism, immobilization or disuse, reflex sympathetic dystrophy syndrome, regional osteoporosis, osteomalacia, bone loss associated with joint replacement, HIV associated bone loss, bone loss associated with loss of growth hormone, bone loss associated with cystic fibrosis, fibrous dysplasia, chemotherapy associated bone loss, tumor induced bone loss, cancer-related bone loss, hormone ablative bone loss, multiple myeloma, drug-induced bone loss, anorexia nervosa, disease associated facial bone loss, disease associated cranial bone loss, disease associated bone loss of the jaw, disease associated bone loss of the skull, and bone loss associated with space travel. Further conditions relate to bone loss associated with aging, including facial bone loss associated with aging, cranial bone loss associated with aging, jaw bone loss associated with aging, and skull bone loss associated with aging.

Compositions of the present invention may also be useful for improving outcomes in orthopedic procedures, dental procedures, implant surgery, joint replacement, bone grafting, bone cosmetic surgery and bone repair such as fracture healing, nonunion healing, delayed union healing and facial reconstruction. One or more compositions may be administered before, during and/or after the procedure, replacement, graft, surgery or repair.

The invention also provides a diagnostic kit comprising at least one anti-sclerostin binding agent according to the present invention. The binding agent may be an antibody. In addition, such a kit may optionally comprise one or more of the following:
(1) instructions for using the one or more binding agent(s) for screening, diagnosis, prognosis, therapeutic monitoring or any combination of these applications;
(2) a labeled binding partner to the anti-sclerostin binding agent(s);
(3) a solid phase (such as a reagent strip) upon which the anti-sclerostin binding agent(s) is immobilized; and
(4) a label or insert indicating regulatory approval for screening, diagnostic, prognostic or therapeutic use or any combination thereof.

If no labeled binding partner to the binding agent(s) is provided, the binding agent(s) itself can be labeled with one or more of a detectable marker(s), *e.g*., a chemiluminescent, enzymatic, fluorescent, or radioactive moiety.

The following examples are offered by way of illustration, and not by way of limitation.

### EXAMPLES

### EXAMPLE 1

### RECOMBINANT EXPRESSION OF SCLEROSTIN

Recombinant human sclerostin/SOST is commercially available from R&D Systems (Minneapolis, MN, USA; 2006 cat# 1406-ST-025). Additionally, recombinant mouse sclerostin/SOST is commercially available from R&D Systems (Minneapolis, MN, USA; 2006 cat# 1589-ST-025).

Alternatively, the different species of sclerostin can be expressed transiently in serum-free suspension adapted 293T or 293EBNA cells. Transfections can be performed as 500 mL or 1L cultures. The following reagents and materials are available from Gibco BRL (now Invitrogen, Carlsbad, CA). Catalog numbers are listed in parentheses: serum-free DMEM (21068-028); DMEM/F12 (3:1) (21068/11765); 1X Insulin-Transferrin-Selenium Supplement (51500-056); 1X Pen Strep Glut (10378-016); 2mM 1-Glutamine (25030-081); 20 mM HEPES (15630-080); 0.01% Pluronic F68 (24040-032). Briefly, the cell inoculum (5.0-10.0 X 10⁵ cells/mL X culture volume) is centrifuged at 2,500 RPM for 10 minutes at 4°C to remove the conditioned medium.

The cells are resuspended in serum-free DMEM and centrifuged again at 2,500 RPM for 10 minutes at 4°C. After aspirating the wash solution, the cells are resuspended in growth medium [DMEM/F12 (3:1) + 1X Insulin-Transferrin-Selenium Supplement + 1X Pen Strep Glut + 2mM L-Glutamine + 20 mM HEPES + 0.01 % Pluronic F68] in a 1L or 3L spinner flask culture. The spinner flask culture is maintained on magnetic stir plate at 125 RPM which is placed in a humidified incubator maintained at 37°C and 5% CO₂. The mammalian expression plasmid DNA (e.g., pcDNA3.1, pCEP4, Invitrogen Life Technologies, Carlsbad, CA), containing the complete coding region (and stop codon) of sclerostin with a Kozak consensus sequence (e.g., CCACC) directly 5' of the start site ATG, is complexed to the transfection reagent in a 50 mL conical tube.

The DNA-transfection reagent complex can be prepared in 5-10% of the final culture volume in serum-free DMEM or OPTI-MEM. The transfection reagents that can be used for this purpose include X-tremeGene RO-1539 (Roche Applied Science, Indianapolis, IN), FuGene6 (Roche Applied Science, Indianapolis, IN), Lipofectamine 2000 (Invitrogen, Carlsbad, CA), and 293fectin (Invitrogen, Carlsbad, CA). 1-5 µg plasmid DNA/mL culture is first added to serum-free DMEM, followed by 1-5 µl transfection reagent/mL culture. The complexes can be incubated at room temperature for approximately 10-30 minutes and then added to the cells in the spinner flask. The transfection/expression can be performed for 4-7 days, after which the conditioned medium (CM) is harvested by centrifugation at 4,000 RPM for 60 minutes at 4°C.

### EXAMPLE 2

### PURIFICATION OF RECOMBINANT SCLEROSTIN

Recombinant sclerostin was purified from mammalian host cells as follows. All purification processes were carried out at room temperature. One purification scheme was used to purify various species of sclerostin, including murine and human sclerostin. The purification scheme used affinity chromatography followed by cation exchange chromatography.

### Heparin Chromatography

The mammalian host cell conditioned medium (CM) was centrifuged in a Beckman J6-M1 centrifuge at 4000 rpm for 1 hour at 4°C to remove cell debris. The CM supernatant was then filtered through a sterile 0.2 µm filter. (At this point the sterile filtered CM may be optionally stored frozen until purification.) If the CM was frozen, it was thawed at the following temperatures, or combination thereof: 4°C, room temperature or warm water. Following thawing, the CM was filtered through a sterile 0.2 µm filter and optionally concentrated by tangential flow ultrafiltration (TFF) using a 10 kD molecular weight cut-off membrane. The CM concentrate was filtered through a sterile 0.2 µm filter and then loaded onto a Heparin High Performance (Heparin HP) column (GE Healthcare, formerly Amersham Biosciences) equilibrated in PBS. Alternatively, the filtered CM supernatant may be loaded directly onto the Heparin HP column equilibrated in PBS.

After loading, the Heparin HP column was washed with PBS until the absorbance at 280 nm of the flow-through returned to baseline (*i.e*., absorbance measured before loading CM supernatant). The sclerostin was then eluted from the column using a linear gradient from 150 mM to 2M sodium chloride in PBS. The absorbance at 280 nm of the eluate was monitored and fractions containing protein were collected. The fractions were then assayed by Coomassie-stained SDS-PAGE to identify fractions containing a polypeptide that migrates at the size of glycosylated sclerostin. The appropriate fractions from the column were combined to make the Heparin HP pool.

### Cation Exchange Chromatography

The sclerostin eluted from the Heparin HP column was further purified by cation exchange chromatography using SP High Performance (SPHP) chromatography media (GE Healthcare, formerly Amersham Biosciences). The Heparin HP pool was buffer exchanged into PBS by dialysis using 10,000 MWCO membranes (Pierce Slide-A-Lyzer). The dialyzed Heparin HP pool was then loaded onto an SPHP column equilibrated in PBS. After loading, the column was washed with PBS until the absorbance at 280 nm of the flow-through returned to baseline. The sclerostin was then eluted from the SPHP column using a linear gradient from 150 mM to 1 M sodium chloride in PBS. The absorbance at 280 nm of the eluate was monitored and the eluted sclerostin was collected in fractions. The fractions were then assayed by Coomassie-stained SDS-PAGE to identify fractions containing a polypeptide that migrates at the size of glycosylated sclerostin. The appropriate fractions from the column were combined to make the SPHP pool.

### Formulation

Following purification, the SPHP pool was formulated in PBS by dialysis using 10,000 MWCO membranes (Pierce Slide-A-Lyzer). If concentration of sclerostin was necessary, a centrifugal device (Amicon Centricon or Centriprep) with a 10,000 MWCO membrane was used. Following formulation the sclerostin was filtered through a sterile 0.2 µm filter and stored at 4°C or frozen.

### EXAMPLE 3

### ELISA-BASED CROSS-BLOCKING ASSAY

An antibody is coated on an ELISA plate at 2 µg/ml. While plates are blocking, the test antibody is incubated with human sclerostin at a final concentration of 25 ng/ml for one hour at room temperature in a separate plate. This complex is then transferred to the blocked ELISA plate and incubated for a further one hour at room temperature. Plates are washed and a pool of biotinylated anti-sclerostin antibodies at 1 ug/ml is then added and incubated for one hour at room temperature. Plates are then washed and streptavidin-horseradish peroxidase conjugate added at a 1:5000 dilution. Plates are developed with TMB. Blocking antibodies are able to reduce the ELISA signal due to inhibition of sclerostin binding to the coated antibodies. Positive crossblocking wells are considered to be those wells which decreased the signal by at least 40%.

### EXAMPLE 4

### ELISA-BASED CROSS-BLOCKING ASSAY

Liquid volumes used in this example would be those typically used in 96-well plate ELISAs (e.g. 50-200 µl/well). Ab-X and Ab-Y, in this example are assumed to have molecular weights of about 145 Kd and to have 2 sclerostin binding sites per antibody molecule. An anti-sclerostin antibody (Ab-X) is coated (e.g. 50µ of 1 µg/ml) onto a 96-well ELISA plate [e.g. Corning 96 Well EIA/RIA Flat Bottom Microplate (Product # 3590), Coming Inc., Acton, MA] for at least one hour. After this coating step the antibody solution is removed, the plate is washed once or twice with wash solution (e.g., PBS and 0.05% Tween 20) and is then blocked using an appropriate blocking solution (e.g., PBS, 1% BSA, 1% goat serum and 0.5% Tween 20) and procedures known in the art. Blocking solution is then removed from the ELISA plate and a second anti-sclerostin antibody (Ab-Y), which is being tested for it's ability to cross-block the coated antibody, is added in excess (e.g. 50µl of 10µg/ml) in blocking solution to the appropriate wells of the ELISA plate. Following this, a limited amount (e.g. 50µl of 10 ng/ml) of sclerostin in blocking solution is then added to the appropriate wells and the plate is incubated for at least one hour at room temperature while shaking. The plate is then washed 2-4 times with wash solution. An appropriate amount of a sclerostin detection reagent [e.g., biotinylated anti-sclerostin polyclonal antibody that has been pre-complexed with an appropriate amount of a streptavidin-horseradish peroxidase (HRP) conjugate] in blocking solution is added to the ELISA plate and incubated for at least one hour at room temperature. The plate is then washed at least 4 times with wash solution and is developed with an appropriate reagent [e.g. HRP substrates such as TMB (colorimetric) or various HRP luminescent substrates]. The background signal for the assay is defined as the signal obtained in wells with the coated antibody (in this case Ab-X), second solution phase antibody (in this case Ab-Y), sclerostin buffer only (i.e. no sclerostin) and sclerostin detection reagents. The positive control signal for the assay is defined as the signal obtained in wells with the coated antibody (in this case Ab-X), second solution phase antibody buffer only (i.e. no second solution phase antibody), sclerostin and sclerostin detection reagents. The ELISA assay needs to be run in such a manner so as to have the positive control signal be at least 6 times the background signal.

To avoid any artifacts (e.g. significantly different affinities between Ab-X and Ab-Y for sclerostin) resulting from the choice of which antibody to use as the coating antibody and which to use as the second (competitor) antibody, the cross-blocking assay needs to be run in two formats:
1) format 1 is where Ab-X is the antibody that is coated onto the ELISA plate and Ab-Y is the competitor antibody that is in solution
   and
2) format 2 is where Ab-Y is the antibody that is coated onto the ELISA plate and Ab-X is the competitor antibody that is in solution.

Ab-X and Ab-Y are defined as cross-blocking if, either in format 1 or in format 2, the solution phase anti-sclerostin antibody is able to cause a reduction of between 60% and 100%, specifically between 70% and 100%, and more specifically between 80% and 100%, of the sclerostin detection signal (i.e. the amount of sclerostin bound by the coated antibody) as compared to the sclerostin detection signal obtained in the absence of the solution phase anti-sclerostin antibody (i.e. the positive control wells).

In the event that a tagged version of sclerostin is used in the ELISA, such as a N-terminal His-tagged Sclerostin (R&D Systems, Minneapolis, MN, USA; 2005 cat# 1406-ST-025) then an appropriate type of sclerostin detection reagent would include an HRP labeled anti-His antibody. In addition to using N-terminal His-tagged Sclerostin, one could also use C-terminal His-tagged Sclerostin. Furthermore, various other tags and tag binding protein combinations that are known in the art could be used in this ELISA-based cross-blocking assay (e.g., HA tag with anti-HA antibodies; FLAG tag with anti-FLAG antibodies; biotin tag with streptavidin).

From the foregoing, although specific embodiments of the invention have been described herein for purposes of illustration, various modifications may be made without deviating from the spirit and scope of the invention. Accordingly, the invention is not limited except as by the appended claims. All publications, published patent applications, and patent documents disclosed herein are hereby incorporated by reference.

### ASPECTS OF THE DISCLOSURE

(1) An isolated antibody selected from the group consisting of Antibody AA - Antibody WW.
(2) A polypeptide comprising one or more complementarity determining regions of Antibody AA, wherein the peptide comprises a binding affinity for human sclerostin of less than or equal to 1 x 10⁻⁷ M.
(3) A polypeptide comprising one or more complementarity determining regions of Antibody BB, wherein the peptide comprises a binding affinity for human sclerostin of less than or equal to 1 x 10⁻⁷ M.
(4) A polypeptide comprising one or more complementarity determining regions of Antibody CC, wherein the peptide comprises a binding affinity for human sclerostin of less than or equal to 1 x 10⁻⁷ M.
(5) A polypeptide comprising one or more complementarity determining regions of Antibody DD, wherein the peptide comprises a binding affinity for human sclerostin of less than or equal to 1 x 10⁻⁷ M.
(6) A polypeptide comprising one or more complementarity determining regions of Antibody EE, wherein the peptide comprises a binding affinity for human sclerostin of less than or equal to 1 x 10⁻⁷ M.
(7) A polypeptide comprising one or more complementarity determining regions of Antibody FF, wherein the peptide comprises a binding affinity for human sclerostin of less than or equal to 1 x 10⁻⁷ M.
(8) A polypeptide comprising one or more complementarity determining regions of Antibody GG, wherein the peptide comprises a binding affinity for human sclerostin of less than or equal to 1 x 10⁻⁷ M.
(9) A polypeptide comprising one or more complementarity determining regions of Antibody HH, wherein the peptide comprises a binding affinity for human sclerostin of less than or equal to 1 x 10⁻⁷ M.
(10) A polypeptide comprising one or more complementarity determining regions of Antibody II, wherein the peptide comprises a binding affinity for human sclerostin of less than or equal to 1 x 10⁻⁷ M.
(11) A polypeptide comprising one or more complementarity determining regions of Antibody JJ, wherein the peptide comprises a binding affinity for human sclerostin of less than or equal to 1 x 10⁻⁷ M.
(12) A polypeptide comprising one or more complementarity determining regions of Antibody KK, wherein the peptide comprises a binding affinity for human sclerostin of less than or equal to 1 x 10⁻⁷ M.
(13) A polypeptide comprising one or more complementarity determining regions of Antibody LL, wherein the peptide comprises a binding affinity for human sclerostin of less than or equal to 1 x 10⁻⁷ M.
(14) A polypeptide comprising one or more complementarity determining regions of Antibody MM, wherein the peptide comprises a binding affinity for human sclerostin of less than or equal to 1 x 10⁻⁷ M.
(15) A polypeptide comprising one or more complementarity determining regions of Antibody NN, wherein the peptide comprises a binding affinity for human sclerostin of less than or equal to 1 x 10⁻⁷ M.
(16) A polypeptide comprising one or more complementarity determining regions of Antibody OO, wherein the peptide comprises a binding affinity for human sclerostin of less than or equal to 1 x 10⁻⁷ M.
(17) A polypeptide comprising one or more complementarity determining regions of Antibody PP, wherein the peptide comprises a binding affinity for human sclerostin of less than or equal to 1 x 10⁻⁷ M.
(18) A polypeptide comprising one or more complementarity determining regions of Antibody QQ, wherein the peptide comprises a binding affinity for human sclerostin of less than or equal to 1 x 10⁻⁷ M.
(19) A polypeptide comprising one or more complementarity determining regions of Antibody RR, wherein the peptide comprises a binding affinity for human sclerostin of less than or equal to 1 x 10⁻⁷ M.
(20) A polypeptide comprising one or more complementarity determining regions of Antibody SS, wherein the peptide comprises a binding affinity for human sclerostin of less than or equal to 1 x 10⁻⁷ M.
(21) A polypeptide comprising one or more complementarity determining regions of Antibody TT, wherein the peptide comprises a binding affinity for human sclerostin of less than or equal to 1 x 10⁻⁷ M.
(22) A polypeptide comprising one or more complementarity determining regions of Antibody UU, wherein the peptide comprises a binding affinity for human sclerostin of less than or equal to 1 x 10⁻⁷ M.
(23) A polypeptide comprising one or more complementarity determining regions of Antibody VV, wherein the peptide comprises a binding affinity for human sclerostin of less than or equal to 1 x 10⁻⁷ M.
(24) A polypeptide comprising one or more complementarity determining regions of Antibody WW, wherein the peptide comprises a binding affinity for human sclerostin of less than or equal to 1 x 10⁻⁷ M.
(25) The polypeptide of any of (2)-(24), wherein the peptide comprises a binding affinity for human sclerostin of less than or equal to 1 x 10⁻⁸ M.
(26) The polypeptide of any of (2)-(24), wherein the peptide comprises a binding affinity for human sclerostin of less than or equal to 1 x 10⁻⁹ M.
(27) The polypeptide of any of (2)-(24), wherein the peptide comprises a binding affinity for human sclerostin of less than or equal to 1 x 10⁻¹⁰ M.
(28) The polypeptide of any of (2)-(27), wherein the peptide cross- blocks binding of any of Antibodies AA-WW to human sclerostin.
(29) The polypeptide of any of (2)-(28), wherein the polypeptide comprises one or more amino acid sequences selected from the group consisting of SEQ ID NO : 101, 104, 110, 116, 122, 128, 134, 140, 146, 152, 158, 164, 170, 176, 182, 188, 194, 200, 206, 212, 218, 224, 230; SEQ ID NO: 102, 105, 111, 117, 123, 129, 135, 141, 147, 153, 159, 165, 171, 177, 183, 189, 195, 201, 207, 213, 219, 225, 23 1; SEQ ID NO: 103, 106, 112, 118, 124, 130, 136, 142, 148, 154, 160, 166, 172, 178, 184, 190, 196, 202, 208, 214, 220, 226, 232; SEQ ID NO: 98, 107, 113, 119, 125, 131, 137, 143, 149, 155, 161, 167, 173, 179, 185, 191, 197, 203, 209, 215, 221, 227, 233; SEQ ID NO: 99, 108, 114, 120, 132, 144, 150, 156, 162, 168, 174, 180, 186, 192, 198, 204, 210, 216, 222, 228, 234; SEQ ID NO: 100, 109, 115, 121, 127, 133, 139, 145, 151, 157, 163, 169, 175, 181, 187, 193, 199, 205, 211, 217, 223, 229, and 235.
(30) A polypeptide comprising the amino acid sequence of SEQ ID NO: 16, 17, 18, 19, 44, 45, 46, and/or 47.
(31) An isolated nucleic acid molecule comprising a polynucleotide encoding the polypeptide of any of (2)-(30).
(32) A vector comprising the isolated nucleic acid molecule of (31).
(33) A host cell comprising the vector of (32).
(34) A method of making a protein comprising culturing a host cell of (33) under conditions wherein the encoded protein is expressed.

## Claims

1. An isolated antibody selected from the group consisting of Antibody DD, KK, OO, BB, CC, EE-JJ, LL-NN, PP-WW and AA.

2. The antibody of claim 1, which is a monoclonal, preferably where the monoclonal is a human monoclonal, humanized monoclonal or a chimeric monoclonal.

3. The antibody of claim 1 or 2 which is an antibody fragment selected from a F(ab')₂, Fab, Fab' and Fv fragment.

4. A polypeptide comprising one or more complementarity determining regions of Antibody DD, KK, OO, BB, CC, EE-JJ, LL-NN, PP-WW or AA, wherein the peptide comprises a binding affinity for human sclerostin of less than or equal to 1 x 10⁻⁷ M.

5. The polypeptide of claim 4 wherein the peptide comprises a binding affinity for human sclerostin of less than or equal to 1 x 10⁻⁸ M, or less than or equal to 1 x 10⁻⁹ M, less than or equal to 1 x 10⁻¹⁰ M.

6. The polypeptide of claim 4 or 5, wherein the peptide cross-blocks binding of any of Antibodies DD, KK, OO, BB, CC, EE-JJ, LL-NN, PP-WW and AA to human sclerostin.

7. The polypeptide of any of claims 4 to 6, wherein the polypeptide comprises one or more amino acid sequences selected from the group consisting of SEQ ID NO: 101, 104, 110, 116, 122, 128, 134, 140, 146, 152, 158, 164, 170, 176, 182, 188, 194, 200, 206, 212, 218, 224, 230; SEQ ID NO: 102, 105, 111, 117, 123, 129, 135, 141, 147, 153, 159, 165, 171, 177, 183, 189, 195, 201, 207, 213, 219, 225, 23 1; SEQ ID NO: 103, 106, 112, 118, 124, 130, 136, 142, 148, 154, 160, 166, 172, 178, 184, 190, 196, 202, 208, 214, 220, 226, 232; SEQ ID NO: 98, 107, 113, 119, 125, 131, 137, 143, 149, 155, 161, 167, 173, 179, 185, 191, 197, 203, 209, 215, 221, 227, 233; SEQ ID NO: 99, 108, 114, 120, 132, 144, 150, 156, 162, 168, 174, 180, 186, 192, 198, 204, 210, 216, 222, 228, 234; SEQ ID NO: 100, 109, 115, 121, 127, 133, 139, 145, 151, 157, 163, 169, 175, 181, 187, 193, 199, 205, 211, 217, 223, 229, and 235.

8. A polypeptide comprising the amino acid sequence of SEQ ID NO: 16, 17, 18, 19, 44, 45, 46, and/or 47.

9. An isolated nucleic acid molecule comprising a polynucleotide encoding the polypeptide of any of claims 4 to 8.

10. A vector comprising the isolated nucleic acid molecule of claim 9.

11. A host cell comprising the vector of claim 10.

12. A method of making a protein comprising culturing a host cell of claim 11 under conditions wherein the encoded protein is expressed.

13. A pharmaceutical composition comprising an antibody according to any one of claims 1 to 3 or a polypeptide according to any one of claims 4 to 8 along with a pharmaceutically or physiologically acceptable carrier, excipient or diluent.

14. An antibody according to any one of claims 1 to 3 or a polypeptide according to any one of claims 4 to 8 for use in a method of bone repair or a method of treating a dysplasia or a cause of osteopenia, osteoporosis or bone loss.

15. The antibody or polypeptide of claim 14 for use in:
(a) treating osteopenia or osteoporosis;
(b) treating achondroplasia, cleidocranial dysostosis, enchondromatosis, fibrous dysplasia, Gaucher's Disease, hypophosphatemic rickets, Marfan's syndrome, multiple hereditary exotoses, neurofibromatosis, osteogenesis imperfecta, osteopetrosis, osteopoikilosis, sclerotic lesions, pseudoarthrosis, and pyogenic osteomyelitis, periodontal disease, anti-epileptic drug induced bone loss, primary and secondary hyperparathyroidism, familial hyperparathyroidism syndromes, weightlessness induced bone loss, osteoporosis in men, postmenopausal bone loss, osteoarthritis, renal osteodystrophy, infiltrative disorders of bone, oral bone loss, osteonecrosis of the jaw, juvenile Paget's disease, melorheostosis, metabolic bone diseases, mastocytosis, sickle cell anemia/disease, organ transplant related bone loss, kidney transplant related bone loss, systemic lupus erythematosus, ankylosing spondylitis, epilepsy, juvenile arthritides, thalassemia, mucopolysaccharidoses, fabry disease, Turner syndrome, Down Syndrome, Klinefelter Syndrome, leprosy, Perthes' Disease, adolescent idiopathic scoliosis, infantile onset multi-system inflammatory disease, Winchester Syndrome, Menkes Disease, Wilson's Disease, ischemic bone disease (such as Legg-Calve-Perthes disease, regional migratory osteoporosis), anemic states, conditions caused by steroids, glucocorticoid-induced bone loss, heparin-induced bone loss, bone marrow disorders, scurvy, malnutrition, calcium deficiency, idiopathic osteopenia or osteoporosis, congenital osteopenia or osteoporosis, alcoholism, chronic liver disease, postmenopausal state, chronic inflammatory conditions, rheumatoid arthritis, inflammatory bowel disease, ulcerative colitis, inflammatory colitis, Crohn's disease, oligomenorrhea, amenorrhea, pregnancy, diabetes mellitus, hyperthyroidism, thyroid disorders, parathyroid disorders, Cushing's disease, acromegaly, hypogonadism, immobilization or disuse, reflex sympathetic dystrophy syndrome, regional osteoporosis, osteomalacia, bone loss associated with joint replacement, HIV associated bone loss, bone loss associated with loss of growth hormone, bone loss associated with cystic fibrosis, fibrous dysplasia, chemotherapy associated bone loss, tumor induced bone loss, cancer-related bone loss, hormone ablative bone loss, multiple myeloma, drug-induced bone loss, anorexia nervosa, disease associated facial bone loss, disease associated cranial bone loss, disease associated bone loss of the jaw, disease associated bone loss of the skull, and bone loss associated with space travel. Further conditions relate to bone loss associated with aging, including facial bone loss associated with aging, cranial bone loss associated with aging, jaw bone loss associated with aging, and skull bone loss associated with aging; or
(c) improving outcomes in orthopedic procedures, dental procedures, implant surgery, joint replacement, bone grafting, bone cosmetic surgery and bone repair such as fracture healing, nonunion healing, delayed union healing and facial reconstruction.
